# EUROPÄISCHE PATENTSCHRIFT

(11) **EP 1 636 228 B1**
(45) Veröffentlichungstag und Bekanntmachung des Hinweises auf die Patenterteilung: **22.10.2008**
(21) Anmeldenummer: 04733768.8
(22) Anmeldetag: 19.05.2004
(51) Int. Cl.: C07D 471/04, A61K 31/4985, A61P 17/14, A61P 9/10, A61P 35/00

(54) **NEUE PYRIDOPYRAZINE UND DEREN VERWENDUNG ALS MODULATOREN VON KINASEN**
NOVEL PYRIDOPYRAZINES AND USE THEREOF AS KINASE MODULATORS
NOUVELLES PYRIDOPYRAZINES ET LEUR UTILISATION EN TANT QUE MODULATEURS DE KINASES

(30) Priorität: 23.05.2003 DE 10323345; 06.05.2004 DE 102004022383
(43) Veröffentlichungstag der Anmeldung: 22.03.2006
(73) Patentinhaber: Æterna Zentaris GmbH, 60314 Frankfurt am Main (DE)
(72) Erfinder: GÜNTHER, Eckhard, 63477 Maintal (DE); CLAUS, Eckhard, 60388 Frankfurt/Main (DE); SEIPELT, Irene, 63069 Offenbach (DE); RAPP, Ulf-R., 97082 Würzburg (DE); WIXLER, Ludmilla, 97072 Würzburg (DE)
(86) Internationale Anmeldenummer: PCT/EP2004/005388
(87) Internationale Veröffentlichungsnummer: WO 2004/104003

(56) Entgegenhaltungen:
- WO-A-99/17759
- WO-A-03/084473
- US-A1- 2002 107 251
- C. TEMPLE, JR. ET. AL.: "Potential Antimitotic Agents. Synthesis of Some Ethyl Benzopyrazin-7-yl-carbamates, Ethyl Pyrido[3,4-b]pyrazin-7-yl-cabamates, and Ethyl Pyrido[3,4-e]-as-triazin-7-yl-carbamates." JOURNAL OF MEDICINAL CHEMISTRY, Bd. 33, 1990, Seiten 3044-50, XP002293472 in der Anmeldung erwähnt

## Beschreibung

Die Erfindung betrifft Kinase-Modulatoren vom Typ der Pyrido[2,3-b]pyrazine, deren Herstellung und Verwendung als Arzneimittel zur Modulation von fehlgeleiteten zellulären Signaltransduktionsprozessen, insbesondere zur Beeinflussung der Funktion von Tyrosin- und Serin/Threoninkinasen und zur Behandlung von malignen bzw. benignen Tumorerkrankungen und anderen, auf pathologischen Zellproliferationen beruhenden Erkrankungen, wie z. B. Restenose, Psoriasis, Arteriosklerose und Leberzirrhose.

Die Aktivierung von Proteinkinasen ist ein zentrales Ereignis bei zellulären Signaltransduktions-Prozessen. Eine aberrante Kinaseaktivierung wird bei diversen Krankheitszuständen beobachtet. Daher ist die gezielte Inhibition von Kinasen ein fundamentales therapeutisches Ziel.
Die Phosphorylierung von Proteinen wird im Allgemeinen durch extrazelluläre Signale initiiert und stellt einen universellen Mechanismus für die Kontrolle von verschiedenen zellulären Ereignissen, wie z. B. metabolischen Prozessen, Zellwachstum, Zellmigration, Zelldifferenzierung, Membrantransport und Apoptose dar. Für die Proteinphosphorylierung ist die Proteinfamilie der Kinasen verantwortlich. Diese Enzyme katalysieren den Phosphat-Transfer zu spezifischen Substratproteinen. Basierend auf der Substratspezifität werden die Kinasen in zwei Hauptklassen, die Tyrosinkinasen und die Serin/Threonin-Kinasen unterteilt. Sowohl die Rezeptor-Tyrosin-kinasen als auch die cytoplasmatischen Tyrosin- und Serin/Threoninkinasen sind wichtige Proteine der Signaltransduktion der Zelle. Eine Überexpression bzw. Entartung dieser Proteine spielt eine wichtige Rolle bei auf pathologischen Zellproliferationen beruhenden Erkrankungen. Dazu zählen unter anderem Stoffwechselerkrankungen, Erkrankung des Bindegewebes und der Blutgefäße, sowie maligne und benigne Tumorerkrankungen. Bei der Tumorentstehung und Entwicklung treten sie häufig als Onkogene d.h. als aberrante, konstitutiv aktive Kinaseproteine auf. Die Folgen dieser übermäßigen Kinaseaktivierung sind z. B. das unkontrollierte Zellwachstum und der reduzierte Zelltod. Auch die Stimulation von tumorinduzierten Wachstumsfaktoren kann Ursache für die Überstimulation von Kinasen sein. Die Entwicklung von Kinasemodulatoren ist daher von besonderem Interesse für alle pathogenen Prozesse, die durch Kinasen beeinflusst werden.

Die Erfindung ist daher darauf ausgerichtet, neue Verbindungen zu schaffen, die als Modulatoren von Rezeptor-Tyrosinkinasen und cytoplasmatischen Tyrosin- und Serin/Threoninkinasen geeignet sind. Da nicht alle in fehlregulierten Signaltransduktionskaskaden hintereinander geschalteten Kinasen - wie z. B. bei Raf/Mek/Erk - als onkogene Kinasen bzw. als konstitutiv aktive Enzyme vorliegen müssen, werden in dieser Erfindung auch die nicht-aktiven Kinasen als therapeutische Zielproteine betrachtet, d.h. die neuen Verbindungen können sowohl an aktiven als auch an nicht-aktiven Kinasen binden und damit die Signaltransduktion beeinflussen.

In 6- oder 7-Position substituierte Pyrido[2,3-b]pyrazin-Derivate finden als pharmakologisch aktive Verbindungen und als Synthesebausteine in der pharmazeutischen Chemie vielfältige Verwendung. Beispielsweise werden in der Patentschrift WO 99/17759 Pyrido[2,3-b]pyrazine beschrieben, die in 6-Position unter anderem Alkyl-, Aryl- und Heteroarylsubstituierte Carbamate tragen. Diese Verbindungen sollen dazu verwendet werden, die Funktion von Serin-Threonin-Proteinkinasen zu modulieren.
In dem Patent WO 04/005472 von White et al. werden unter anderem in 6-Position Carbamat-substituierte Pyrido[2,3-b]pyrazine beschrieben, die als antibakterielle Substanzen das Wachstum von Bakterien hemmen. Eine Antitumorwirkung ist nicht beschrieben.
Bestimmte Diphenylchinoxaline und -pyrido[2,3-b]pyrazine mit speziellen Alkylpyrrolidin-, Alkylpiperidin- oder Alkylsulfonamid-Resten an einem Phenylring, die zusätzlich auch Harnstoff- oder Carbamat-Substitutionen in 6- oder 7-Position tragen können, werden in den Patentschriften WO 03/084473 (Barnett et al.), WO 03/086394 (Bilodeau et al.) und WO 03/086403 (Lindsley et al.) als Inhibitoren der Aktivität der Serin/Threonin-Kinase Akt beschrieben. Für diese Verbindungen wird eine Verwendung bei der Behandlung von Krebserkrankungen angegeben. Für die dort beschriebenen Pyrido[2,3-b]pyrazin-Beispiel-Verbindungen ist kein definierter Hinweis auf eine biologische Wirkung angegeben. Ausserdem besteht ein deutlicher struktureller Unterschied zu den in dieser Erfindung beschriebenen erfindungsgemäßen Pyrido[2,3-b]pyrazinen.

US 2002/0107251 A1 (Shimazaki et al.) beschreibt 3-Oxo-5-aza-chinoxaline, die in 4-Position arylsubstituiert sind sowie in den Positionen 6 bis 8 Alkoxy- oder Arylthiosubstituenten tragen können. Die Verbindungen sind als TNF-Inhibitoren für die Behandlung von Krebs und entzündlichen Erkrankungen verwendbar. Die Verbindungen weichen von den anmeldungsgemäßen Verbindungen strukturell ab.

Weiterhin werden in dem Patent WO 03/024448 von Delorme et al. Amid- und Acrylamid-substituierte Pyrido[2,3-b]pyrazine beschrieben, die als zusätzliche Substituenten auch Carbamate enthalten und als Histon Deacetylase-Inhibitoren zur Behandlung von Zellproliferationserkrankungen verwendet werden können.
In einer weiteren Publikation (C. Temple, Jr.; J. Med. Chem. 1990, 3044-3050) wird an einem Beispiel die Synthese eines 6-Ethylcarbamat-substituierten Pyrido[2,3-b]pyrazin-Derivates beschrieben. Eine Antitumorwirkung ist weder offenbart noch nahegelegt.
Die Synthese von weiteren Derivaten des 6-Ethylcarbamat-substituierten Pyrido[2,3-b]pyrazins wird in einer Veröffentlichung von R. D. Elliott beschrieben (J. Org. Chem. 1968, 2393-2397). Eine biologische Wirkung dieser Verbindungen ist weder beschrieben noch nahegelegt.
In der Publikation von C.Temple, Jr. J. Med. Chem. 1968,1216-1218 wird die Synthese und Untersuchung von 6-Ethylcarbamat-substituierten Pyrido[2,3-b]pyrazinen als potentielle Antimalaria-Wirkstoffe beschrieben. Eine Antitumorwirkung ist weder offenbart noch nahegelegt.

Es wurde jetzt überraschend gefunden, dass neue Verbindungen aus der Reihe der Pyrido[2,3-b]pyrazine, welche in 6- oder 7-Position z. B. mit Harnstoff-, Thioharnstoff, Guanidin- oder Amidingruppen substituiert sind, zur Herstellung von Arzneimitteln zur Modulation von fehlgeleiteten zellulären Signaltransduktionsprozessen, insbesondere zur Beeinflussung der Funktion von Tyrosin- und Serin/Threoninkinasen und zur Behandlung von malignen bzw. benignen Tumorerkrankungen, wie z. B. der Brust, Prostata, Lunge, Haut, Eierstöcke und anderen, auf pathologischen Zellprollferationen beruhenden Erkrankungen geeignet sind. Gemäß diesem Aspekt werden in der vorliegenden Anmeldung neue Verbindungen aus der Reihe der Pyrido[2,3-b]pyrazine gemäß der allgemeinen Formel I beschrieben, 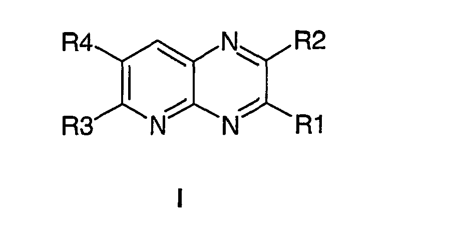 worin die Substituenten R1-R4 folgende Bedeutung haben:

R1 und R2 können unabhängig voneinander:
(i) Wasserstoff
(ii) Hydroxyl
(iii) Halogen, wie beispielsweise Chlor oder Brom
(iv) Alkyl, wobei der Alkylrest gesättigt ist und aus 1 bis 8 C-Atomen bestehen kann,
(v) unsubstituiertes oder substituiertes Aryl, wobei der Arylrest mit F, Cl, Br, I, CF₃, CN, NH₂, NH-Alkyl, NH-Cycloalkyl, NH-Heterocyclyl, NH-Aryl, NH-Heteroaryl, NH-Alkyl-Cycloalkyl, NH-Alkyl-Heterocyclyl, NH-Alkyl-Aryl, NH-Alkyl-Heteroaryl, NH-Alkyl-NH₂, NH-Alkyl-OH, N(Alkyl)₂, NHC(O)-Alkyl, NHC(O)-Cycloalkyl, NHC(O)-Heterocyclyl, NHC(O)-Aryl, NHC(O)-Heteroaryl, NHC(O)-Alkyl-Aryl, NHC(O)-Alkyl-Heteroaryl, NHSO₂-Alkyl, NHSO₂-Cycloalkyl, NHSO₂-Heterocyclyl, NHSO₂-Aryl, NHS₂-Heteroaryl, NHSO₂-Alkyl-Aryl, NHSO₂-Alkyl-Heteroaryl, NO₂, SH, S-Alkyl, S-Aryl, S-Heteroaryl, OH, OCF₃, O-Alkyl, O-Cycloalkyl, O-Heterocyclyl, O-Aryl, O-Heteroaryl, 0-Alkyl-Cycloalkyl, O-Alkyl-Heterocyclyl, O-Alkyl-Aryl, O-Alkyl-Heteroaryl, 0-Alkyl-OH, O-(CH₂)ₙ-O, OC(O)-Alkyl, OC(O)-Cycloalkyl, OC(O)-Heterocyclyl, OC(O)-Aryl, OC(O)-Heteroaryl, OC(O)-Alkyl-Aryl, OC(O)-Alkyl-Heteroaryl, OSO₃H, OSO₂-Alkyl, OSO₂-Cycloalkyl, OSO₂-Heterocyclyl, OSO₂-Aryl, OSO₂-Heteroaryl, OSO₂-Alkyl-Aryl, OSO₂-Alkyl-Heteroaryl, OP(O)(OH)₂, C(O)-Alkyl, C(O)-Aryl, C(O)-Heteroaryl, CO₂H, CO₂-Alkyl, CO₂-Cycloalkyl, CO₂-Heterocyclyl, CO₂-Aryl, CO₂-Heteroaryl, CO₂-Alkyl-Cycloalkyl, CO₂-Alkyl-Heterocyclyl, CO₂-Alkyl-Aryl, CO₂-Alkyl-Heteroaryl, C(O)-NH₂, C(O)NH-Alkyl, C(O)NH-Cycloalkyl, C(O)NH-Heterocyclyl, C(O)NH-Aryl, C(O)NH-Heteroaryl, C(O)NH-Alkyl-Cycloalkyl, C(O)NH-Alkyl-Heterocyclyl, C(O)NH-Alkyl-Aryl, C(0)NH-Alkyl-Heteroaryl, C(O)N(Alkyl)₂, C(O)N(Cycloalkyl)₂, C(O)N(Aryl)₂, C(O)N(Heteroaryl)₂, SO-Alkyl, SO-Aryl, SO₂-Alkyl, SO₂-Aryl, SO₂NH₂, SO₂NH-Alkyl, SO₂NH-Aryl, SO₂NH-Heteroaryl, SO₂NH-Alkyl-Aryl, S03H, SO₂O-Alkyl, SO_{2O}-Aryl, SO₂O-Alkyl-Aryl, Alkyl, Cycloalkyl, Heterocyclyl, Aryl oder Heteroaryl, ein- oder mehrfach, gleich oder verschieden substituiert sein kann, n den Wert 1,2 oder 3 annehmen kann, und die Alkyl-, Cycloalkyl-, Heterocyclyl-, Aryl-, Heteroaryl-, Alkyl-Cycloalkyl-, Alkyl-Heterocyclyl-, Alkyl-Aryl- und Alkyl-Heteroarylsubstituenten ihrerseits wiederum substituiert sein können,
(vi) unsubstituiertes oder substituiertes Heteroaryl, wobei der Heteroarylrest mit F, CI, Br, I, CF₃, CN, NH₂, NH-Alkyl, NH-Cycloalkyl, NH-Heterocyclyl, NH-Aryl, NH-Heteroaryl, NH-Alkyl-Cycloalkyl, NH-Alkyl-Heterocyclyl, NH-Alkyl-Aryl, NH-Alkyl-Heteroaryl, NH-Alkyl-NH₂, NH-Alkyl-OH, N(Alkyl)₂, NHC(O)-Alkyl, NHC(O)-Cycloalkyl, NHC(O)-Heterocyclyl, NHC(O)-Aryl, NHC(O)-Heteroaryl, NHC(O)-Alkyl-Aryl, NHC(O)-Alkyl-Heteroaryl, NHSO₂-Alkyl, NHSO₂-Cycloalkyl, NHSO₂-Heterocyclyl, NHSO₂-Aryl, NHSO₂-Heteroaryl, NHSO₂-Alkyl-Aryl, NHSO₂-Alkyl-Heteroaryl, NO₂, SH, S-Alkyl, S-Aryl, S-Heteroaryl, OH, OCF₃, O-Alkyl, O-Cycloalkyl, O-Aryl, O-Heteroaryl, O-Alkyl-Cycloalkyl, 0-Alkyl-Heterocyclyl, O-Alkyl-Aryl, O-Alkyl-Heteroaryl, OC(O)-Alkyl, OC(O)-Cycloalkyl, OC(O)-Heterocyclyl, OC(O)-Aryl, OC(O)-Heteroaryl, OC(O)-Alkyl-Aryl, OC(O)-Alkyl-Heteroaryl, OSO₃H, OSO₂-Alkyl, OSO₂-Cycloalkyl, OSO₂-Heterocyclyl, OSO₂-Aryl, OSO₂-Heteroaryl, OSO₂-Alkyl-Aryl, OSO₂-Alkyl-Heteroaryl, OP(O)(OH)₂, C(O)-Alkyl, C(O)-Aryl, C(O)-Heteroaryl, CO₂H, CO₂-Alkyl, CO₂-Cycloalkyl, CO₂-Heterocyclyl, CO₂-Aryl, CO₂-Heteroaryl, CO₂-Alkyl-Cycloalkyl, CO₂-Alkyl-Heterocyclyl, CO₂-Alkyl-Aryl, CO₂-Alkyl-Heteroaryl, C(O)-NH₂, C(O)NH-Alkyl, C(O)NH-Cycloalkyl, C(O)NH-Heterocyclyl, C(O)NH-Aryl, C(O)NH-Heteroaryl, C(O)NH-Alkyl-Cycloalkyl, C(O)NH-Alkyl-Heterocyclyl, C(O)NH-Alkyl-Aryl, C(O)NH-Alkyl-Heteroaryl, C(O)N(Alkyl)₂, C(O)N(Cycloalkyl)₂, C(O)N(Aryl)₂, C(O)N(Heteroaryl)₂, SO₂NH₂, SO₂NH-Alkyl, SO₂NH-Aryl, SO₂NH-Heteroaryl, SO₂NH-Alkyl-Aryl, S03H, SO₂O-Alkyl, SO₂O-Aryl, SO₂O-Alkyl-Aryl, Alkyl, Cycloalkyl, Heterocyclyl, Aryl oder Heteroaryl, ein- oder mehrfach, gleich oder verschieden substituiert sein kann, und die Alkyl-, Cycloalkyl-, Heterocyclyl-, Aryl- und Heteroarylsubstituenten ihrerseits wiederum substituiert sein können,
(vii) OR5, wobei R5 Alkyl, Cycloalkyl, Heterocyclyl, Aryl, Heteroaryl, Alkyl-Cycloalkyl, Alkyl-Heterocyclyl, Alkyl-Aryl oder Alkyl-Heteroaryl sein kann, und die Alkyl-, Cycloalkyl-, Heterocyclyl-, Aryl-, Heteroaryl-, Alkyl-Cycloalkyl, Alkyl-Heterocyclyl, Alkyl-Aryl oder Alkyl-Heteroarylsubstituenten ihrerseits wiederum substituiert sein können,
(viii) SR6, wobei R6 Alkyl, Cycloalkyl, Heterocyclyl, Aryl, Heteroaryl, Alkyl-Cycloalkyl, Alkyl-Heterocyclyl, Alkyl-Aryl oder Alkyl-Heteroaryl sein kann, und die Alkyl-, Cycloalkyl-, Heterocyclyl-, Aryl- und Heteroaryl-, Alkyl-Cycloalkyl-, Alkyl-Heterocyclyl-, Alkyl-Aryl- oder Alkyl-Heteroarylsubstituenten ihrerseits wiederum substituiert sein können,
(ix) NR7R8, wobei R7 und R8 unabhängig voneinander Wasserstoff, Alkyl, Cycloalkyl, Heterocyclyl, Aryl, Heteroaryl, Alkyl-Cycloalkyl, Alkyl-Heterocyclyl, Alkyl-Aryl oder Alkyl-Heteroaryl sein können, und die Alkyl-, Cycloalkyl-, Heterocyclyl-, Aryl- und Heteroaryl-, Alkyl-Cycloalkyl, Alkyl-Heterocyclyl, Alkyl-Aryl oder Alkyl-Heteroarylsubstituenten ihrerseits wiederum substituiert sein können, oder R7 und R8 zusammen Cycloalkyl oder Heterocyclyl bedeuten, wobei Cycloalkyl und Heterocyclyl ihrerseits wiederum substituiert sein können,
bedeuten.

R3 und R4 können unabhängig voneinander Wasserstoff oder NR9R10 bedeuten, unter der Voraussetzung, dass, wenn R3 = NR9R10 ist, R4 = H ist, und wenn R4 = NR9R10 ist, R3 = H ist,
wobei R9 Wasserstoff, Alkyl, Cycloalkyl, Heterocyclyl, Aryl, Heteroaryl, Alkyl-Cycloalkyl, Alkyl-Heterocyclyl, Alkyl-Aryl oder Alkyl-Heteroaryl sein kann, und die Alkyl-, Cycloalkyl-, Heterocyclyl-, Aryl- und Heteroaryl-, Alkyl-Cycloalkyl, Alkyl-Heterocyclyl, Alkyl-Aryl oder Alkyl-Heteroarylsubstituenten ihrerseits wiederum substituiert sein können,
und R10:
-C(Y)NR11 R12 bedeuten kann, wobei Y = O, S und R11 und R12 unabhängig voneinander
(i) Wasserstoff,
(ii) unsubstituiertes oder substituiertes Alkyl, wobei der Alkylrest mit F, Cl, Br, I, CF₃, CN, NH₂, NH-Alkyl, NH-Cycloalkyl, NH-Heterocyclyl, NH-Aryl, NH-Heteroaryl, NH-Alkyl-Cycloalkyl, NH-Alkyl-Heterocyclyl, NH-Alkyl-Aryl, NH-Alkyl-Heteroaryl, N(Alkyl)₂, NHC(O)-Alkyl, NHC(O)-Cycloalkyl, NHC(O)-Heterocyclyl, NHC(O)-Aryl, NHC(O)-Heteroaryl, NHC(O)-Alkyl-Aryl, NHC(O)-Alkyl-Heteroaryl, NHSO₂-Alkyl, NHSO₂-Cycloalkyl, NHSO₂-Heterocyclyl, NHSO₂-Aryl, NHSO₂-Heteroaryl, NHSO₂-Alkyl-Aryl, NHSO₂-Alkyl-Heteroaryl, NO₂, SH, S-Alkyl, S-Cycloalkyl, S-Heterocyclyl, S-Aryl, S-Heteroaryl, OH, OCF₃, O-Alkyl, O-Cycloalkyl, O-Heterocyclyl, O-Aryl, O-Heteroaryl, O-Alkyl-Cycloalkyl, O-Alkyl-Heterocyclyl, O-Alkyl-Aryl, O-Alkyl-Heteroaryl, OC(O)-Alkyl, OC(O)-Cycloalkyl, OC(O)-Heterocyclyl, OC(O)-Aryl, OC(O)-Heteroaryl, OC(O)-Alkyl-Aryl, OC(O)-Alkyl-Heteroaryl, OSO₃H, OSO₂-Alkyl, OSO₂-Cycloalkyl, OSO₂-Heterocyclyl, OSO₂-Aryl, OSO₂-Heteroaryl, OSO₂-Alkyl-Aryl, OSO₂-Alkyl-Heteroaryl, OP(O)(OH)₂, C(O)-Alkyl, C(O)-Aryl, C(O)-Heteroaryl, CO₂H, CO₂-Alkyl, CO₂-Cycloalkyl, CO₂-Heterocyclyl, CO₂-Aryl, CO₂-Heteroaryl, CO₂-Alkyl-Cycloalkyl, CO₂-Alkyl-Heterocyclyl, CO₂-Alkyl-Aryl, CO₂-Alkyl-Heteroaryl, C(O)-NH₂, C(O)NH-Alkyl, C(O)NH-Cycloalkyl, C(O)NH-Heterocyclyl, C(O)NH-Aryl, C(O)NH-Heteroaryl, C(O)NH-Alkyl-Cycloalkyl, C(O)NH-Alkyl-Heterocyclyl, C(O)NH-Alkyl-Aryl, C(O)NH-Alkyl-Heteroaryl, C(O)N(Alkyl)₂, C(O)N(Cycloalkyl)₂, C(O)N(Aryl)₂, C(O)N(Heteroaryl)₂, SO-Alkyl, SO-Aryl, SO₂-Alkyl, SO₂-Aryl, SO₂NH₂, SO₂NH-Alkyl, SO₂NH-Aryl, SO₂NH-Heteroaryl, SO₂NH-Alkyl-Aryl, SO₃H, SO₂O-Alkyl, SO₂O-Aryl, SO₂O-Alkyl-Aryl, Cycloalkyl, Heterocyclyl, .Aryl oder Heteroaryl, ein- oder mehrfach, gleich oder verschieden substituiert sein kann,
(iii) unsubstituiertes oder substituiertes Cycloalkyl, wobei der Cycloalkylrest mit F, CI, Br, I, NH₂, NH-Alkyl, NH-Cycloalkyl, NH-Heterocyclyl, NH-Aryl, NH-Heteroaryl, NH-Alkyl-Aryl, NH-Alkyl-Heteroaryl, N(Alkyl)₂, NHC(O)-Alkyl, NHC(O)-Cycloalkyl, NHC(O)-Heterocyclyl, NHC(O)-Aryl, NHC(O)-Heteroaryl, NHC(O)-Alkyl-Aryl, NHC(O)-Alkyl-Heteroaryl, NHSO₂-Alkyl, NHSO₂-Cycloalkyl, NHSO₂-Heterocyclyl, NHSO₂-Aryl, NHSO₂-Heteroaryl, NHSO₂-Alkyl-Aryl, NHSO₂-Alkyl-Heteroaryl, OH, O-Alkyl, O-Cycloalkyl, O-Heterocyclyl, O-Aryl, O-Heteroaryl, O-Alkyl-Aryl, O-Alkyl-Heteroaryl, OC(O)-Alkyl, OC(O)-Cycloalkyl, OC(O)-Heterocyclyl, OC(O)-Aryl, OC(O)-Heteroaryl, OC(O)-Alkyl-Aryl, OC(O)-Alkyl-Heteroaryl, OSO₃H, OSO₂-Alkyl, OSO₂-Cycloalkyl, OSO₂-Heterocyclyl, OSO₂-Aryl, OSO₂-Heteroaryl, OSO₂-Alkyl-Aryl, OSO₂-Alkyl-Heteroaryl, OP(O)(OH)₂, CO₂H, CO₂-Alkyl, CO₂-Cycloalkyl, CO₂-Heterocyclyl, CO₂-Aryl, CO₂-Heteroaryl, CO₂-Alkyl-Cycloalkyl, CO₂-Alkyl-Heterocyclyl, CO₂-Alkyl-Aryl, CO₂-Alkyl-Heteroaryl, C(O)-NH₂, C(O)NH-Alkyl, C(O)NH-Cycloalkyl, C(O)NH-Heterocyclyl, C(O)NH-Aryl, C(O)NH-Heteroaryl, C(O)NH-Alkyl-Cycloalkyl, C(O)NH-Alkyl-Heterocyclyl, C(O)NH-Alkyl-Aryl, C(O)NH-Alkyl-Heteroaryl, C(O)N(Alkyl)₂, C(O)N(Cycloalkyl)₂, C(O)N(Aryl)₂, C(O)N(Heteroaryl)₂, Alkyl, oder Aryl ein- oder mehrfach, gleich oder verschieden substituiert sein kann,
(iv) unsubstituiertes oder substituiertes Heterocyclyl, wobei der Heterocyclylrest mit OH, O-Alkyl, O-Aryl, NH₂, NH-Alkyl, NH-Aryl, Alkyl, Alkyl-Aryl oder Aryl ein- oder mehrfach, gleich oder verschieden substituiert sein kann,
(v) unsubstituiertes oder substituiertes Aryl, wobei der Arylrest mit F, Cl, Br, I, CF₃, CN, NH₂, NH-Alkyl, NH-Cycloalkyl, NH-Heterocyclyl, NH-Aryl, NH-Heteroaryl, NH-Alkyl-Cycloalkyl, NH-Alkyl-Heterocyclyl, NH-Alkyl-Aryl, NH-Alkyl-Heteroaryl, NH-Alkyl-NH₂, NH-Alkyl-OH, N(Alkyl)₂, NHC(O)-Alkyl, NHC(O)-Cycloalkyl, NHC(O)-Heterocyclyl, NHC(O)-Aryl, NHC(O)-Heteroaryl, NHC(O)-Alkyl-Aryl, NHC(O)-Alkyl-Heteroaryl, NHSO₂-Alkyl, NHSO₂-Cycloalkyl, NHSO₂-Heterocyclyl, NHSO₂-Aryl, NHSO₂-Heteroaryl, NHSO₂-Alkyl-Aryl, NHSO₂-Alkyl-Heteroaryl, NO₂, SH, S-Alkyl, S-Cycloalkyl, S-Heterocyclyl, S-Aryl, S-Heteroaryl, OH, OCF₃, O-Alkyl, O-Cycloalkyl, 0-Heterocyclyl, O-Aryl, O-Heteroaryl, O-Alkyl-Cycloalkyl, O-Alkyl-Heterocyclyl, O-Alkyl-Aryl, O-Alkyl-Heteroaryl, O-Alkyl-OH, O-(CH₂)ₙ-O, OC(O)-Alkyl, OC(O)-Cycloalkyl, OC(O)-Heterocyclyl, OC(O)-Aryl, OC(O)-Heteroaryl, OC(O)-Alkyl-Aryl, OC(O)-Alkyl-Heteroaryl, OSO₃H, OSO₂-Alkyl, OSO₂-Cycloalkyl, OSO₂-Heterocyclyl, OSO₂-Aryl, OSO₂-Heteroaryl, OSO₂-Alkyl-Aryl, OSO₂-Alkyl-Heteroaryl, OP(O)(OH)₂, C(O)-Alkyl, C(O)-Aryl, C(O)-Heteroaryl, CO₂H, CO₂-Alkyl, CO₂-Cycloalkyl, CO₂-Heterocyclyl, CO₂-Aryl, CO₂-Heteroaryl, CO₂-Alkyl-Cycloalkyl, CO₂-Alkyl-Heterocyclyl, CO₂-Alkyl-Aryl, CO₂-Alkyl-Heteroaryl, C(O)-NH₂, C(O)NH-Alkyl, C(O)NH-Cycloalkyl, C(O)NH-Heterocyclyl, C(O)NH-Aryl, C(O)NH-Heteroaryl, C(O)NH-Alkyl-Cycloalkyl, C(O)NH-Alkyl-Heterocyclyl, C(O)NH-Alkyl-Aryl, C(O)NH-Alkyl-Heteroaryl, C(O)N(Alkyl)₂, C(O)N(Cycloalkyl)₂, C(O)N(Aryl)₂, C(O)N(Heteroaryl)₂, SO-Alkyl, SO-Aryl, SO₂-Alkyl, SO₂-Aryl, SO₂NH₂, SO₂NH-Alkyl, SO₂NH-Aryl, SO₂NH-Heteroaryl, SO₂NH-Alkyl-Aryl, S03H, SO₂O-Alkyl, SO₂O-Aryl, SO₂O-Alkyl-Aryl, Alkyl, Cycloalkyl, Heterocyclyl, Aryl oder Heteroaryl, ein- oder mehrfach, gleich oder verschieden substituiert sein kann, und n den Wert 1, 2 oder 3 annehmen kann,
(vi) unsubstituiertes oder substituiertes Heteroaryl, wobei der Heteroarylrest F, CI, Br, I, CF₃, CN, NH₂, NH-Alkyl, NH-Cycloalkyl, NH-Heterocyclyl, NH-Aryl, NH-Heteroaryl, NH-Alkyl-Cycloalkyl, NH-Alkyl-Heterocyclyl, NH-Alkyl-Aryl, NH-Alkyl-Heteroaryl, NH-Alkyl-NH₂, NH-Alkyl-OH, N(Alkyl)₂, NHC(O)-Alkyl, NHC(O)-Cycloalkyl, NHC(O)-Heterocyclyl, NHC(O)-Aryl, NHC(O)-Heteroaryl, NHC(O)-Alkyl-Aryl, NHC(O)-Alkyl-Heteroaryl, NHSO₂-Alkyl, NHSO₂-Cycloalkyl, NHSO₂-Heterocyclyl, NHSO₂-Aryl, NHSO₂-Heteroaryl, NHSO₂-Alkyl-Aryl, NHSO₂-Alkyl-Heteroaryl, NO₂, SH, S-Alkyl, S-Aryl, S-Heteroaryl, OH, OCF₃, O-Alkyl, O-Cycloalkyl, O-Aryl, O-Heteroaryl, O-Alkyl-Cycloalkyl, 0-Alkyl-Heterocyclyl, O-Alkyl-Aryl, O-Alkyl-Heteroaryl, OC(O)-Alkyl, OC(O)-Cycloalkyl, OC(O)-Heterocyclyl, OC(O)-Aryl, OC(O)-Heteroaryl, OC(O)-Alkyl-Aryl, OC(O)-Alkyl-Heteroaryl, OSO₃H, OSO₂-Alkyl, OSO₂-Cycloalkyl, OSO₂-Heterocyclyl, OSO₂-Aryl, OSO₂-Heteroaryl, OSO₂-Alkyl-Aryl, OSO₂-Alkyl-Heteroaryl, OP(O)(OH)₂, C(O)-Alkyl, C(O)-Aryl, C(O)-Heteroaryl, CO₂H, CO₂-Alkyl, CO₂-Cycloalkyl, CO₂-Heterocyclyl, CO₂-Aryl, CO₂-Heteroaryl, CO₂-Alkyl-Cycloalkyl, CO₂-Alkyl-Heterocyclyl, CO₂-Alkyl-Aryl, CO₂-Alkyl-Heteroaryl, C(O)-NH₂, C(O)NH-Alkyl, C(O)NH-Cycloalkyl, C(O)NH-Heterocyclyl, C(O)NH-Aryl, C(O)NH-Heteroaryl, C(O)NH-Alkyl-Cycloalkyl, C(O)NH-Alkyl-Heterocyclyl, C(O)NH-Alkyl-Aryl, C(O)NH-Alkyl-Heteroaryl, C(O)N(Alkyl)₂, C(O)N(Cycloalkyl)₂, C(O)N(Aryl)₂, C(O)N(Heteroaryl)₂, SO₂NH₂, SO₂NH-Alkyl, SO₂NH-Aryl, SO₂NH-Heteroaryl, SO₂NH-Alkyl-Aryl, SO₃H, SO₂O-Alkyl, SO₂O-Aryl, SO₂O-Alkyl-Aryl, Alkyl, Cycloalkyl, Heterocyclyl, Aryl oder Heteroaryl, ein- oder mehrfach, gleich oder verschieden substituiert sein kann,
(vii) -C(O)-R17, wobei R17 Alkyl, Aryl oder Heteroaryl sein kann, und die Alkyl und Arylsubstituenten ihrerseits wiederum substituiert sein können,
(viii) oder R11 und R12 zusammen Cycloalkyl oder Heterocyclyl bedeuten können,
-C(Y)NR13R14 bedeuten kann, wobei Y = NH und R13 und R14 unabhängig voneinander
(i) Wasserstoff,
(ii) unsubstituiertes oder substituiertes Alkyl, wobei der Alkylrest mit F, Cl, Br, I, CF₃, CN, NH₂, NH-Alkyl, NH-Cycloalkyl, NH-Heterocyclyl, NH-Aryl, NH-Heteroaryl, NH-Alkyl-Aryl, NH-Alkyl-Heteroaryl, N(Alkyl)₂, NHC(O)-Alkyl, NHC(O)-Cycloalkyl, NHC(O)-Heterocyclyl, NHC(O)-Aryl, NHC(O)-Heteroaryl, NHSO₂-Alkyl, NHSO₂-Cycloalkyl, NHSO₂-Aryl, NHSO₂-Heteroaryl, NO₂, SH, S-Alkyl, S-Cycloalkyl, S-Heterocyclyl, S-Aryl, S-Heteroaryl, OH, OCF₃, O-Alkyl, O-Cycloalkyl, O-Heterocyclyl, O-Aryl, O-Heteroaryl, O-Alkyl-Cycloalkyl, 0-Alkyl-Aryl, O-Alkyl-Heteroaryl, OC(O)-Alkyl, OC(O)-Cycloalkyl, OC(O)-Heterocyclyl, OC(O)-Aryl, OC(O)-Heteroaryl, OSO₂-Alkyl, OSO₂-Cycloalkyl, OSO₂-Aryl, OSO₂-Heteroaryl, C(O)-Alkyl, C(O)-Aryl, CO₂H, CO₂-Alkyl, CO₂-Cycloalkyl, CO₂-Heterocyclyl, CO₂-Aryl, CO₂-Heteroaryl, CO₂-Alkyl-Cycloalkyl, CO₂-Alkyl-Heterocyclyl, CO₂-Alkyl-Aryl, CO₂-Alkyl-Heteroaryl, C(O)-NH₂, C(O)NH-Alkyl, C(O)NH-Cycloalkyl, C(O)NH-Heterocyclyl, C(O)NH-Aryl, C(O)NH-Heteroaryl, C(O)NH-Alkyl-Cycloalkyl, C(O)NH-Alkyl-Heterocyclyl, C(O)NH-Alkyl-Aryl, C(O)NH-Alkyl-Heteroaryl, C(O)N(Alkyl)₂, C(O)N(Cycloalkyl)₂, C(O)N(Aryl)₂, C(O)N(Heteroaryl)₂, SO-Alkyl, SO-Aryl, SO₂-Alkyl, SO₂-Aryl, SO₂NH₂, SO₃H, Alkyl, Cycloalkyl, Heterocyclyl, Aryl oder Heteroaryl, ein- oder mehrfach, gleich oder verschieden substituiert sein kann,
(iii) unsubstituiertes oder substituiertes Cycloalkyl, wobei der Cycloalkylrest mit F, Cl, Br, I, NH₂, NH-Alkyl, NH-Cycloalkyl, NH-Heterocyclyl, NH-Aryl, NH-Heteroaryl, NH-Alkyl-Aryl, NH-Alkyl-Heteroaryl, N(Alkyl)₂, NHC(O)-Alkyl, NHC(O)-Cycloalkyl, NHC(O)-Heterocyclyl, NHC(O)-Aryl, NHC(O)-Heteroaryl, NHSO₂-Alkyl, NHSO₂-Cycloalkyl, NHSO₂-Aryl, NHSO₂-Heteroaryl, OH, 0-Alkyl, O-Cycloalkyl, O-Heterocyclyl, O-Aryl, O-Heteroaryl, O-Alkyl-Aryl, 0-Alkyl-Heteroaryl, OC(O)-Alkyl, OC(O)-Cycloalkyl, OC(O)-Heterocyclyl, OC(O)-Aryl, OC(O)-Heteroaryl, OSO₂-Alkyl, OSO₂-Cycloalkyl, OSO₂-Aryl, OSO₂-Heteroaryl, CO₂H, CO₂-Alkyl, CO₂-Cycloalkyl, CO₂-Heterocyclyl, CO₂-Aryl, CO₂-Heteroaryl, C(O)-NH₂, C(O)NH-Alkyl, C(O)NH-Cycloalkyl, C(O)NH-Heterocyclyl, C(O)NH-Aryl, C(O)NH-Heteroaryl, C(O)NH-Alkyl-Aryl, C(O)NH-Alkyl-Heteroaryl, C(O)N(Alkyl)₂, Alkyl, oder Aryl, ein- oder mehrfach, gleich oder verschieden substituiert sein kann,
(iv) unsubstituiertes oder substituiertes Heterocyclyl, wobei der Heterocyclylrest mit OH, O-Alkyl, O-Aryl, NH₂, NH-Alkyl, NH-Aryl, Alkyl, oder Aryl ein- oder mehrfach, gleich oder verschieden substituiert sein kann,
(v) unsubstituiertes oder substituiertes Aryl, wobei der Arylrest mit F, Cl, Br, I, CF₃, CN, NH₂, NH-Alkyl, NH-Cycloalkyl, NH-Heterocyclyl, NH-Aryl, NH-Heteroaryl, NH-Alkyl-Cycloalkyl, NH-Alkyl-Heterocyclyl, NH-Alkyl-Aryl, NH-Alkyl-Heteroaryl, NH-Alkyl-NH₂, NH-Alkyl-OH, N(Alkyl)₂, NHC(O)-Alkyl, NHC(O)-Cycloalkyl, NHC(O)-Heterocyclyl, NHC(O)-Aryl, NHC(O)-Heteroaryl, NHSO₂-Alkyl, NHSO₂-Aryl, NHSO₂-Heteroaryl, NO₂, SH, S-Alkyl, S-Cycloalkyl, . S-Heterocyclyl, S-Aryl, S-Heteroaryl, OH, OCF₃, O-Alkyl, O-Cycloalkyl, 0-Heterocyclyl, O-Aryl, O-Heteroaryl, O-Alkyl-Cycloalkyl, O-Alkyl-Heterocyclyl, O-Alkyl-Aryl, O-Alkyl-Heteroaryl, O-Alkyl-OH, O-(CH₂)ₙ-O, OC(O)-Alkyl, OC(O)-Cycloalkyl, OC(O)-Heterocyclyl, OC(O)-Aryl, OC(O)-Heteroaryl, OSO₂-Alkyl, OSO₂-Cycloalkyl, OSO₂-Aryl, OSO₂-Heteroaryl, C(O)-Alkyl, C(O)-Aryl, C(O)-Heteroaryl, CO₂H, CO₂-Alkyl, CO₂-Cycloalkyl, CO₂-Heterocyclyl, CO₂-Aryl, CO₂-Heteroaryl, CO₂-Alkyl-Cycloalkyl, CO₂-Alkyl-Heterocyclyl, CO₂-Alkyl-Aryl, CO₂-Alkyl-Heteroaryl, C(O)-NH₂, C(O)NH-Alkyl, C(O)NH-Cycloalkyl, C(O)NH-Heterocyclyl, C(O)NH-Aryl, C(O)NH-Heteroaryl, C(O)NH-Alkyl-Cycloalkyl, C(O)NH-Alkyl-Heterocyclyl, C(O)NH-Alkyl-Aryl, C(O)NH-Alkyl-Heteroaryl, C(O)N(Alkyl)₂, C(O)N(Cycloalkyl)₂, C(O)N(Aryl)₂, C(O)N(Heteroaryl)₂, SO-Alkyl, SO-Aryl, SO₂-Alkyl, SO₂-Aryl, SO₂NH₂, SO₂NH-Alkyl, SO₂NH-Aryl, SO₂NH-Heteroaryl, S03H, SO₂O-Alkyl, SO₂O-Aryl; SO₂O-Heteroaryl, Alkyl, Cycloalkyl, Heterocyclyl, Aryl oder Heteroaryl, ein- oder mehrfach, gleich oder verschieden substituiert sein kann, und n den Werft 1, 2 oder 3 annehmen kann,
(vi) unsubstituiertes oder substituiertes Heteroaryl, wobei der Heteroarylrest mit F, Cl, Br, I, CF₃, CN, NH₂, NH-Alkyl, NH-Cycloalkyl, NH-Heterocyclyl, NH-Aryl, NH-Heteroaryl, NH-Alkyl-Aryl, NH-Alkyl-Heteroaryl, N(Alkyl)₂, NHC(O)-Alkyl, NHC(O)-Cycloalkyl, NHC(O)-Heterocyclyl, NHC(O)-Aryl, NHC(O)-Heteroaryl, NHSO₂-Alkyl, NHSO₂-Aryl, NHSO₂-Heteroaryl, NO₂, SH, S-Alkyl, S-Aryl, OH, OCF₃, O-Alkyl, O-Cycloalkyl, O-Heterocyclyl, O-Aryl, O-Heteroaryl, OC(O)-Alkyl, OC(O)-Cycloalkyl, OC(O)-Heterocyclyl, OC(O)-Aryl, OC(O)-Heteroaryl, OSO₂-Alkyl, OSO₂-Cycloalkyl, OSO₂-Aryl, OSO₂-Heteroaryl, C(O)-Alkyl, C(O)-Aryl, C(O)-Heteroaryl, CO₂H, CO₂-Alkyl, CO₂-Cycloalkyl, CO₂-Heterocyclyl, CO₂-Aryl, CO₂-Heteroaryl, CO₂-Alkyl-Cycloalkyl, CO₂-Alkyl-Heterocyclyl, CO₂-Alkyl-aryl, CO₂-Alkyl-Heteroaryl, C(O)-NH₂, C(O)NH-Alkyl, C(O)NH-Cycloalkyl, C(O)NH-Heterocyclyl, C(O)NH-Aryl, C(O)NH-Heteroaryl, C(O)NH-Alkyl-Cycloalkyl, C(O)NH-Alkyl-Heterocyclyl, C(O)NH-Alkyl-Aryl, C(O)NH-Alkyl-Heteroaryl, C(O)N(Alkyl)₂, C(O)N(Cycloalkyl)₂, C(O)N(Aryl)₂, C(O)N(Heteroaryl)₂, SO₂-Alkyl, SO₂-Aryl, SO₂NH₂, SO₂NH-Alkyl, SO₂NH-Aryl, SO₂NH-Heteroaryl, S03H, SO₂O-Alkyl, SO₂O-Aryl, SO₂O-Heteroaryl, Alkyl, Cycloalkyl, Heterocyclyl, Aryl oder Heteroaryl, ein- oder mehrfach, gleich oder verschieden substituiert sein kann,
(vii) oder R13 und R14 zusammen Cycloalkyl oder Heterocyclyl bedeuten können,
-C(NR15)R16 bedeuten kann, wobei R15 = H und R16
(i) unsubstituiertes oder substituiertes Alkyl, wobei der Alkylrest mit F, Cl, Br, I, CF₃, NH₂, NH-Alkyl, NH-Cycloalkyl, NH-Heterocyclyl, NH-Aryl, NH-Heteroaryl, NH-Alkyl-Aryl, NH-Alkyl-Heteroaryl, N(Alkyl)₂, NHC(O)-Alkyl, NHC(O)-Cycloalkyl, NHC(O)-Heterocyclyl, NHC(O)-Aryl, NHC(O)-Heteroaryl, NHSO₂-Alkyl, NHSO₂-Cycloalkyl, NHSO₂-Aryl, NHSO₂-Heteroaryl, NO₂, SH, S-Alkyl, S-Cycloalkyl, S-Heterocyclyl, S-Aryl, S-Heteroaryl, OH, OCF₃, O-Alkyl, 0-Cycloalkyl, O-Heterocyclyl, O-Aryl, O-Heteroaryl, O-Alkyl-Cycloalkyl, O-Alkyl-Aryl, O-Alkyl-Heteroaryl, OC(O)-Alkyl, OC(O)-Cycloalkyl, OC(O)-Heterocyclyl, OC(O)-Aryl, OC(O)-Heteroaryl, OSO₂-Alkyl, OSO₂-Cycloalkyl, OSO₂-Aryl, OSO₂-Heteroaryl, C(O)-Alkyl, C(O)-Aryl, CO₂H, CO₂-Alkyl, CO₂-Cycloalkyl, CO₂-Heterocyclyl, CO₂-Aryl, CO₂-Heteroaryl, CO₂-Alkyl-Cycloalkyl, CO₂-Alkyl-Heterocyclyl, CO₂-Alkyl-Aryl, CO₂-Alkyl-Heteroaryl, C(O)-NH₂, C(O)NH-Alkyl, C(O)NH-Cycloalkyl, C(O)NH-Heterocyclyl, C(O)NH-Aryl, C(O)NH-Heteroaryl, C(O)NH-Alkyl-Cycloalkyl, C(O)NH-Alkyl-Heterocyclyl, C(O)NH-Alkyl-Aryl, C(O)NH-Alkyl-Heteroaryl, C(O)N(Alkyl)₂, C(O)N(Cycloalkyl)₂, C(O)N(Aryl)₂, C(O)N(Heteroaryl)₂, SO-Alkyl, SO-Aryl, SO₂-Alkyl, SO₂-Aryl, SO₂NH₂, SO₃H, Alkyl, Cycloalkyl, Heterocyclyl, Aryl oder Heteroaryl, ein- oder mehrfach, gleich oder verschieden substituiert sein kann,
(ii) unsubstituiertes oder substituiertes Cycloalkyl, wobei der Cycloalkylrest mit F, CI, Br, I, NH₂, NH-Alkyl, NH-Cycloalkyl, NH-Heterocyclyl, NH-Aryl, NH-Heteroaryl, NH-Alkyl-Aryl, NH-Alkyl-Heteroaryl, N(Alkyl)₂, NHC(O)-Alkyl, NHC(O)-Cycloalkyl, NHC(O)-Heterocyclyl, NHC(O)-Aryl, NHC(O)-Heteroaryl, NHSO₂-Alkyl, NHSO₂-Cycloalkyl, NHSO₂-Aryl, NHSO₂-Heteroaryl, OH, 0-Alkyl, O-Cycloalkyl, O-Heterocyclyl, O-Aryl, O-Heteroaryl, O-Alkyl-Aryl, 0-Alkyl-Heteroaryl, OC(O)-Alkyl, OC(O)-Cycloalkyl, OC(O)-Heterocyclyl, OC(O)-Aryl, OC(O)-Heteroaryl, OSO₂-Alkyl, OSO₂-Cycloalkyl, OSO₂-Aryl, OSO₂-Heteroaryl, CO₂H, CO₂-Alkyl, CO₂-Cycloalkyl, CO₂-Heterocyclyl, CO₂-Aryl, CO₂-Heteroaryl, C(O)-NH₂, C(O)NH-Alkyl, C(O)NH-Cycloalkyl, C(O)NH-Heterocyclyl, C(O)NH-Aryl, C(O)NH-Heteroaryl, C(O)NH-Alkyl-Aryl, C(O)NH-Alkyl-Heteroaryl, C(O)N(Alkyl)₂, Alkyl, oder Aryl, ein- oder mehrfach, gleich oder verschieden substituiert sein kann,
(iii) unsubstituiertes oder substituiertes Heterocyclyl, wobei der Heterocyclylrest mit OH, O-Alkyl, O-Aryl, NH₂, NH-Alkyl, NH-Aryl, Alkyl oder Aryl ein- oder mehrfach, gleich oder verschieden substituiert sein kann,
(iv) unsubstituiertes oder substituiertes Aryl, wobei der Arylrest mit F, Cl, Br, I, CF₃, NH₂, NH-Alkyl, NH-Cycloalkyl, NH-Heterocyclyl, NH-Aryl, NH-Heteroaryl, NH-Alkyl-Cycloalkyl, NH-Alkyl-Heterocyclyl, NH-Alkyl-Aryl, NH-Alkyl-Heteroaryl, NH-Alkyl-NH₂, NH-Alkyl-OH, N(Alkyl)₂, NHC(O)-Alkyl, NHC(O)-Cycloalkyl, NHC(O)-Heterocyclyl, NHC(O)-Aryl, NHC(O)-Heteroaryl, NHSO₂-Alkyl, NHSO₂-Aryl, NHSO₂-Heteroaryl, NO₂, SH, S-Alkyl, S-Cycloalkyl, S-Heterocyclyl, S-Aryl, S-Heteroaryl, OH, OCF₃, O-Alkyl, O-Cycloalkyl, O-Heterocyclyl, O-Aryl, O-Heteroaryl, O-Alkyl-Cycloalkyl, O-Alkyl-Heterocyclyl, O-Alkyl-Aryl, O-Alkyl-Heteroaryl, O-Alkyl-OH, O-(CH₂)ₙ-O, OC(O)-Alkyl, OC(O)-Cycloalkyl, OC(O)-Heterocyclyl, OC(O)-Aryl, OC(O)-Heteroaryl, OSO₂-Alkyl, OSO₂-Cycloakyl, OSO₂-Aryl, OSO₂-Heteroaryl, C(O)-Alkyl, C(O)-Aryl, C(O)-Heteroaryl, CO₂H, CO₂-Alkyl, CO₂-Cycloalkyl, CO₂-Heterocyclyl, CO₂-Aryl, CO₂-Heteroaryl, CO₂-Alkyl-Cycloalkyl, CO₂-Alkyl-Heterocyclyl, CO₂-Alkylaryl, CO₂-Alkyl-Heteroaryl, C(O)-NH₂, C(O)NH-Alkyl, C(O)NH-Cycloalkyl, C(O)NH-Heterocyclyl, C(O)NH-Aryl, C(O)NH-Heteroaryl, C(O)NH-Alkyl-Cycloalkyl, C(O)NH-Alkyl-Heterocyclyl, C(O)NH-Alkyl-Aryl, C(O)NH-Alkyl-Heteroaryl, C(O)N(Alkyl)₂, C(O)N(Cycloalkyl)₂, C(O)N(Aryl)₂, C(O)N(Heteroaryl)₂, SO-Alkyl, SO-Aryl, SO₂-Alkyl, SO₂-Aryl, SO₂NH₂, SO₂NH-Alkyl, SO₂NH-Aryl, SO₂NH-Heteroaryl, SO3H, SO₂O-Alkyl, SO₂O-Aryl, SO₂O-Heteroaryl, Alkyl, Cycloalkyl, Heterocyclyl, Aryl oder Heteroaryl, ein- oder mehrfach, gleich oder verschieden substituiert sein kann, und n den Wert 1, 2 oder 3 annehmen kann,
(v) unsubstituiertes oder substituiertes Heteroaryl, wobei der Heteroarylrest mit F, Cl, Br, I, CF₃, NH₂, NH-Alkyl, NH-Cycloalkyl, NH-Heterocyclyl, NH-Aryl, NH-Heteroaryl, NH-Alkyl-Aryl, NH-Alkyl-Heteroaryl, N(Alkyl)₂, NHC(O)-Alkyl, NHC(O)-Cycloalkyl, NHC(O)-Heterocyclyl, NHC(O)-Aryl, NHC(O)-Heteroaryl, NHSO₂-Alkyl, NHSO₂-Aryl, NHSO₂-Heteroaryl, NO₂, SH, S-Alkyl, S-Aryl, OH, OCF₃, O-Alkyl, O-Cycloalkyl, O-Heterocyclyl, O-Aryl, O-Heteroaryl, OC(O)-Alkyl, OC(O)-Cycloalkyl, OC(O)-Heterocyclyl, OC(O)-Aryl, OC(O)-Heteroaryl, OSO₂-Alkyl, OSO₂-Cycloalkyl, OSO₂-Aryl, OSO₂-Heteroaryl, C(O)-Alkyl, C(O)-Aryl, C(O)-Heteroaryl, CO₂H, CO₂-Alkyl, CO₂-Cycloalkyl, CO₂-Heterocyclyl, CO₂-Aryl, CO₂-Heteroaryl, CO₂-Alkyl-Cycloalkyl, CO₂-Alkyl-Heterocyclyl, CO₂-Alkyl-aryl, CO₂-Alkyl-Heteroaryl, C(O)-NH₂, C(O)NH-Alkyl, C(O)NH-Cycloalkyl, C(O)NH-Heterocyclyl, C(O)NH-Aryl, C(O)NH-Heteroaryl, C(O)NH-Alkyl-Cycloalkyl, C(O)NH-Alkyl-Heterocyclyl, C(O)NH-Alkyl-Aryl, C(O)NH-Alkyl-Heteroaryl, C(O)N(Alkyl)₂, C(O)N(Cycloalkyl)₂, C(O)N(Aryl)₂, C(O)N(Heteroaryl)₂, SO₂-Alkyl, SO₂-Aryl, SO₂NH₂, SO₂NH-Alkyl, SO₂NH-Aryl, SO₂NH-Heteroaryl, SO3H, SO₂O-Alkyl, SO₂O-Aryl, SO₂O-Heteroaryl, Alkyl, Cycloalkyl, Heterocyclyl, Aryl oder Heteroaryl, ein- oder mehrfach, gleich oder verschieden substituiert sein kann, bedeuten können.

Der Ausdruck "Alkyl" umfasst im Sinne dieser Erfindung acyclische gesättigte oder ungesättigte Kohlenwasserstoffreste, die verzweigt oder geradkettig sein können, mit 1 bis 8 C-Atomen, d.h. C₁-₈-Alkanyle, C₂-₈-Alkenyle und C₂-₈-Alkinyle. Dabei weisen Alkenyle mindestens eine C-C-Doppelbindung und Alkinyle mindestens eine C-C-Dreifachbindung auf. Es ist bevorzugt, dass der Alkylrest ausgewählt ist aus der Gruppe, die Methyl, Ethyl, *n*-Propyl, 2-Propyl, *n*-Butyl, *sec*.-Butyl, *tert*.-Butyl, *n*-Pentyl, *iso*-Pentyl, *neo*-Pentyl, *n*-Hexyl, 2-Hexyl, *n*-Octyl, Ethylenyl (Vinyl), Ethinyl, Propenyl (-CH₂CH=CH₂; -CH=CH-CH₃, -C(=CH₂)-CH₃), Propinyl (-CH₂-C≡CH, -C≡C-CH₃), Butenyl, Butinyl, Pentenyl, Pentinyl, Hexenyl, Hexinyl, Heptenyl, Heptinyl, Octenyl und Octinyl enthält.

Der Ausdruck "Cycloalkyl" bedeutet für die Zwecke dieser Erfindung cyclische Kohlenwasserstoffe mit 3-12 Kohlenstoffen, die gesättigt oder ungesättigt sein können. Die Bindung an die Verbindungen der allgemeinen Struktur 1 kann über jedes beliebige und mögliche Ringglied des Cycloalkyl-Restes erfolgen. Der Cycloalkyl-Rest kann auch Teil eines bi- oder polycyclischen Systems sein.

Der Ausdruck "Heterocyclyl" steht für einen 3-, 4-, 5-, 6-, 7- oder 8-gliedrigen cyclischen organischen Rest, der mindestens 1, ggf. 2, 3, 4 oder 5 Heteroatome enthält, wobei die Heteroatome gleich oder verschieden sind und der cyclische Rest gesättigt oder ungesättigt, aber nicht aromatisch ist. Die Bindung an die Verbindungen der allgemeinen Struktur I kann über jedes beliebige und mögliche Ringglied des Heterocyclyl-Restes erfolgen. Der Heterocyclus kann auch Teil eines bi- oder polycyclischen Systems sein. Bevorzugte Heteroatome sind Stickstoff, Sauerstoff und Schwefel. Es ist bevorzugt, dass der Heterocyclyl-Rest ausgewählt ist aus der Gruppe, die Tetrahydrofuryl, Tetrahydropyranyl, Pyrrolidinyl, Piperidinyl, Piperazinyl und Morpholinyl enthält.

Der Ausdruck "Aryl" bedeutet im Sinne dieser Erfindung aromatische Kohlenwasserstoffe mit 6 bis 14 C-Atomen, u.a. Phenyle, Naphthyle und Anthracenyle. Die Reste können auch mit weiteren gesättigten, (partiell) ungesättigten oder aromatischen Ringsystemen kondensiert sein. Die Bindung an die Verbindungen der allgemeinen Struktur I kann über jedes beliebige und mögliche Ringglied des Aryl-Restes erfolgen.

Der Ausdruck "Heteroaryl" steht für einen 5-, 6- oder 7-gliedrigen cyclischen aromatischen Rest, der mindestens 1, ggf. auch 2, 3, 4 oder 5 Heteroatome enthält, wobei die Heteroatome gleich oder verschieden sind. Die Bindung an die Verbindungen der allgemeinen Struktur I kann über jedes beliebige und mögliche Ringglied des Heteroaryl-Restes erfolgen. Der Heterocyclus kann auch Teil eines bi- oder polycyclischen Systems sein. Bevorzugte Heteroatome sind Stickstoff, Sauerstoff und Schwefel. Es ist bevorzugt, dass der Heteroaryl-Rest ausgewählt ist aus der Gruppe, die Pyrrolyl, Furyl, Thienyl, Thiazolyl, Oxazolyl, Isoxazolyl, Pyrazolyl, Imidazolyl, Pyridinyl, Pyrimidinyl, Pyridazinyl, Pyrazinyl, Phthalazinyl, Indolyl; Indazolyl, Indolizinyl, Chinolinyl, Isochinolinyl, Chinoxalinyl, Chinazolinyl, Carbazolyl, Phenazinyl, Phenothiazinyl, Acridinyl enthält.

Die Ausdrücke "Alkyl-Cycloalkyl", "Alkyl-Heterocyclyl", "Alkyl-Aryl" oder "Alkyl-Heteroaryl" bedeuten für die Zwecke der vorliegenden Erfindung, daß Alkyl, Cycloalkyl, Heterocyclyl, Aryl und Heteroaryl die oben definierten Bedeutungen haben und der Cycloalkyl-, Heterocyclyl-, Aryl- und Heteroaryl-Rest über eine C₁₋₈-Alkyl-Gruppe an die Verbindungen der allgemeinen Struktur 1 gebunden ist.

Im Zusammenhang mit "Alkyl", "Cycloalkyl", "Heterocyclyl", "Aryl", "Heteroaryl", "Alkyl-Cycloalkyl", "Alkyl-Heterocyclyl", "Alkyl-Aryl" und "Alkyl-Heteroaryl" versteht man unter dem Begriff substituiert im Sinne dieser Erfindung, insofern oben in der Beschreibung oder den Ansprüchen nicht explicit definiert, die Substitution eines oder mehrerer Wasserstoffreste durch F, Cl, Br, I, CN, CF₃, NH₂, NH-Alkyl, NH-Aryl, N(Alkyl)₂, NO₂, SH, S-Alkyl, OH, OCF₃, O-Alkyl, O-Aryl, OSO₃H, OP(O)(OH)₂, CHO, CO₂H, SO₃H oder Alkyl. Die Substituenten können gleich oder verschieden sein und die Substitution kann in jeder beliebigen und möglichen Position des Alkyl-, Cycloalkyl-, Heterocyclyl-, Aryl- und Heteroarylrestes vorkommen.

Unter mehrfach substituierten Resten sind solche zu verstehen, die entweder an verschiedenen oder an gleichen Atomen mehrfach, z. B. zwei- oder dreifach substituiert sind, beispielsweise dreifach am gleichen C-Atom wie im Falle von CF₃, -CH₂CF₃ oder an verschiedenen Stellen wie im Falle von -CH(OH)-CH=CH-CHCl₂. Die Mehrfachsubstitution kann mit dem gleichen oder verschiedenen Substituenten erfolgen.

Sofern die erfindungsgemäßen Verbindungen der allgemeinen Formel I mindestens ein Asymmetriezentrum aufweisen, können sie in Form ihrer Racemate, in Form der reinen Enantiomeren und/oder Diastereomeren oder in Form von Mischungen dieser Enantiomeren und/oder Diastereomeren vorliegen. Die Mischungen können in jedem beliebigen Mischungsverhältnis der Stereoisomeren vorliegen.

So lassen sich beispielsweise die erfindungsgemäßen Verbindungen gemäß der allgemeinen Formel 1, welche ein oder mehrere Chiralitätszentren aufweisen und die als Racemate auftreten, nach an sich bekannten Methoden in ihre optischen Isomeren, also Enantiomere oder Diastereomere auftrennen. Die Trennung kann durch Säulentrennung an chiralen Phasen oder durch Umkristallisation aus einem optisch aktiven Lösungsmittel oder unter Verwendung einer optisch aktiven Säure oder Base oder durch Derivatisierung mit einem optisch aktiven Reagenz, wie beispielsweise einem optisch aktiven Alkohol, und anschließender Abspaltung des Restes erfolgen.

Sofern möglich, können die erfindungsgemäßen Verbindungen in Form der Tautomeren vorliegen.

Die erfindungsgemäßen Verbindungen der allgemeinen Formel 1 können, falls sie eine ausreichend basische Gruppe, wie zum Beispiel ein primäres, sekundäres oder tertiäres Amin besitzen, mit anorganischen und organischen Säuren in ihre physiologisch verträglichen Salze überführt werden. Vorzugsweise werden die pharmazeutisch annehmbaren Salze der erfindungsgemäßen Verbindungen gemäß der allgemeinen Struktur I mit Salzsäure, Bromwasserstoffsäure, Schwefelsäure, Phosphorsäure, Methansulfonsäure, p-Toluolsulfonsäure, Kohlensäure, Ameisensäure, Essigsäure, Trifluoressigsäure, Sulfoessigsäure, Oxalsäure, Malonsäure, Maleinsäure, Bernsteinsäure, Weinsäure, Traubensäure, Äpfelsäure, Embonsäure, Mandelsäure, Fumarsäure, Milchsäure, Citronensäure, Glutaminsäure oder Asparaginsäure gebildet. Bei den gebildeten Salzen handelt es sich u.a. um Hydrochloride, Hydrobromide, Sulfate, Hydrogensulfate, Phosphate, Methansulfonate, Tosylate, Carbonate, Hydrogencarbonate, Formiate, Acetate, Triflate, Sulfoacetate, Oxalate, Malonate, Maleate, Succinate, Tartrate, Malate, Embonate, Mandelate, Fumarate, Lactate, Citrate, Glutaminate und Aspartate. Die Stöchiometrie der gebildeten Salze der erfindungsgemäßen Verbindungen kann dabei ganzzahlige oder nicht ganzzahlige Vielfache von eins betragen.

Die erfindungsgemäßen Verbindungen der allgemeinen Formel I können, falls sie eine ausreichend saure Gruppe, wie zum Beispiel die Carboxygruppe enthalten, mit anorganischen und organischen Basen in ihre physiologisch verträglichen Salze überführt werden. Als anorganische Basen kommen beispielsweise Natriumhydroxid, Kaliumhydroxid, Calciumhydroxid, als organische Basen Ethanolamin, Diethanolamin, Triethanolamin, Cyclohexylamin, Dibenzylethylendiamin und Lysin in Betracht. Die Stöchiometrie der gebildeten Salze der erfindungsgemäßen Verbindungen kann dabei ganzzahlige oder nicht ganzzahlige Vielfache von eins betragen.

Ebenfalls bevorzugt sind Solvate und insbesondere Hydrate der erfindungsgemäßen Verbindungen, die z. B. durch Kristallisation aus einem Lösungsmittel oder aus wässriger Lösung erhalten werden können. Es können sich dabei ein, zwei, drei oder beliebig viele Solvat- oder Wasser-Moleküle mit den erfindungsgemäßen Verbindungen zu Solvaten und Hydraten verbinden.

Es ist bekannt, dass chemische Substanzen Festkörper ausbilden, die in verschiedenen Ordnungszuständen vorliegen, die man als polymorphe Formen oder Modifikationen bezeichnet. Die verschiedenen Modifikationen einer polymorphen Substanz können sich in ihren physikalischen Eigenschaften stark unterscheiden. Die erfindungsgemäßen Verbindungen der allgemeinen Formel I können in verschiedenen polymorphen Formen vorliegen, dabei können bestimmte Modifikationen metastabil sein.

Die Verfahren zur Herstellung erfindungsgemäßer substituierter Pyrido[2,3-b]pyrazine werden nachstehend erläutert.

Die Verbindungen der allgemeinen Formel I sind gemäß der folgenden Schemata (Schema 1 - 5) erhältlich:

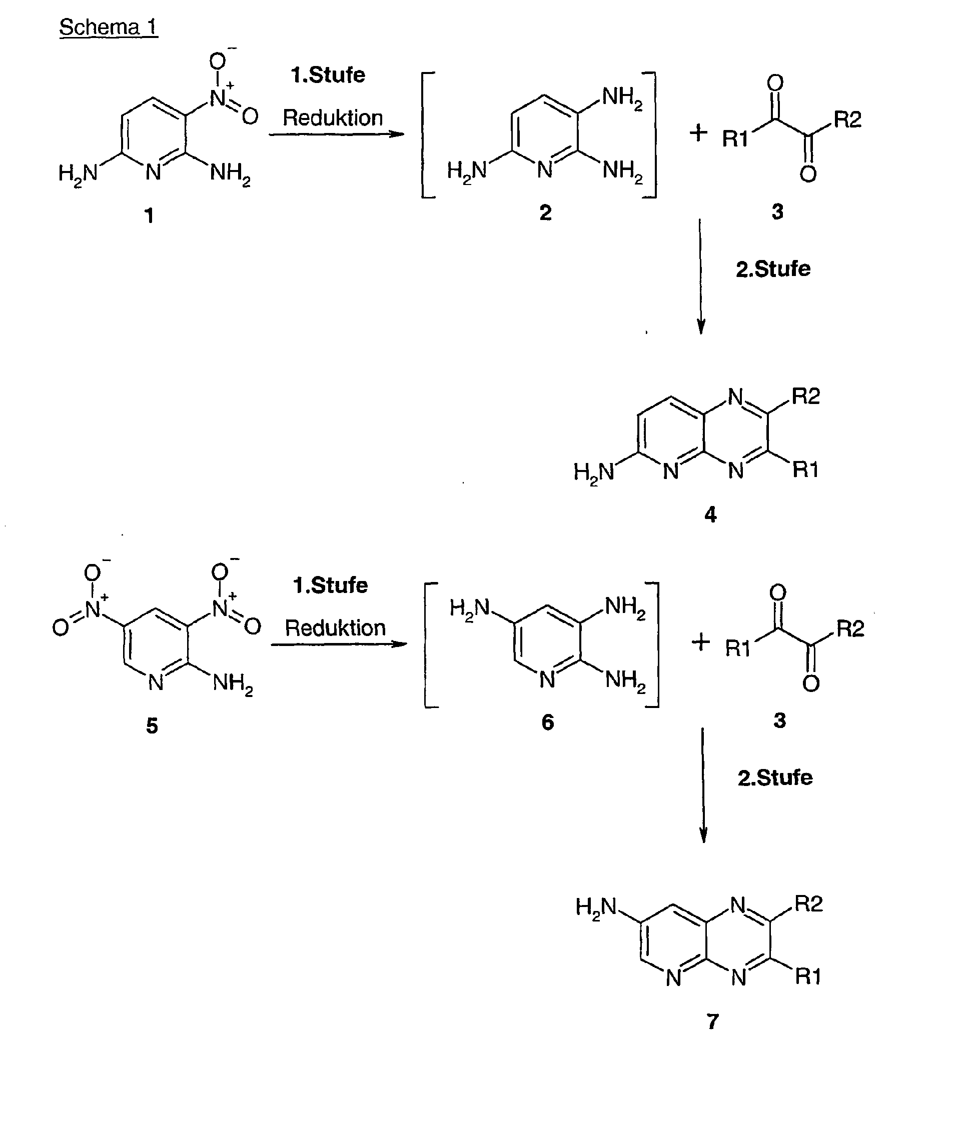

Vorstufen für ausgewählte Beispiele der erfindungsgemäßen Pyrido[2,3-b]pyrazine gemäß der allgemeinen Formel I, bei denen die Substituenten R1 und/oder R2 die Reste OR5, SR6, NR7R8 sein sollen, sind beispielsweise nach dem Verfahren in Schema 2 bzw. einem entsprechenden, dem Fachmann bekannten Verfahren erhältlich.

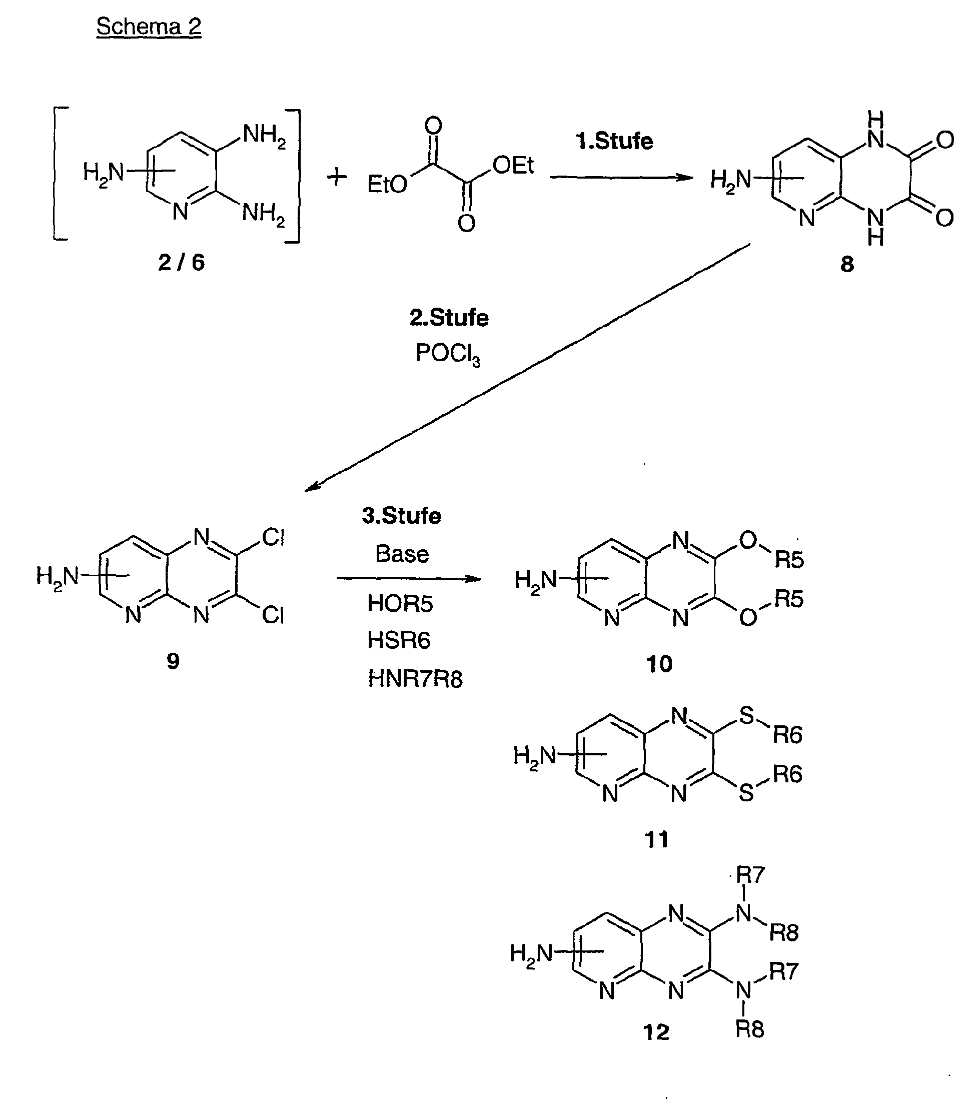

Vorstufen für ausgewählte Beispiele der erfindungsgemäßen Pyrido[2,3-b]pyrazine gemäß der allgemeinen Formel I, bei denen der Substituent R9 # H sein soll, sind beispielsweise nach dem Verfahren in Schema 3 erhältlich.

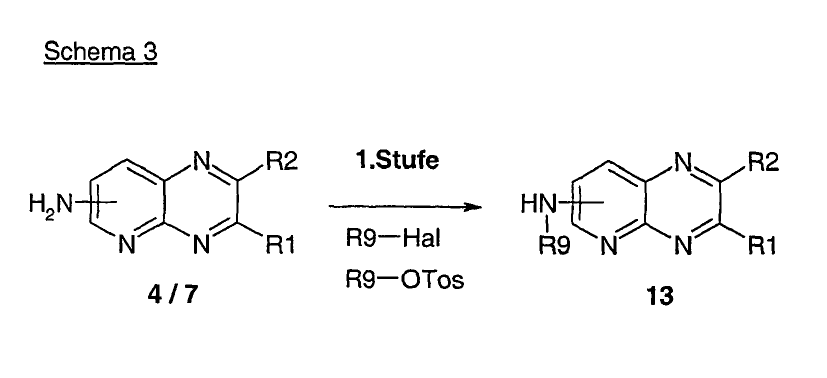

Die Umsetzung der Vorstufen **4, 7,** und **13** aus den Schemata 1-3 zu den erfindungsgemäßen substituierten Pyrido[2,3-b]pyrazinen gemäß der allgemeinen Formel I kann beispielsweise nach den Verfahren in Schema 4 erfolgen.

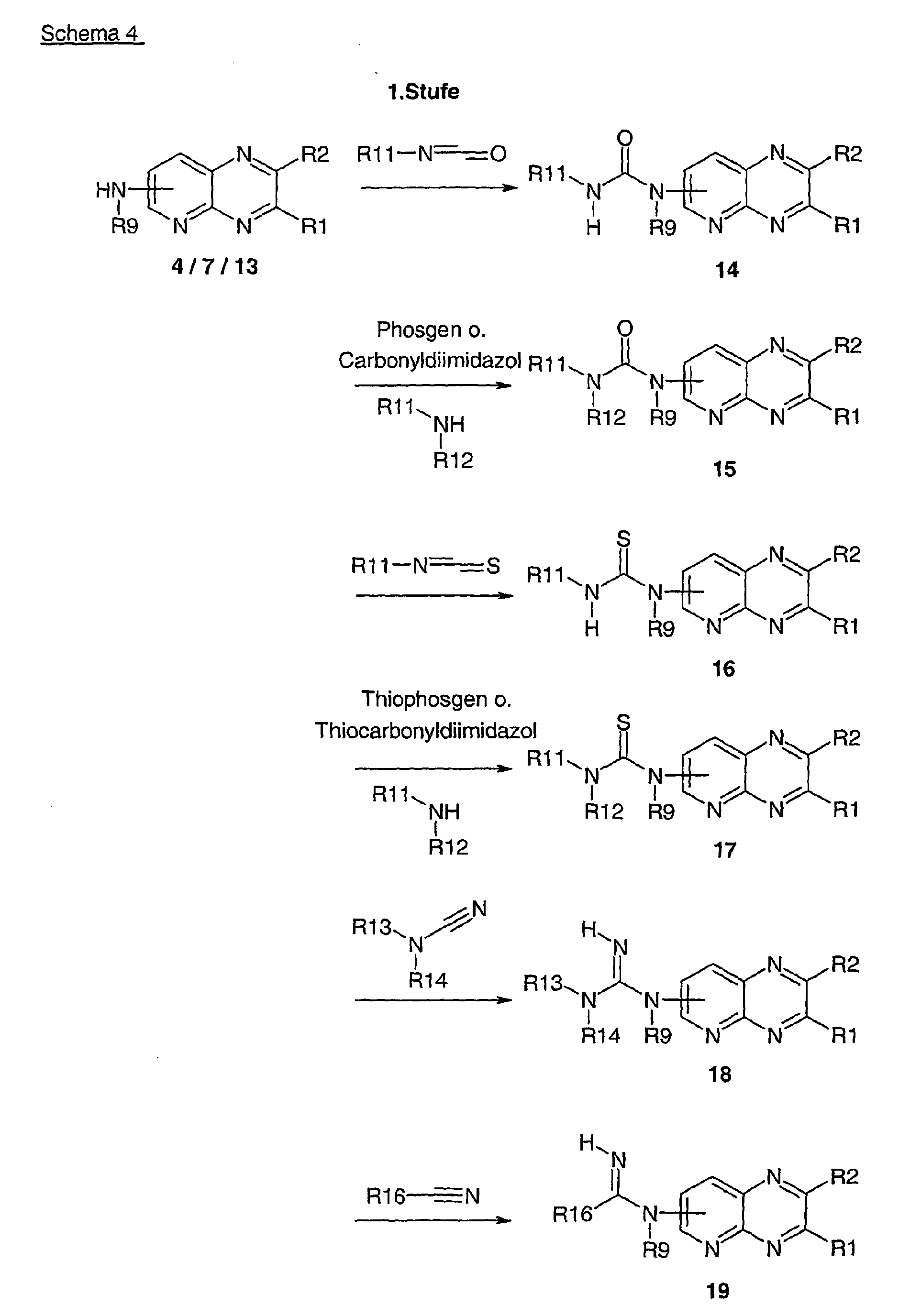

Ausgewählte Beispiele der erfindungsgemäßen Pyrido[2,3-b]pyrazine gemäß der allgemeinen Formel I, bei denen die Substituenten R1 und R2 ausgewählte Carbonsäureester-, Carbonsäureamid-, Sulfonsäureester- oder Sulfonamid-substituierte Reste sein können, sind beispielsweise nach dem Verfahren in Schema 5 oder entsprechenden, dem Fachmann bekannten Verfahren erhältlich.

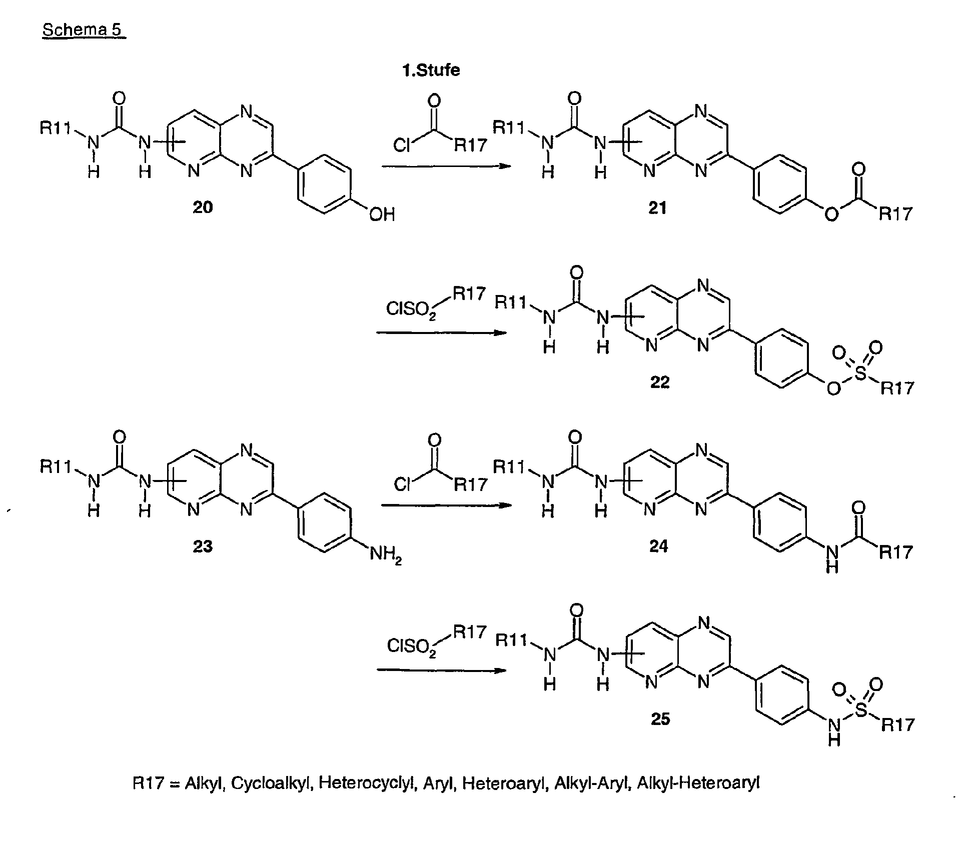

Die Ausgangsverbindungen sind entweder im Handel erhältlich oder können nach an sich bekannten Verfahrensweisen hergestellt werden. Die Edukte **4, 7** und **10-13** stellen wertvolle Zwischenverbindungen für die Herstellung der erfindungsgemäßen Pyridopyrazine der allgemeinen Formel I dar.

Für die Herstellung der Ausgangs- und Zielverbindungen sei beispielsweise auf folgende Primärliteratur verwiesen, deren Inhalt hiermit Bestandteil der Offenbarung der vorliegenden Anmeldung werden soll:
1) Houben-Weyl, Methoden der Organischen Chemie, Band 4/1 a, S. 343-350
2) Houben-Weyl, Methoden der Organischen Chemie, 4.Aufl., Band E 7b (Teil 2), S. 579; Degussa GB 1184848 (1970); S. Seko, et al. EP 735025 (1996)
3) D. Catarzi, et al.; J. Med Chem. 1996, 1330-1336; J. K. Seydel, et al.; J. Med. Chem. 1994, 3016-3022
4) Houben-Weyl, Methods of Organic Chemistry, Volume E 9c, S.231-235
5) Houben-Weyl/Science of Synthesis, Volume 16, S. 1269
6) C. Goenczi, et al. J. Chem. Soc. Perkin Trans.1 2000, 9, 1417-1422
7) M. S. A. EI-Gaby, et al. Indian J. Chem. Sect. B 2001, 40, 195- 200; M. R. Myers, et al. Bioorg. Med. Chem. Lett. 2003, 13, 3091-3096 ; A. R. Renslo, et al. J. Amer. Chem. Soc. 1999, 121, 7459-7460; C. O. Okafor, et al. J. Heterocyclic Chemistry 1983, 20, 199-203
8) J. Yin, et al. Org. Lett. 2002, 4, 3481-3484 ; O. A. El-Sayed, et al. Arch. Pharm. 2002, 335, 403-410 ; C. Temple, et al. J. Med. Chem. 1992, 35, 988-993
9) A. M. Thompson, et al. J. Med. Chem. 2000, 4200-4211
10) G. Heinisch, et al. Arch. Pharm. 1997, 207-210
11) N. A. Dales, et al. Org. Lett. 2001, 2313-2316; G. Dannhardt, et al. Arch. Pharm. 2000, 267-274
12) M. L. Mussous, et al. Tetrahedron 1999, 4077-4094; A. Kling, et al. Bioorg. Med. Chem. Lett. 2002, 441-446
13) I. K. Khanna, et al. J. Med. Chem. 2000, 3168-3185
14) L. Younghee, et al. Bioorg. Med. Chem. Lett. 2000, 2771-2774; N. L. Reddy, et al. J. Med. Chem. 1998, 3298,3302
15) A. V. Wizuycia, et al. J. Org. Chem. 2002, 67, 7151-7154; K. Kano, et al. J. Amer. Chem. Soc. 2002, 124, 9937-9944; M. L. Bushey, et al. J. Amer. Chem. Soc. 2003, 125, 8264-8269, A. Casini, et al. Bioorg. Med. Chem. Lett. 2003, 13, 837-840

### Allgemeine Vorschrift zur Darstellung der Verbindungen der allgemeinen Formel I :

### Schema 1: 1. Stufe

2,6-Diamino-3-nitropyridin oder 2-Amino-3,5-dinitro-pyridin werden in einem geeigneten, inerten Lösungsmittel, wie beispielsweise Methanol, Ethanol, Dimethylformamid oder Dioxan, gelöst. Nach Zugabe eines Katalysators, beispielsweise Raney-Nickel, Palladium auf Kohle oder Platin(IV)dioxid, setzt man das Reaktionsgemisch unter eine Wasserstoff-Atmosphäre, wobei ein Druck zwischen 1 und 5 bar eingestellt wird. Man läßt das Reaktionsgemisch mehrere Stunden, beispielsweise 1-16 Stunden, in einem Temperaturbereich zwischen 20 °C und 60 °C reagieren. Nach beendeter Umsetzung filtriert man die unlöslichen Rückstände ab, wobei das Filtermedium beispielsweise aus Kieselgel, Celite oder handelsüblichen Glasfaserfiltern bestehen kann, und wäscht mit dem entsprechenden Lösungsmittel nach. Das Rohprodukt wird, in Lösung vorliegend, ohne weitere Aufreinigung für die nächste Umsetzung verwendet.

### 2. Stufe

Das 1,2-Dion-Derivat wird in einem geeigneten, inerten Lösungsmittel, beispielsweise Methanol, Ethanol, Dioxan, Toluol oder Dimethylformamid, vorgelegt. 2,3,6-Triaminopyridin oder 2,3,5-Triaminopyridin werden direkt nach der Reduktion als Lösung der Rohprodukte in einem der oben genannten Lösungsmittel zum vorgelegten 1,2-Dion gegeben, gegebenenfalls unter Zugabe einer Säure, wie z. B. Essigsäure oder einer Base, beispielsweise Kaliumhydroxid. Das Reaktionsgemisch läßt man in einem Temperaturbereich von 20 °C bis 80 °C einige Zeit, beispielsweise 20 Minuten bis 40 Stunden, reagieren. Nach beendeter Umsetzung wird ein eventuell ausgefallener Niederschlag abfiltriert, wobei das Filtermedium beispielsweise aus handelsüblichem Filterpapier bestehen kann, mit dem entsprechenden Lösungsmittel nachgewaschen und der zurückbleibende Feststoff im Vakuum getrocknet, bzw. wird das Reaktionsgemisch im Vakuum vom Lösungsmittel befreit. Bei Verwendung von Dimethylformamid wird das Reaktionsgemisch in eine große Menge Wasser eingerührt und der ausgefallene Niederschlag abfiltriert bzw. die wässrige Phase mit einem geeigneten organischen Lösungsmittel, wie z. B. Dichlormethan oder Ethylacetat, extrahiert und die organischen Phasen im Vakuum eingeengt. Die Reinigung des zurückbleibenden Rohproduktes erfolgt durch Umkristallisieren aus einem geeigneten Lösungsmittel, beispielsweise Dioxan, oder durch Säulen- bzw. Flash-Chromatographie an Kieselgel oder Aluminiumoxid. Als Laufmittel dient beispielsweise ein Gemisch aus Methanol und Dichlormethan.

### Schema 2: 1. Stufe

2,3,6-Triaminopyridin oder 2,3,5-Triaminopyridin werden direkt nach der Reduktion als Lösung der Rohprodukte in einem der oben genannten Lösungsmittel vorgelegt. Nach Zugabe eines Oxalsäure-Derivates, wie z. B. Oxalsäurediethylester oder Oxalylchlorid, läßt man das Reaktionsgemisch, gegebenenfalls unter Zugabe einer Säure, wie z. B. Salzsäure, Schwefelsäure oder Eisessig, in einem Temperaturbereich von 20°C bis 150 °C einige Zeit, beispielsweise 10 Minuten bis 24 Stunden, reagieren. Nach beendeter Umsetzung wird ein eventuell ausgefallener Niederschlag abfiltriert, wobei das Filtermedium beispielsweise aus handelsüblichem Filterpapier bestehen kann, mit dem entsprechenden Lösungsmittel nachgewaschen und der zurückbleibende Feststoff im Vakuum getrocknet, bzw. wird das Reaktionsgemisch im Vakuum vom Lösungsmittel befreit. Bei Verwendung von Dimethylformamid wird das Reaktionsgemisch in eine große Menge Wasser eingerührt und der ausgefallene Niederschlag abfiltriert bzw. die wässrige Phase mit einem geeigneten organischen Lösungsmittel, wie z. B. Dichlormethan oder Ethylacetat, extrahiert und die organischen Phasen im Vakuum eingeengt. Die Reinigung des zurückbleibenden Rohproduktes erfolgt durch Umkristallisieren aus einem geeigneten Lösungsmittel, beispielsweise Dioxan oder Toluol, oder durch Säulen- bzw. Flash-Chromatographie an Kieselgel oder Aluminiumoxid. Als Laufmittel dient beispielsweise ein Gemisch aus Methanol und Dichlormethan.

### 2.Stufe

Das Dion-Derivat **8** wird in einem geeigneten, inerten Lösungsmittel, beispielsweise Dimethylformamid, Dioxan oder Toluol, oder ohne Lösungsmittel vorgelegt. Ein Chlorierungsmittel, z. B. Phosphorylchlorid oder Thionylchlorid, wird bei Raumtemperatur zugegeben und das Reaktionsgemisch lässt man in einem Temperaturbereich von 20 °C bis 100 °C einige Zeit, beispielsweise 1 Stunde bis 24 Stunden, reagieren. Nach beendeter Umsetzung wird das Reaktionsgemisch auf Wasser gegossen und mit einer geeigneten wässrigen Base, beispielsweise Natronlauge, neutralisiert. Ein eventuell ausgefallener Niederschlag wird abfiltriert, wobei das Filtermedium beispielsweise aus handelsüblichem Filterpapier bestehen kann, mit dem entsprechenden Lösungsmittel nachgewaschen und der zurückbleibende Feststoff im Vakuum getrocknet, oder die wässrige Phase wird mit einem geeigneten organischen Lösungsmittel, wie z. B. Dichlormethan oder Ethylacetat, extrahiert, und die organischen Phasen im Vakuum eingeengt. Die Reinigung des zurückbleibenden Rohproduktes erfolgt durch Umkristallisieren aus einem geeigneten Lösungsmittel, beispielsweise Dioxan oder Toluol, oder durch Säulen- bzw. Flash-Chromatographie an Kieselgel oder Aluminiumoxid. Als Laufmittel dient beispielsweise ein Gemisch aus Methanol und Dichlormethan.

### 3. Stufe

Die Zwischenstufe **9** kann mit einem entsprechenden Alkohol, Thiol oder Amin und gegebenenfalls mit einer geeigneten Base, vorzugsweise Natriumhydrid, Pyridin, Triethylamin, Kaliumcarbonat oder Natriummethanolat in Methanol, in einem geeigneten, inerten Lösungsmittel, wie beispielsweise Dimethylformamid, Dimethylsulfoxid, Methanol, Toluol, oder in einer Base als Lösungsmittel, wie z. B. Pyridin oder Triethylamin, oder ohne Lösungsmittel, umgesetzt werden. Das Reaktionsgemisch lässt man einige Zeit, beispielsweise 30 Minuten bis 2 Tage, in einem Temperaturbereich zwischen 20 °C und 140 °C reagieren. Nach beendeter Umsetzung wird ein eventuell ausgefallener Niederschlag abfiltriert, wobei das Filtermedium beispielsweise aus handelsüblichem Filterpapier bestehen kann, mit dem entsprechenden Lösungsmittel nachgewaschen und der zurückbleibende Feststoff im Vakuum getrocknet, bzw. wird das Reaktionsgemisch im Vakuum vom Lösungsmittel befreit. Bei Verwendung von Dimethylformamid oder Dimethylsulfoxid wird das Reaktionsgemisch in eine große Menge Wasser eingerührt und der ausgefallene Niederschlag abfiltriert bzw. die wässrige Phase mit einem geeigneten organischen Lösungsmittel, wie z. B. Dichlormethan oder Ethylacetat, extrahiert und die organischen Phasen im Vakuum eingeengt. Die Reinigung des zurückbleibenden Rohproduktes erfolgt durch Umkristallisieren aus einem geeigneten Lösungsmittel, beispielsweise Dioxan oder Toluol, oder durch Säulen- bzw. Flash-Chromatographie an Kieselgel oder Aluminiumoxid. Als Laufmittel dient beispielsweise ein Gemisch aus Methanol und Dichlormethan.

### Schema 3: 1. Stufe

Die Zwischenstufen **4** und **7** können mit einem entsprechenden, geeigneten Chlorid, Bromid oder Tosylat und gegebenenfalls mit einer geeigneten Base, vorzugsweise Natriumhydrid, Pyridin, Triethylamin, Kaliumcarbonat oder Natriummethanolat in Methanol, in einem geeigneten, inerten Lösungsmittel, wie beispielsweise Dimethylformamid, Dimethylsulfoxid, Methanol oder in einer Base als Lösungsmittel, wie z. B. Pyridin oder Triethylamin, oder ohne Lösungsmittel, umgesetzt werden. Das Reaktionsgemisch lässt man einige Zeit, beispielsweise 1 Stunde bis 24 Stunden, in einem Temperaturbereich zwischen 20 °C und 150°C reagieren. Alternativ können die Zwischenstufen **4** und **7** mit einem entsprechenden Aryl-Bromid oder -lodid und einem geeigneten Katalysator, wie z. B. Palladiumacetat oder Pd₂(dba)₃, und einem geeigneten Liganden, wie z. B. BINAP, und einer geeigneten Base, beispielsweise, Kaliumcarbonat oder Natrium-tert.butanolat, in einem geeignetem Lösungsmittel, wie beispielsweise Toluol oder Dioxan, umgesetzt werden. Das Reaktionsgemisch lässt man einige Zeit, beispielsweise 10 Stunden bis 30 Stunden, in einem Temperaturbereich zwischen 60 °C und 120 °C reagieren. Nach beendeter Umsetzung wird ein eventuell ausgefallener Niederschlag abfiltriert, wobei das Filtermedium beispielsweise aus handelsüblichem Filterpapier bestehen kann, mit dem entsprechenden Lösungsmittel nachgewaschen und der zurückbleibende Feststoff im Vakuum getrocknet, bzw. werden evtl. vorhandene Katalysatorreste abfiltriert mit dem entsprechenden Lösungsmittel nachgewaschen und das Lösungsmittel im Vakuum entfernt, bzw. wird das Reaktionsgemisch im Vakuum vom Lösungsmittel befreit. Bei Verwendung von Dimethylformamid oder Dimethylsulfoxid wird das Reaktionsgemisch in eine große Menge Wasser eingerührt und der ausgefallene Niederschlag abfiltriert bzw. die wässrige Phase mit einem geeigneten organischen Lösungsmittel, wie z. B. Dichlormethan oder Ethylacetat, extrahiert und die organischen Phasen im Vakuum eingeengt. Die Reinigung des zurückbleibenden Rohproduktes erfolgt durch Umkristallisieren aus einem geeigneten Lösungsmittel, beispielsweise EtOH, oder durch Säulen- bzw. Flash-Chromatographie an Kieselgel oder Aluminiumoxid. Als Laufmittel dient beispielsweise ein Gemisch aus Methanol und Dichlormethan.

### Schema 4: 1. Stufe

Im Anschluß an die Grundverfahren können in Folgereaktionen die nach dem Grundverfahren entstandenen Produkte in einer dem Fachmann bekannten Vorgehensweise zu erfindungsgemässen Folgeprodukten gemäß der Formel I umgesetzt werden.

So kann, wenn das Produkt ein Derivat der Verbindung **14** gemäß Schema 4 sein soll, nach Ablauf der Grundreaktionen das Reaktionsprodukt **4, 7** oder **13** mit einem entsprechenden Isocyanat und gegebenenfalls einer geeigneten Base, vorzugsweise Natriumhydrid, Kaliumhexamethyldisilazid, Pyridin, Triethylamin oder Kaliumcarbonat, in einem geeigneten, inerten Lösungsmittel, wie beispielsweise Dimethylformamid, Dimethylsulfoxid, Acetonitril, Dichlormethan, 1,2-Dichlorethan oder Dioxan, oder in einer Base als Lösungsmittel, wie z. B. Pyridin oder Triethylamin, oder ohne Lösungsmittel, umgesetzt werden. Das Reaktionsgemisch läßt man mehrere Stunden, beispielsweise 1 - 24 Stunden, in einem Temperaturbereich zwischen 0 und 80 °C reagieren. Nach beendeter Umsetzung wird ein eventuell ausgefallener Niederschlag abfiltriert, wobei das Filtermedium beispielsweise aus handelsüblichem Filterpapier bestehen kann, mit dem entsprechenden Lösungsmittel nachgewaschen und der zurückbleibende Feststoff im Vakuum getrocknet, bzw. wird das Reaktionsgemisch im Vakuum vom Lösungsmittel befreit. Bei Verwendung von Dimethylformamid oder Dimethylsulfoxid wird das Reaktionsgemisch in eine große Menge Wasser eingerührt und der ausgefallene Niederschlag abfiltriert bzw. die wässrige Phase mit einem geeigneten organischen Lösungsmittel, wie z. B. Dichlormethan oder Ethylacetat, extrahiert und die organischen Phasen im Vakuum eingeengt. Die Reinigung des zurückbleibenden Rohproduktes erfolgt durch Umkristallisieren aus einem geeigneten Lösungsmittel, beispielsweise Ethanol oder Ethylacetat, oder durch Säulen- bzw. Flash-Chromatographie an Kieselgel oder Aluminiumoxid. Als Laufmittel dient beispielsweise ein Gemisch aus Methanol und Dichlormethan.

Oder es kann alternativ, wenn das Produkt ein Derivat der Verbindung **15** gemäß Schema 4 sein soll, nach Ablauf der Grundreaktionen das Reaktionsprodukt **4, 7** oder **13** mit Phosgen oder Carbonyldiimidazol und einem entsprechenden Amin in einem geeigneten, inerten Lösungsmittel, wie beispielsweise Dimethylformamid, Tetrahydrofuran, Toluol, Dichlormethan oder Acetonitril umgesetzt werden. Gegebenenfalls wird eine geeignete Base, vorzugsweise Pyridin, Natriumhydrogencarbonat, Triethylamin, N-Methyl-morpholin oder Natriumacetat verwendet. Das Reaktionsgemisch läßt man einige Zeit, beispielsweise 15 Minuten bis 24 Stunden, in einem Temperaturbereich zwischen 0 und 60 °C reagieren. Nach beendeter Umsetzung wird ein eventuell ausgefallener Niederschlag abfiltriert, wobei das Filtermedium beispielsweise aus handelsüblichem Filterpapier bestehen kann, mit dem entsprechenden Lösungsmittel nachgewaschen und der zurückbleibende Feststoff im Vakuum getrocknet, bzw. wird das Reaktionsgemisch im Vakuum vom Lösungsmittel befreit. Bei Verwendung von Dimethylformamid wird das Reaktionsgemisch in eine große Menge Wasser eingerührt und der ausgefallene Niederschlag abfiltriert bzw. die wässrige Phase mit einem geeigneten organischen Lösungsmittel, wie z. B. Dichlormethan oder Ethylacetat, extrahiert und die organischen Phasen im Vakuum eingeengt. Die Reinigung des zurückbleibenden Rohproduktes erfolgt durch Umkristallisieren aus einem geeigneten Lösungsmittel, beispielweise Ethanol oder Ethylacetat, oder durch Säulen- bzw. Flash-Chromatographie an Kieselgel oder Aluminiumoxid. Als Laufmittel dient beispielsweise ein Gemisch aus Methanol und Dichlormethan.

So kann, wenn das Produkt ein Derivat der Verbindung **16** gemäß Schema 4 sein soll, nach Ablauf der Grundreaktionen das Reaktionsprodukt **4, 7** oder **13** mit einem entsprechenden Isothiocyanat und gegebenenfalls einer geeigneten Base, vorzugsweise Natriumhydrid, Triethylamin, Kaliumcarbonat oder Pyridin, in einem geeigneten, inerten Lösungsmittel, wie beispielsweise Dimethylformamid, Tetrahydrofuran, Aceton oder Toluol, oder in einer Base als Lösungsmittel, wie z. B. Pyridin oder Triethylamin, oder ohne Lösungsmittel, umgesetzt werden. Das Reaktionsgemisch läßt man einige Zeit, beispielsweise 30 Minuten bis 90 Stunden, in einem Temperaturbereich zwischen 0 und 115°C reagieren. Nach beendeter Umsetzung wird ein eventuell ausgefallener Niederschlag abfiltriert, wobei das Filtermedium beispielsweise aus handelsüblichem Filterpapier bestehen kann, mit dem entsprechenden Lösungsmittel nachgewaschen und der zurückbleibende Feststoff im Vakuum getrocknet, bzw. wird das Reaktionsgemisch im Vakuum vom Lösungsmittel befreit. Bei Verwendung von Dimethylformamid wird das Reaktionsgemisch in eine große Menge Wasser eingerührt und der ausgefallene Niederschlag abfiltriert bzw. die wässrige Phase mit einem geeigneten organischen Lösungsmittel, wie z. B. Dichlormethan oder Ethylacetat, extrahiert und die organischen Phasen im Vakuum eingeengt. Die Reinigung des zurückbleibenden Rohproduktes erfolgt durch Umkristallisieren aus einem geeigneten Lösungsmittel, beispielsweise Ethanol oder Ethylacetat, oder durch Säulen- bzw. Flash-Chromatographie an Kieselgel oder Aluminiumoxid. Als Laufmittel dient beispielsweise ein Gemisch aus Methanol und Dichlormethan.

Oder es kann alternativ, wenn das Produkt ein Derivat der Verbindung **17** gemäß Schema 4 sein soll, nach Ablauf der Grundreaktionen das Reaktionsprodukt **4, 7** oder **13** mit Thiophosgen oder Thiocarbonyldiimidazol und einem entsprechenden Amin in einem geeigneten, inerten Lösungsmittel, wie beispielsweise Dimethylformamid, Tetrahydrofuran, Toluol, Dichlormethan, Ethanol oder Acetonitril umgesetzt werden. Gegebenenfalls wird eine geeignete Base, vorzugsweise Pyridin, Natriumhydrogencarbonat, Kaliumcarbonat, Triethylamin oder Imidazol verwendet. Das Reaktionsgemisch läßt man mehrere Stunden, beispielsweise 1 bis 24 Stunden, in einem Temperaturbereich zwischen -10 und 80°C reagieren. Nach beendeter Umsetzung wird ein eventuell ausgefallener Niederschlag abfiltriert, wobei das Filtermedium beispielsweise aus handelsüblichem Filterpapier bestehen kann, mit dem entsprechenden Lösungsmittel nachgewaschen und der zurückbleibende Feststoff im Vakuum getrocknet, bzw. wird das Reaktionsgemisch im Vakuum vom Lösungsmittel befreit. Bei Verwendung von Dimethylformamid wird das Reaktionsgemisch in eine große Menge Wasser eingerührt und der ausgefallene Niederschlag abfiltriert bzw. die wässrige Phase mit einem geeigneten organischen Lösungsmittel, wie z. B. Dichlormethan oder Ethylacetat, extrahiert und die organischen Phasen im Vakuum eingeengt. Die Reinigung des zurückbleibenden Rohproduktes erfolgt durch Umkristallisieren aus einem geeigneten Lösungsmittel, beispielweise Ethanol oder Ethylacetat, oder durch Säulen- bzw. Flash-Chromatographie an Kieselgel oder Aluminiumoxid. Als Laufmittel dient beispielsweise ein Gemisch aus Methanol und Dichlormethan.

So kann, wenn das Produkt ein Derivat der Verbindung **18** gemäß Schema 4 sein soll, nach Ablauf der Grundreaktionen das Reaktionsprodukt **4, 7** oder **13** mit einem entsprechenden Aminonitril und gegebenenfalls einer geeigneten Base, vorzugsweise Triethylamin oder Pyridin, oder einer geeigneten Säure, vorzugsweise Salzsäure, in einem geeigneten, inerten Lösungsmittel, wie beispielsweise Aceton, Toluol, Chlorbenzol, Ethanol, Tetrahydrofuran oder Dimethylsulfoxid, oder in einer Base als Lösungsmittel, wie z. B. Pyridin oder Triethylamin, oder ohne Lösungsmittel umgesetzt werden. Das Reaktionsgemisch läßt man mehrere Stunden, beispielsweise 2 bis 140 Stunden, in einem Temperaturbereich zwischen 20 und 135°C reagieren. Nach beendeter Umsetzung wird ein eventuell ausgefallener Niederschlag abfiltriert, wobei das Filtermedium beispielsweise aus handelsüblichem Filterpapier bestehen kann, mit dem entsprechenden Lösungsmittel nachgewaschen und der zurückbleibende Feststoff im Vakuum getrocknet, bzw. wird das Reaktionsgemisch im Vakuum vom Lösungsmittel befreit. Die Reinigung des zurückbleibenden Rohproduktes erfolgt durch Umkristallisieren aus einem geeigneten Lösungsmittel, beispielsweise Dioxan, oder durch Säulen- bzw. Flash-Chromatographie an Kieselgel oder Aluminiumoxid oder durch HPLC. Als Laufmittel dient beispielsweise ein Gemisch aus Methanol und Dichlormethan, bzw. bei HPLC-Aufreinigung beispielsweise ein Laufmittelgemisch aus Acetonitril und Wasser.

Oder es kann, wenn das Produkt ein Derivat der Verbindung **19** gemäß Schema 4 sein soll, nach Ablauf der Grundreaktionen das Reaktionsprodukt **4, 7** oder **13** mit einem entsprechenden Nitril und gegebenenfalls einer geeigneten Base, vorzugsweise Natriumhydrid, Pyridin, Triethylamin oder Natriumhexamethyldisilazid, oder einem geeigneten Katalysator, beispielsweise Aluminiumtrichlorid, Trimethylaluminium, Eisessig oder Schwefelsäure, in einem geeigneten, inerten Lösungsmittel, wie beispielsweise Dioxan, Toluol oder Ethanol, oder in einer Base als Lösungsmittel, wie z. B. Pyridin oder Triethylamin, oder ohne Lösungsmittel umgesetzt werden. Das Reaktionsgemisch läßt man einige Zeit, beispielsweise 30 Minuten bis 24 Stunden, in einem Temperaturbereich zwischen 0 und 200°C reagieren. Nach beendeter Umsetzung wird ein eventuell ausgefallener Niederschlag abfiltriert, wobei das Filtermedium beispielsweise aus handelsüblichem Filterpapier bestehen kann, mit dem entsprechenden Lösungsmittel nachgewaschen und der zurückbleibende Feststoff im Vakuum getrocknet, bzw. wird das Reaktionsgemisch im Vakuum vom Lösungsmittel befreit. Die Reinigung des zurückbleibenden Rohproduktes erfolgt durch Umkristallisieren aus einem geeigneten Lösungsmittel, beispielsweise Dioxan, oder durch Säulen- bzw. Flash-Chromatographie an Kieselgel oder Aluminiumoxid oder durch HPLC. Als Laufmittel dient beispielsweise ein Gemisch aus Methanol und Dichlormethan bzw. bei HPLC-Aufreinigung beispielsweise ein Laufmittelgemisch aus Acetonitril und Wasser.

### Schema 5: 1. Stufe

Im Anschluß an die Grundverfahren können in Folgereaktionen die nach dem Grundverfahren entstandenen Produkte in einer dem Fachmann bekannten Vorgehensweise zu erfindungsgemässen Folgeprodukten gemäß der Formel I umgesetzt werden.

So kann, wenn das Produkt ein Derivat der Verbindung **21** oder **24** gemäß Schema 5 sein soll, nach Ablauf der Grundreaktionen das Reaktionsprodukt **20** oder **23** mit einem entsprechenden Carbonsäurechlorid und gegebenenfalls einer geeigneten Base, vorzugsweise Natriumhydrid, Kaliumhydroxid, Pyridin, Triethylamin oder Kaliumcarbonat, in einem geeigneten, inerten Lösungsmittel, wie beispielsweise Tetrahydrofuran, Toluol, Acetonitril, Dichlormethan, Aceton oder Dioxan, oder in einer Base als Lösungsmittel, wie z. B. Pyridin oder Triethylamin, oder ohne Lösungsmittel, umgesetzt werden. Das Reaktionsgemisch läßt man einige Zeit, beispielsweise 30 Minuten bis 12 Stunden, in einem Temperaturbereich zwischen 0 und 110 °C reagieren. Nach beendeter Umsetzung wird ein eventuell ausgefallener Niederschlag abfiltriert, wobei das Filtermedium beispielsweise aus handelsüblichem Filterpapier bestehen kann, mit dem entsprechenden Lösungsmittel nachgewaschen und der zurückbleibende Feststoff im Vakuum getrocknet, bzw. wird das Reaktionsgemisch im Vakuum vom Lösungsmittel befreit. Alternativ kann das Reaktionsgemisch in eine große Menge Wasser eingerührt und der ausgefallene Niederschlag abfiltriert bzw. die wässrige Phase, nach Neutralisation mit einer geeigneten wässrigen Säure, wie z. B. Salzsäure, mit einem geeigneten organischen Lösungsmittel, wie z. B. Dichlormethan oder Ethylacetat, extrahiert und die organische Phase im Vakuum eingeengt werden. Die Reinigung des zurückbleibenden Rohproduktes erfolgt durch Umkristallisieren aus einem geeigneten Lösungsmittel, beispielsweise Ethanol, oder durch Säulen- bzw. Flash-Chromatographie an Kieselgel oder Aluminiumoxid. Als Laufmittel dient beispielsweise ein Gemisch aus Methanol und Dichlormethan.

Oder es kann alternativ, wenn das Produkt ein Derivat der Verbindung **22** oder **25** gemäß Schema 5 sein soll, nach Ablauf der Grundreaktionen das Reaktionsprodukt **20** oder **23** mit einem entsprechenden Sulfonsäurechlorid und gegebenenfalls einer geeigneten Base, vorzugsweise Natriumhydrid, Kaliumhydroxid, Pyridin, Triethylamin oder Kaliumcarbonat, in einem geeigneten, inerten Lösungsmittel, wie beispielsweise Tetrahydrofuran, Toluol, Acetonitril, Dichlormethan, Aceton, Dimethylformamid oder Dioxan, oder in einer Base als Lösungsmittel, wie z. B. Pyridin oder Triethylamin, oder ohne Lösungsmittel, umgesetzt werden. Das Reaktionsgemisch läßt man einige Zeit, beispielsweise 30 Minuten bis 16 Stunden, in einem Temperaturbereich zwischen 0 und 80 °C reagieren. Nach beendeter Umsetzung wird ein eventuell ausgefallener Niederschlag abfiltriert, wobei das Filtermedium beispielsweise aus handelsüblichem Filterpapier bestehen kann, mit dem entsprechenden Lösungsmittel nachgewaschen und der zurückbleibende Feststoff im Vakuum getrocknet, bzw. wird das Reaktionsgemisch im Vakuum vom Lösungsmittel befreit. Alternativ kann das Reaktionsgemisch in eine große Menge Wasser eingerührt und der ausgefallene Niederschlag abfiltriert bzw. die wässrige Phase, nach Neutralisation mit einer geeigneten wässrigen Säure, wie z. B. Salzsäure, mit einem geeigneten organischen Lösungsmittel, wie z. B. Dichlormethan oder Ethylacetat, extrahiert und die organische Phase im Vakuum eingeengt werden. Bei Verwendung von Dimethylformamid wird das Reaktionsgemisch in eine große Menge Wasser eingerührt und der ausgefallene Niederschlag abfiltriert bzw. die wässrige Phase mit einem geeigneten organischen Lösungsmittel, wie z. B. Dichlormethan oder Ethylacetat, extrahiert und die organischen Phasen im Vakuum eingeengt. Die Reinigung des zurückbleibenden Rohproduktes erfolgt durch Umkristallisieren aus einem geeigneten Lösungsmittel, beispielsweise Ethanol, oder durch Säulen- bzw. Flash-Chromatographie an Kieselgel oder Aluminiumoxid. Als Laufmittel dient beispielsweise ein Gemisch aus Methanol und Dichlormethan.

Unter einigen der genannten Reaktionsbedingungen können OH-, SH- und NH₂-Gruppen möglicherweise unerwünschte Nebenreaktionen eingehen. Es ist daher bevorzugt, diese mit Schutzgruppen zu versehen oder im Falle von NH₂ durch NO₂ zu ersetzen und nachfolgend die Schutzgruppe abzuspalten oder die NO₂-Gruppe zu reduzieren. So kann in Abwandlung der oben beschriebenen Verfahren in den Ausgangsverbindungen mindestens eine OH-Gruppe beispielsweise durch eine Benzyloxygruppe und/oder mindestens eine SH-Gruppe beispielsweise durch eine S-Benzylgruppe und/oder mindestens eine NH₂-Gruppe durch eine NO₂-Gruppe ersetzt werden. Nachfolgend kann mindestens eine - vorzugsweise alle - Benzyloxygruppe/n beispielsweise mit Wasserstoff und Palladium auf Kohle und/oder mindestens eine - vorzugsweise alle - S-Benzylgruppe/n beispielsweise mit Natrium in Ammoniak abgespalten und/oder mindestens eine - vorzugsweise alle - NO₂-Gruppe/n beispielsweise mit Wasserstoff und Raney-Nickel zu NH₂ reduziert werden.

Unter einigen der genannten Reaktionsbedingungen können OH-, NH₂- und COOH-Gruppen möglicherweise unerwünschte Nebenreaktionen eingehen. Es ist daher bevorzugt, Ausgangsverbindungen und Zwischenstufen, welche mindestens eine OH- und/oder mindestens eine NH₂- und/oder mindestens eine COOH-Gruppe enthalten, in entsprechende Carbonsäureester- und Carbonsäureamid-Derivate zu überführen. In Abwandlung der oben beschriebenen Verfahren können Ausgangsverbindungen und Zwischenstufen, welche mindestens eine OH-Gruppe besitzen, und/oder welche mindestens eine NH₂-Gruppe besitzen, durch Umsetzung mit einer aktivierten Carbonsäuregruppe, beispielsweise einer Carbonsäurechloridgruppe, in Carbonsäureester- bzw. Carbonsäureamid-Derivate überführt werden. In Abwandlung der oben beschriebenen Verfahren können Ausgangsverbindungen und Zwischenstufen, welche mindestens eine COOH-Gruppe besitzen, durch Umsetzung mit einem Aktivierungsmittel, wie beispielsweise Thionylchlorid oder Carbonyldiimidazol, und nachfolgender Umsetzung mit einem geeigneten Alkohol oder Amin in Carbonsäureester- bzw. Carbonsäureamid-Derivate überführt werden.

Die erfindungsgemäßen Pyrido[2,3-b]pyrazin-Derivate gemäß der allgemeinen Formel I sind als Wirkstoffe in Arzneimitteln zur Modulation von fehlgeleiteten zellulären Signaltransduktionsprozessen, insbesondere zur Beeinflussung der Funktion von Tyrosin- und Serin/Threoninkinasen und bei malignen bzw. benignen Tumorerkrankungen, wie z. B. der Brust, Prostata, Lunge, Haut, Eierstöcke und anderen, auf pathologischen Zellproliferationen beruhenden Erkrankungen, wie z. B. Restenose, Psoriasis, Arteriosklerose und Leberzirrhose zur Behandlung von Menschen, Säugetieren und Geflügel geeignet. Säugetiere können Haustiere wie Pferde, Kühe, Hunde, Katzen, Hasen, Schafe und dergleichen sein.

Die medizinische Wirkung der erfindungsgemäßen Pyrido[2,3-b]pyrazin-Derivate kann zum Beispiel auf einer Modulation der Signaltransduktion durch Wechselwirkung mit Rezeptor-Tyrosinkinasen als auch mit cytoplasmatischen Tyrosin- und Serin/Threoninkinasen beruhen. Daneben sind noch weitere bekannte und unbekannte Wirkmechanismen zur Bekämpfung von malignen Prozessen denkbar.

Gemäß einem weiteren Aspekt der Erfindung wird ein Verfahren zur Bekämpfung von Tumoren beim Menschen, in Säugetieren und in Geflügel bereitgestellt, welches dadurch gekennzeichnet ist, daß mindestens ein Pyrido[2,3-b]pyrazin-Derivat gemäß der allgemeinen Formel I dem Menschen, einem Säugetier oder Geflügel in einer für die Tumorbehandlung wirksamen Menge verabreicht wird. Die für die Behandlung zu verabreichende therapeutisch effektive Dosis des jeweiligen erfindungsgemäßen Pyrido[2,3-b]pyrazin-Derivates richtet sich u.a. nach der Art und dem Stadium der Tumorerkrankung, dem Alter und Geschlecht des Patienten, der Art der Verabreichung und der Dauer der Behandlung. Die erfindungsgemäßen Arzneimittel können als flüssige, halbfeste und feste Arzneiformen verabreicht werden. Dies erfolgt in der jeweils geeigneten Weise in Form von Aerosolen, Pulver, Puder und Streupuder, Tabletten, Dragees, Emulsionen, Schäume, Lösungen, Suspensionen, Gele, Salben, Pasten, Pillen, Pastillen, Kapseln oder Suppositorien.

Die Arzneiformen enthalten neben mindestens einem erfindungsgemäßen Bestandteil je nach eingesetzter galenischer Form gegebenenfalls Hilfsstoffe, wie unter anderem Lösungsmittel, Lösungsbeschleuniger, Lösungsvermittler, Emulgatoren, Netzmittel, Antischaummittel, Gelbildner, Verdickungsmittel, Filmbildner, Bindemittel, Puffer, Salzbildner, Trocknungsmittel, Fließregulierungsmittel, Füllstoffe, Konservierungsstoffe, Antioxidatien, Farbstoffe, Formentrennmittel, Gleitmittel, Sprengmittel, Geschmacks - und Geruchskorrigentien. Die Auswahl der Hilfsstoffe sowie die einzusetzenden Mengen derselben hängt von der gewählten galenischen Form ab und orientiert sich an den dem Fachmann bekannten Rezepturen.

Die erfindungsgemäßen Arzneimittel können in einer geeigneten Darreichungsform auf die Haut, epicutan als Lösung, Suspension, Emulsion, Schaum, Salbe, Paste oder Pflaster; über die Mund- und Zungenschleimhaut, buccal, lingual oder sublingual als Tablette, Pastille, Dragees, Linctus oder Gurgelwasser; über die Magen- und Darmschleimhaut, enteral als Tablette, Dragees, Kapsel, Lösung, Suspension oder Emulsion; über die Rectumschleimhaut, rectal als Suppositorium, Rectalkapsel oder Salbe; über die Nasenschleimhaut, nasal als Tropfen, Salben oder Spray; über das Bronchial- und Alveolarepithel, pulmonal oder per inhalationem als Aerosol oder Inhalat; über die Conjunctiva, conjunctival als Augentropfen, Augensalbe, Augentabletten, Lamellae oder Augenwasser; über die Schleimhäute der Genitalorgane, intravaginal als Vaginalkugeln, Salben und Spülung, intrauterin als Uterus-Pessare; über die ableitenden Harnwege, intraurethral als Spülung, Salbe oder Arzneistäbchen; in eine Arterie, intraarteriell als Injektion; in eine Vene, intravenös als Injektion oder Infusion; in die Haut, intracutan als Injektion oder Implantat; unter die Haut, subcutan als Injektion oder Implantat; in den Muskel, intramusculär als Injektion oder Implantat; in die Bauchhöhle, intraperitoneal als Injektion oder Infusion verabreicht werden.

Die erfindungsgemäßen Verbindungen der allgemeinen Struktur I können in Hinblick auf praktische therapeutische Erfordernisse mittels geeigneter Maßnahmen in ihrer Arzneistoffwirkung verlängert werden. Dieses Ziel kann auf chemischem und/oder galenischem Wege erreicht werden. Beispiele für die Erzielung einer Wirkungsverlängerung sind der Einsatz von Implantaten und Liposomen, die Bildung von schwerlöslichen Salzen und Komplexen oder der Einsatz von Kristall-Suspensionen.

Besonders bevorzugt sind dabei Arzneimittel, die mindestens eine Verbindung aus der nachfolgenden Gruppe der Pyrido[2,3-b]pyrazin-Derivate der allgemeinen Struktur I enthalten und die in Form ihrer freien Base oder auch als pharmazeutisch annehmbare Salze physiologisch verträglicher Säuren vorliegen können:
1-Allyl-3-(3-phenyl-pyrido[2,3-b]pyrazin-6-yl)-thioharnstoff (Beispiel 1)
1-Allyl-3-(3-naphthalin-2-yl-pyrido[2,3-b]pyrazin-6-yl)-thioharnstoff (Bsp. 2)
1-Allyl-3-[3-(4-methoxy-phenyl)-pyrido[2,3-b]pyrazin-6-yl]-thioharnstoff (Bsp. 3)
1-Allyl-3-[3-(4-hydroxy-phenyl)-pyrido[2,3-b]pyrazin-6-yl]-thioharnstoff-Hydrochlorid (Bsp. 4)
1-(2-Methyl-allyl)-3-(3-phenyl-pyrido[2,3-b]pyrazin-6-yl)-thioharnstoff (Bsp. 5)
1-(2-Methyl-allyl)-3-(3-naphthalin-2-yl-pyrido[2,3-b]pyrazin-6-yl)-thioharnstoff (Bsp. 6)
1-[3-(4-Methoxy-phenyl)-pyrido[2,3-b]pyrazin-6-yl]-3-(2-methyl-allyl)-thioharnstoff (Bsp.7)
1-(3-Naphthalin-2-yl-pyrido[2,3-b]pyrazin-6-yl)-3-(4-nitro-phenyl)-thioharnstoff (Bsp. 8)
1-[3-(4-Methoxy-phenyl)-pyrido[2,3-b]pyrazin-6-yl]-3-(4-nitro-phenyl)-thioharnstoff (Bsp. 9)
1-*tert*-Butyl-3-(3-phenyl-pyrido[2,3-b]pyrazin-6-yl)-thioharnstoff (Bsp. 10)
1-Cyclopropyl-3-(3-phenyl-pyrido[2,3-b]pyrazin-6-yl)-thioharnstoff (Bsp. 11)
1-Methyl-3-(3-phenyl-pyrido[2,3-b]pyrazin-6-yl)-thioharnstoff (Bsp. 12)
1-Benzyl-3-(3-phenyl-pyrido[2,3-b]pyrazin-6-yl)-thioharnstoff (Bsp. 13)
1-(4-Fluoro-phenyl)-3-(3-phenyl-pyrido[2,3-b]pyrazin-6-yl)-thioharnstoff (Bsp. 14)
1-Cyclohexyl-3-(3-phenyl-pyrido[2,3-b]pyrazin-6-yl)-thioharnstoff (Bsp. 15)
1-Isopropyl-3-(3-phenyl-pyrido[2,3-b]pyrazin-6-yl)-thioharnstoff (Bsp. 16)
1-Furan-2-ylmethyl-3-(3-phenyl-pyrido[2,3-b]pyrazin-6-yl)-thioharnstoff (Bsp. 17)
1-Methyl-3-[3-(4-nitro-phenyl)-pyrido[2,3-b]pyrazin-6-yl]-thioharnstoff (Bsp.18)
1-[3-(4-Hydroxy-phenyl)-pyrido[2,3-]pyrazin-6-yl]-3-methyl-thioharnstoff (Bsp. 19)
1-Allyl-3-[3-(4-nitro-phenyl)-pyrido[2,3-b]pyrazin-6-yl]-thioharnstoff (Bsp. 20)
4-[6-(3-Allyl-thioharnstoff)-pyrido[2,3-b]pyrazin-3-yl]-benzoesäureethyl-ester (Bsp. 21)
1-Allyl-3-[3-(3-hydroxy-phenyl)-pyrido[2,3-b]pyrazin-6-yl]-thioharnstoff (Bsp. 22)
1-Allyl-3-(3-benzo[1,3]dioxol-5-yl-pyrido[2,3-b]pyrazin-6-yl)-thioharnstoff (Bsp. 23)
1-[3-(4-Hydroxy-phenyl)-pyrido[2,3-b]pyrazin-6-yl]-3-prop-2-ynyl-thioharnstoff (Bsp. 24)
1-Allyl-3-[3-(4-hydroxy-phenyl)-pyrido[2,3-b]pyrazin-6-yl]-thioharnstoff (Bsp. 25)
1-[3-(4-Hydroxy-phenyl)-pyrido[2,3-b]pyrazin-6-yl]-3-((propenyl)-thioharnstoff (Bsp. 26)
1-Allyl-3-[2,3-bis-(4-hydroxy-phenyl)-pyrido[2,3-b]pyrazin-6-yl]-thioharnstoff (Bsp. 27)
1-[2,3-Bis-(4-hydroxy-phenyl)-pyrido[2,3-b]pyrazin-6-yl]-3-((propenyl)-thioharnstoff (Bsp. 28)
1-Allyl-3-[2-(4-hydroxy-phenyl)-pyrido[2,3-b]pyrazin-6-yl]-thioharnstoff (Bsp. 29)
1-Allyl-3-[3-(4-nitro-phenyl)-pyrido[2,3-b]pyrazin-7-yl]-thioharnstoff (Bsp. 30)
1-Cyclopropyl-3-(3-phenyl-pyrido[2,3-b]pyrazin-6-yl)-harnstoff (Bsp. 31)
1-Allyl-3-[3-(4-hydroxy-phenyl)-pyrido[2,3-b]pyrazin-6-yl]-harnstoff (Bsp. 32)
1-(3-Phenyl-pyrido[2,3-b]pyrazin-6-yl)-3-p-tolyl-harnstoff (Bsp. 33)
1-(4-Chloro-3-trifluoromethyl-phenyl)-3-(3-phenyl-pyrido[2,3-b]pyrazin-6-yl)-harnstoff (Bsp. 34)
1-(2-Morpholin-4-yl-ethyl)-3-(3-phenyl-pyrido[2,3-b]pyrazin-6-yl)-harnstoff (Bsp. 35)
1-Phenethyl-3-(3-phenyl-pyrido[2,3-]pyrazin-6-yl)-harnstoff (Bsp. 36)
1-(2,3-Di-pyridin-2-yl-pyrido[2,3-b]pyrazin-6-yl)-3-ethyl-harnstoff (Bsp. 37)
1-(2,3-Dimethyl-pyrido[2,3-b]pyrazin-6-yl)-3-ethyl-harnstoff (Bsp.38)

### Ausführungsbeispiele:

Gemäß den allgemeinen Synthesevorschriften, denen die Syntheseschemata 1 - 4 zugrundeliegen, wurden folgende Verbindungen synthetisiert, die unter der Angabe der jeweiligen chemischen Bezeichnung aus der nachfolgenden Übersicht hervorgehen. Ferner sind ihre NMR-spektroskopischen Daten und Schmelzpunkte beigefügt. In der sich anschließenden Tabelle 1 sind aus der allgemeinen Formel II und den Substituenten R1, R2, R3, R4, sowie R5 und Y die Strukturen dieser Verbindungen zu ersehen.

Die eingesetzten Chemikalien und Lösungsmittel wurden kommerziell bei den herkömmlichen Anbietern erworben (Acros, Aldrich, Fluka, Lancaster, Maybridge, Merck, Sigma, TCl, etc.) oder synthetisiert.

Die Erfindung soll anhand der nachfolgenden Beispiele näher erläutert werden, ohne darauf beschränkt zu sein.

### Beispiel 1:

### Herstellung von 3-Phenyl-pyrido[2,3-b]pyrazin-6-ylamin (Umsetzung gemäß Schema 1, 1. und 2. Stufe)

Eine Lösung aus 1.22 g 2,6-Diamino-3-nitropyridin (7.92 mmol) in 210 ml Ethanol wird mit Raney-Nickel als Katalysator bei 50°C und 5 bar hydriert. Nach beendeter Hydrierung saugt man den Katalysator über einen Glasfaserfilter ab. In die Vorlage werden vor der Filtration 1.68 g Phenylglyoxal-Hydrat (11.03 mmol) in 50 ml Ethanol vorgelegt. Dann wird der Katalysator unter Stickstoff als Schutzgas abfiltriert und die Hydrierlösung direkt in den Reaktionskolben gesaugt. Das grün-blaue Reaktionsgemisch wird unter Stickstoff 30 min. unter Rückfluß erhitzt. Das Gemisch läßt man abkühlen und entfernt das Lösungsmittel im Vakuum. Man erhält schließlich einen dunkelbraunen Feststoff. Säulenchromatographische Reinigung an Kieselgel (Laufmittelgemisch Dichlormethan / Methanol) liefert einen hellgelben kristallinen Feststoff. Herstellung von 1-Allyl-3-(3-phenyl-pyrido[2,3-b]pyrazin-6-yl)-thioharnstoff (Umsetzung gemäß Schema 4,1. Stufe)

0.246 g Natriumhydrid (6.14 mmol) werden in 5 ml wasserfreiem Dimethylformamid unter Stickstoff als Schutzgas vorgelegt. Das Gemisch wird im Eisbad auf 0°C abgekühlt. 1.05 g 3-Phenyl-pyrido[2,3-b]pyrazin-6-ylamin (4.72 mmol) werden in 5 ml wasserfreiem Dimethylformamid gelöst und tropfenweise zugegeben. Man entfernt das Kühlbad und läßt das Gemisch 30 Minuten bei RT rühren. Danach kühlt man das Gemisch im Eisbad wieder auf 0°C ab und fügt 0.469 g Allylisothiocyanat (4.72 mmol) in 4 ml wasserfreiem Dimethylformamid gelöst, tropfenweise hinzu. Nach beendeter Zugabe entfernt man das Kühlbad und läßt das Gemisch noch 1,5 Stunden bei Raumtemperatur rühren. Zur Aufarbeitung gießt man das Gemisch in ca. 250 ml destilliertes Wasser und saugt den ausgefallenen orangefarbenen Feststoff ab. Mehrfache säulenchromatographische Reinigung (Laufmittelgemische Dichlormethan / Methanol) und anschließende Aufreinigung an der präparativen HPLC liefern einen gelben Feststoff.

Schmelzpunkt: 239-240°C (Zers.)
¹H-NMR (d₆-DMSO): δ = 4.40 (m, 2H), 5.30 (d, 1 H), 5.60 (d, 1 H), 6.07-6.17 (m, 1 H), 7.55-7.70 (m, 4H), 8.35 (d, 2H), 8.45 (d, 1 H), 9.50 (s, 1 H), 11.35 (s, 1 H), 12.55 (m, 1H).

Folgende Beispiele wurden gemäß Beispiel 1 und den allgemeinen Synthesevorschriften synthetisiert :

### Beispiel 2: 1-Allyl-3-(3-naphthalin-2-yl-pyrido[2,3-b]pyrazin-6-yl)-thioharnstoff

Smp.: 242-243°C (Zers.)
¹H-NMR (d₆-DMSO): δ = 4.42 (m, 2H), 5.37 (d, 1 H), 5.65 (d, 1 H), 6.07-6.19 (m, 1 H), 7.57-7.68 (m, 3H), 7.97-8.05 (m, 1 H), 8.07-8.19 (m, 2H), 8.40-8.52 (m, 2H), 8.99 (s, 1 H), 9.70 (s, 1 H), 11.36 (s, 1 H), 12.56 (t, 1H).

### Beispiel 3: 1-Allyl-3-[3-(4-methoxy-phenyl)-pyrido[2,3-b]pyrazin-6-yl]-thioharnstoff

Smp.: 240-241°C (Zers.)
¹H-NMR (d₆-DMSO): δ = 3.87 (s, 3H), 4.36-4.42 (m, 2H), 5.32 (d, 1 H), 5.60 (d, 1 H), 6.06-6.16 (m, 1 H), 7.16 (d, 2H), 7.60 (d, 1 H), 8.32 (d, 2H), 8.42 (d, 1 H), 9.56 (s, 1 H), 11.29 (s, 1 H), 12.56 (m, 1 H).

### Beispiel 4: 1-Allyl-3-[3-(4-hydroxy-phenyl)-pyrido[2,3-b]pyrazin-6-yl]-thioharnstoff-Hydrochlorid

Smp.: 160-161 °C (Zers.)
¹H-NMR (d₆-DMSO): δ = 4.36-4.43 (m, 2H), 5.31 (d, 1 H), 5.59 (d, 1 H), 6.05-6.16 (m, 1 H), 6.97 (d, 2H), 7.57 (d, 1 H), 8.20 (d, 2H), 8.40 (d, 1 H), 9.41 (s, 1 H), 10.17 (bs, 1 H), 11.24 (s, 1 H), 12.56 (m, 1 H).

### Beispiel 5: 1-(2-Methyl-allyl)-3-(3-phenyl-pyrido[2,3-b]pyrazin-6-yl)-thioharnstoff

Smp.: 225-226°C (Zers.)
¹H-NMR (d₆-DMSO): δ = 1.90 (s, 3H), 4.30-4.35 (m, 2H), 5.01 (s, 1 H), 5.22 (s, 1 H), 7.55-7.80 (m, 4H), 8.30-8.38 (m, 2H), 8.45 (d, 1 H), 9.52 (s, 1 H), 11.32 (s, 1 H), 12.65 (m, 1H).

### Beispiel 6: 1-(2-Methyl-allyl)-3-(3-naphthalin-2-yl-pyrido[2,3-b]pyrazin-6-yl)-thioharnstoff

Smp.: 239-240°C (Zers.)
¹H-NMR (d₆-DMSO): δ = 1.94 (s, 3H), 4.32 (m, 2H), 5.07 (s, 1 H), 5.28 (s, 1 H), 7.60-7.69 (m, 3H), 8.00-8.05 (m, 1 H), 8.07-8.12 (m, 1 H), 8.14 (d, 1 H), 8.42-8.51 (m, 2H), 8.98 (s, 1 H), 9.68 (s, 1 H), 11.32 (s, 1 H), 12.78 (m, 1 H).

### Beispiel 7: 1-[3-(4-Methoxy-phenyl)-pyrido[2,3-b]pyrazin-6-yl]-3-(2-methyl-allyl)-thioharnstoff

Smp.: 251-252°C (Zers.)
¹H-NMR (d₆-DMSO): δ = 1.92 (s, 3H), 3.85 (s, 3H), 4.27-4.35 (m, 2H), 5.02 (s, 1 H), 5.24 (s, 1 H), 7.15 (d, 2H), 7.58 (d, 1 H), 8.31 (d, 2H), 8.41 (d, 1 H), 9.46 (s, 1 H), 11.29 (s, 1 H), 12.68 (m, 1 H).

### Beispiel 8: 1-(3-Naphthalin-2-yl-pyrido[2,3-b]pyrazin-6-yl)-3-(4-nitro-phenyl)-thioharnstoff

Smp.: 260-261°C (Zers.)
¹H-NMR (d₆-DMSO): δ = 7.61-7.68 (m, 3H), 7.72 (d, 2H), 7.75 (d, 1H), 8.01-8.06 (m, 1 H), 8.16 (m, 2H), 8.26 (d, 2H), 8.53 (d, 1 H), 8.58 (d, 1 H), 9.04 (s, 1 H), 9.62 (s, 1 H), 9.76 (s, 1 H), 11.81 (s, 1 H).

### Beispiel 9: 1-[3-(4-Methoxy-phenyl)-pyrido[2,3-b]pyrazin-6-yl]-3-(4-nitro-phenyl)-thioharnstoff

Smp.: 250-251 °C (Zers.)
¹H-NMR (d₆-DMSO): δ = 3.85 (s, 3H), 7.17 (d, 2H), 7.71 (d, 2H), 8.21 (d, 2H), 8.22-8.27 (m, 1 H), 8.36-8.42 (m, 3H), 9.53 (s, 1 H), 9.65 (s, 1 H), 11.77 (s, 1 H).

### Beispiel 10: 1-tert.Butyl-3-(3-phenyl-pyrido[2,3-b]pyrazin-6-yl)-thioharnstoff

Smp.: 227°C (Zers.)
¹H-NMR (d₆-DMSO): δ = 1.65 (s, 9H), 7.53-7.69 (m, 4H), 8.34 (d, 2H), 8.41 (d, 1 H), 9.51 (s, 1 H), 10.98 (s, 1 H), 12.75 (s, 1 H).

### Beispiel 11: 1-Cyclopropyl-3-(3-phenyl-pyrido[2,3-b]pyrazin-6-yl)-thioharnstoff

Smp.: 233-234°C
¹H-NMR (d₆-DMSO): δ = 0.70-0.80 (m, 2H), 0.91-1.00 (m, 2H), 3.20-3.28 (m, 1H), 7.51-7.72 (m, 4H), 8.36 (d, 2H), 8.45 (d, 1 H), 9.52 (s, 1 H), 11.31 (s, 1 H), 12.45 (s, 1 H).

### Beispiel 12: 1-Methyl-3-(3-phenyl-pyrido[2,3-b]pyrazin-6-yl)-thioharnstoff

Smp.: 253-254°C
¹H-NMR (d₆-DMSO): δ = 3.25 (s, 3H), 7.59-7.67 (m, 4H), 8.38 (d, 2H), 8.46 (d, 1 H), 9.52 (s, 1 H), 11.31 (s, 1 H), 12.10 (s, 1 H).

### Beispiel 13: 1-Benzyl-3-(3-phenyl-pyrido[2,3-b]pyrazin-6-yl)-thioharnstoff

Smp.: 232-233°C
¹H-NMR (d₆-DMSO): δ = 4.96 (m, 2H), 7.37-7.48 (m, 3H), 7.54-7.67 (m, 6H), 8.32 (d, 2H), 8.47 (d, 1H), 9.52 (s, 1H), 11.43 (s, 1H), 12.91 (s, 1H).

### Beispiel 14: 1-(4-Fluoro-phenyl)-3-(3-phenyl-pyrido[2,3-b]pyrazin-6-yl)-thioharnstoff

Smp.: 225-226°C
¹H-NMR (d₆-DMSO): δ = 7.33 (m, 2H), 7.57-7.65 (m, 3H), 7.70-7.81 (m, 3H), 8.34 (d, 2H), 8.54 (d, 1H), 9.57 (s, 1H), 11.62 (s, 1H).

### Beispiel 15: 1-Cyclohexyl-3-(3-phenyl-pyrido[2,3-b]pyrazin-6-yl)-thioharnstoff

Smp.: 230-232°C
¹H-NMR (d₆-DMSO): δ = 1.50-1.75 (m, 6H), 1.80-2.00 (m, 4H), 7.55-7.70 (m, 4H), 8.37 (d, 2H), 8.45 (d, 1H), 9.55 (s, 1H), 11.20 (s, 1H), 12.80 (s, 1H).

### Beispiel 16: 1-lsopropyl-3-(3-phenyl-pyrido[2,3-b]pyrazin-6-yl)-thioharnstoff

Smp.: 229-230°C
¹H-NMR (d₆-DMSO): δ = 1.40 (d, 6H), 4.40-4.50 (m, 1H), 7.58-7.66 (m, 4H), 8.36 (d, 2H), 8.44 (d, 1H), 9.52 (s, 1H), 11.20 (s, 1H), 12.48 (s, 1H).

### Beispiel 17: 1-Furan-2-ylmethyl-3-(3-phenyl-pyrido[2,3-b]pyrazin-6-yl)-thioharnstoff

Smp.: 250°C (Zers.)
¹H-NMR (d₆-DMSO): δ = 4.95 (s, 2H), 6.55 (m, 1H), 6.68 (d, 1H), 7.59-7.68 (m, 4H), 7.74 (d, 1H), 8.37 (d, 2H), 8.48 (d, 1H), 9.55 (s, 1H), 11.45 (s, 1H), 12.83 (s, 1H).

### Beispiel 18: 1-Methyl-3-[3-(4-nitro-phenyl)-pyrido[2,3-b]pyrazin-6-yl)-thioharnstoff

Smp.: 270°C
¹H-NMR (d₆-DMSO): δ = 3.25 (s, 3H), 7.70 (d, 1H), 8.44 (d, 2H), 8.50 (d, 1H), 8.64 (d, 2H), 9.64 (s, 1H), 11.38 (s, 1H), 12.03 (s, 1H).

### Beispiel 19: 1-[3-(4-Hydroxy-phenyl)-pyrido[2,3-]pyrazin-6-yl]-3-methyl-thioharnstoff

Smp.: 282°C
¹H-NMR (d₆-DMSO): δ = 3.25 (s, 3H), 6.98 (d, 2H), 7.57 (d, 1H), 8.26 (d, 2H), 8.40 (d, 1H), 9.45 (s, 1H), 10.18 (s, 1H), 11.25 (s, 1H), 12.10 (s, 1H).

### Beispiel 20: 1-Allyl-3-[3-(4-nitro-phenyl)-pyrido[2,3-b]pyrazin-6-yl]-thioharnstoff

Smp.: 244°C (Zers.)
¹H-NMR (d₆-DMSO): δ = 4.40 (s, 2H), 5.36 (d, 1H), 5.59 (d, 1H), 6.08-6.15 (m, 1 H), 7.71 (d, 1H), 8.46 (d, 2H), 8.51 (d, 1H), 8.60 (d, 2H), 9.64 (s, 1H), 11.45 (s, 1H), 12.51 (t, 1H).

### Beispiel 21: 4-[6-(3-Allyl-thioharnstoff)-pyrido[2,3-b]pyrazin-3-yl]-benzoesäureethylester

Smp.: 223-224°C
¹H-NMR (d₆-DMSO): δ = 1.39 (t, 3H), 4.35-4.42 (m, 4H), 5.35 (d, 1H), 5.60 (d, 1H), 6.08-6.15 (m, 1H), 7.68 (d, 1H), 8.17 (d, 2H), 8.47 (d, 2H), 8.50 (d, 1H), 9.60 (s, 1H), 11.40 (s, 1H), 12.52 (t, 1H).

### Beispiel 22: 1-Allyl-3-[3-(3-hydroxy-phenyl)-pyrido[2,3-b]pyrazin-6-yl]-thioharnstoff

Smp.: 205°C (Zers.)
¹H-NMR (d₆-DMSO): δ = 4.41 (s, 2H), 5.33 (d, 1H), 5.58 (d, 1H), 6.07-6.15 (m, 1H), 6.99 (d, 1H), 7.42 (t, 1H), 7.64 (d, 1H), 7.72 (s, 1H), 7.77 (d, 1H), 8.46 (d, 1H), 9.45 (s, 1H), 9.80 (s, 1H), 11.37 (s, 1H), 12.55 (s, 1H).

### Beispiel 23: 1-Allyl-3-(3-benzo[1,3]dioxol-5-yl-pyrido[2,3-b]pyrazin-6-yl)-thioharnstoff

Smp.: 218-220°C (Zers.)
¹H-NMR (d₆-DMSO): δ = 4.40 (s, 2H), 5.31 (d, 1H), 5.60 (d, 1H), 6.08-6.20 (m, 3H), 7.16 (d, 1H), 7.61 (d, 1H), 7.90 (s, 1H), 7.96 (d, 1H), 8.43 (d, 1H), 9.49 (s, 1H), 11.34 (s, 1H), 12.58 (s, 1H).

### Beispiel 24: 1-[3-(4-Hydroxy-phenyl)-pyrido[2,3-b]pyrazin-6-yl]-3-prop-2-ynyl-thioharnstoff

Smp.: 350 °C (Zers.)
¹H-NMR (d₆-DMSO): δ = 2.09 (s, 1H), 2.44 (s, 2H), 6.99 (d, 2H), 7.19 (s, 1H), 7.44 (s, 1H), 8.24 (d, 2H), 8.26 (d, 1H), 9.29 (s, 1H), 10.08 (s, 1H), 11.81 (s, 1H).

### Beispiel 25: 1-Allyl-3-[3-(4-hydroxy-phenyl)-pyrido[2,3-b]pyrazin-6-yl]-thioharnstoff

Smp.: 230 °C (Zers.)
¹H-NMH (d₆-DMSO): δ = 4.40 (s, 2H), 5.34 (d, 1H), 5.60 (d, 1H), 6.07-6.15 (m, 1H), 6.98 (d, 2H), 7.58 (d, 1H), 8.24 (d, 2H), 8.42 (d, 1H), 9.45 (s, 1H), 10.19 (s, 1H), 11.34 (s, 1H), 12.60 (s, 1H).

### Beispiel 26: 1-[3-(4-Hydroxy-phenyl)-pyrido[2,3-b]pyrazin-6-yl]-3-((propenyl)-thioharnstoff

Smp.:
¹H-NMR (d₆-DMSO): δ = 2.12 (d, 3H), 5.17 (m, 1H), 6.96 (d, 2H), 7.22-7.26 (m, 1H), 7.59 (d, 1H), 8.25 (d, 2H), 8.45 (d, 1H), 9.48 (s, 1H), 10.20 (s, 1H), 11.56 (s, 1H), 14.67 (s, 1H).

### Beispiel 27: 1-Allyl-3-[2,3-bis-(4-hydroxy-phenyl)-pyrido[2,3-b]pyrazin-6-yl]-thioharnstoff

Smp.: 270 °C (Zers.)
¹H-NMR (d₆-DMSO): δ = 4.40 (s, 2H), 5.25 (d, 1H), 5.50 (d, 1H), 6.02-6.13 (m, 1H), 6.74 (d, 2H), 6.76 (d, 2H), 7.31 (d, 2H), 7.36 (d, 2H), 7.62 (d, 1H), 8.42 (d, 1H), 9.78 (s, 1H), 9.85 (s, 1H), 11.30 (s, 1H), 12.47 (s, 1H).

### Beispiel 28: 1-[2,3-Bis-(4-hydroxy-phenyl)-pyrido[2,3-b]pyrazin-6-yl]-3-((propenyl)-thioharnstoff

Smp.: 240°C (Zers.)
¹H-NMR (d₆-DMSO): δ = 2.05 (d, 3H), 5.10-5.18 (m, 1H), 6.74 (d, 2H), 6.76 (d, 2H), 7.20-7.26 (m, 1H), 7.34 (d, 2H), 7.39 (d, 2H), 7.63 (d, 1H), 8.45 (d, 1H), 9.79 (s, 1H), 9.89 (s, 1H), 11.55 (s, 1H), 14.56 (d, 1H).

### Beispiel 29: 1-Allyl-3-[2-(4-hydroxy-phenyl)-pyrido[2,3-b]pyrazin-6-yl]-thioharnstoff

Smp.: 260°C (Zers.)
¹H-NMR (d₆-DMSO): δ = 4.40 (s, 2H), 5.28 (d, 1H), 5.48 (d, 1H), 6.03-6.12 (m, 1H), 6.96 (d, 2H), 7.66 (d, 1H), 8.16 (d, 2H), 8.43 (d, 1H), 9.52 (s, 1H), 10.06 (s, 1H), 11.31 (s, 1H), 12.40 (s, 1 H).

### Beispiel 30: 1-Allyl-3-[3-(4-nitro-phenyl)-pyrido[2,3-b]pyrazin-7-yl]-thioharnstoff

Smp.: 250 °C (Zers.)
¹H-NMR (d₆-DMSO): δ = 4.23 (s, 2H), 5.19 (d, 1H), 5.29 (d, 1H), 5.90-6.00 (m, 1H), 8.46 (d, 2H), 8.55 (s, 1H), 8.64 (d, 2H), 8.92 (s, 1H), 9.23 (s, 1H), 9.77 (s, 1H), 10.35 (s, 1H).

### Beispiel 31: 1-Cyclopropyl-3-(3-phenyl-pyrido[2,3-b]pyrazin-6-yl)-harnstoff

Smp.: 158-160 °C
¹H-NMR (d₆-DMSO): δ = 0.52-0.60 (m, 2H), 0.72-0.82 (m, 2H), 2.70-2.79 (m, 1H), 7.57-7.65 (m, 3H), 7.71 (d, 1H), 8.34 (d, 2H), 8.38 (d, 1H), 9.21 (s, 1H), 9.46 (s, 1H), 10.12 (s, 1H).

### Beispiel 32: 1-Allyl-3-[3-(4-hydroxy-phenyl)-pyrido[2,3-b]pyrazin-6-yl]-harnstoff

Smp.: 240 °C (Zers.)
¹H-NMR (d₆-DMSO): δ = 3.98 (s, 2H), 5.19 (d, 1H), 5.37 (d, 1H), 5.96-6.05 (m, 1H), 6.97 (d, 2H), 7.59 (d, 1H), 8.22 (d, 2H), 8.33 (d, 1H), 9.38 (s, 1H), 9.45 (s, 1H), 10.13 (s, 1H), 10.18 (s, 1H).

### Beispiel 33: 1-(3-Phenyl-pyrido[2,3-b]pyrazin-6-yl)-3-p-tolyl-harnstoff

Smp.: 298-299°C
¹H-NMR (d₆-DMSO): δ = 2.29 (s, 3H), 7.20 (d, 2H), 7.52 (d, 2H), 7.59-7.67 (m, 3H), 7.80 (d, 1H), 8.38 (d, 2H), 8.44 (d, 1H), 9.59 (s, 1H), 10.36 (s, 1H), 11.46 (s, 1H).

### Beispiel 34: 1-(4-Chloro-3-trifluoromethyl-phenyl)-3-(3-phenyl-pyrido[2,3-b]pyrazin-6-yl)-harnstoff

Smp.: 250°C
¹H-NMR (d₆-DMSO): δ = 7.58-7.67 (m, 3H), 7.74 (d, 1H), 7.80 (d, 1H), 7.87 (d, 1H), 8.21 (s, 1H), 8.39 (d, 2H), 8.48 (d, 1H), 9.53 (s, 1H), 10.55 (s, 1H), 11.82 (s, 1H).

### Beispiel 35: 1-(2-Morpholin-4-yl-ethyl)-3-(3-phenyl-pyrido[2,3-b]pyrazin-6-yl)-harnstoff

Smp.: 226°C
¹H-NMR (d₆-DMSO): δ = 2.45-2.67 (m, 6H), 3.40-3.48 (m, 2H), 3.60-3.69 (m, 4H), 7.55-7.70 (m, 4H), 8.30-8.40 (m, 3H), 9.29 (s, 1H), 9.42 (s, 1H), 10.18 (s, 1H).

### Beispiel 36: 1-Phenethyl-3-(3-phenyl-pyrido[2,3-]pyrazin-6-yl)-harnstoff

Smp.: 250°C (Zers.)
¹H-NMR (d₆-DMSO): δ = 2.88-2.95 (m, 2H), 3.52-3.60 (m, 2H), 7.18 (t, 1H), 7.28 (t, 2H), 7.42 (d, 2H), 7.58-7.68 (m, 4H), 8.37 (d, 3H), 9.25 (s, 1 H), 9.48 (s, 1 H), 10.18 (s, 1 H).

### Beispiel 37: 1-(2,3-Di-pyridin-2-yl-pyrido[2,3-b]pyrazin-6-yl)-3-ethyl-harnstoff

Smp.: 236-237 °C
¹H-NMR (d₆-DMSO): δ = 1.13-1.22 (m, 3H), 3.28-3.39 (m, 2H), 3.60-3.69 (m, 4H), 7.31-7.39 (m, 2H), 7.79 (d, 1 H), 7.91-7.99 (m, 4H), 8.26 (d, 1 H), 8.29 (d, 1 H), 8.47 (d, 1 H), 9.08 (s, 1 H), 10.20 (s, 1 H).

### Beispiel 38: 1-(2,3-Dimethyl-pyrido[2,3-b]pyrazin-6-yl)-3-ethyl-harnstoff

Smp.: 246-248 °C
¹H-NMR (d₆-DMSO): δ = 1.17 (t, 3H), 2.64 (s, 3H), 2.67 (s, 3H), 3.24-3.40 (m, 2H), 7.55 (d, 1 H), 8.24 (d, 1 H), 9.14 (s, 1 H), 9.91 (s, 1 H).

**Tabelle 1:**

| | | | | | |
|---|---|---|---|---|---|
| 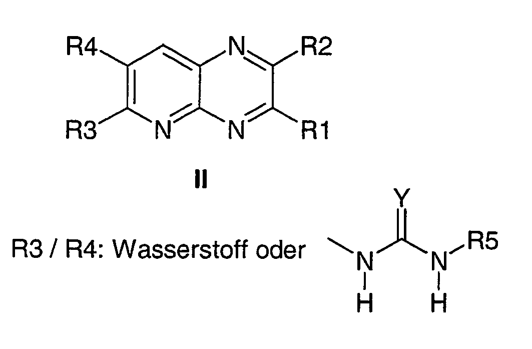 | | | | | |

| Beispiel | R1 | R2 | R3 | R4 | R5 |
|---|---|---|---|---|---|
| 1 | Ph | H | Y = S | H | -CH₂CH=CH₂ |
| 2 | 2-Naphthyl | H | Y = S | H | -CH₂CH=CH₂ |
| 3 | 4-MeO-Ph | H | Y = S | H | -CH₂CH=CH₂ |
| 4 | 4-HO-Ph | H | Y = S | H | -CH₂CH=CH₂, HCl-Salz |
| 5 | Ph | H | Y = S | H | -CH₂C(CH₃)=CH₂ |
| 6 | 2-Naphthyl | H | Y = S | H | -CH₂C(CH₃)=CH₂ |
| 7 | 4-MeO-Ph | H | Y = S | H | -CH₂C(CH₃)=CH₂ |
| 8 | 2-Naphthyl | H | Y = S | H | -Ph-*p*-NO₂ |
| 9 | 4-MeO-Ph | H | Y = S | H | -Ph-*p*-NO₂ |
| 10 | Ph | H | Y = S | H | -C(CH₃)₃ |
| 11 | Ph | H | Y=S | H | -cyclopropyl |
| 12 | Ph | H | Y = S | H | -CH₃ |
| 13 | Ph | H | Y = S | H | -benzyl |
| 14 | Ph | H | Y = S | H | -Ph-P-F |
| 15 | Ph | H | Y = S | H | -cyclohexyl |
| 16 | Ph | H | Y = S | H | -CH(CH₃)₂ |
| 17 | Ph | H | Y = S | H | -1-Furan-2-ylmethyl |
| 18 | 4-NO₂-Ph | H | Y = S | H | -CH₃ |
| 19 | 4-HO-Ph | H | Y = S | H | -CH₃ |
| 20 | 4-NO₂-Ph | H | Y = S | H | -CH₂CH=CH₂ |
| 21 | 4-CO₂Et-Ph | H | Y = S | H | -CH₂CH=CH₂ |
| 22 | 3-HO-Ph | H | Y = S | H | -CH₂CH=CH₂ |
| 23 | 3-Benzo[1,3]-dioxol-5-yl | H | Y = S | H | -CH₂CH=CH₂ |
| 24 | 4-HO-Ph | H | Y = S | H | -propin-2-yl |
| 25 | 4-HO-Ph | H | Y = S | H | -CH₂CH=CH₂ |
| 26 | 4-HO-Ph | H | Y = S | H | -CH=CHCH₃ |
| 27 | 4-HO-Ph | 4-HO-Ph | Y = S | H | -CH₂CH=CH₂ |
| 28 | 4-HO-Ph | 4-HO-Ph | Y = S | H | -CH=CHCH₃ |
| 29 | H | 4-HO-Ph | Y = S | H | -CH₂CH=CH₂ |
| 30 | 4-NO₂-Ph | H | H | Y = S | -CH₂CH=CH₂ |
| 31 | Ph | H | Y = O | H | -cyclopropyl |
| 32 | 4-HO-Ph | H | Y= O | H | -CH₂CH=CH₂ |
| 33 | Ph | H | Y = O | H | -*p*-tolyl |
| 34 | Ph | H | Y = O | H | -Ph-*p*-Cl-*m*-CF₃ |
| 35 | Ph | H | Y = O | H | -CH₂CH₂-Morpholin-4-yl |
| 36 | Ph | H | Y = O | H | -phenethyl |
| 37 | 2-Pyridinyl | 2-Pyridinyl | Y = O | H | -ethyl |
| 38 | Methyl | Methyl | Y = O | H | -ethyl |

### Biologische Wirkungen der erfindungsgemäßen Verbindungen

Die inhibitorische Wirkung der erfindungsgemäßen Verbindungen wurde an folgenden humanen Serin/ Threonin- und Tyrosinkinasen in klassischen Kinaseassays getestet: PKB/Akt1, c-Raf-Mek-Erk, B-Raf-Mek-Erk, Mek-Erk, MAPKs, PDGFRbeta, Flt-3, c-Kit, c-Abl, KDR, FGFR1 und IGF1R. Eingesetzt wurden sowohl die Volllängenkinasen als auch verkürzte Fragmente - mindestens aber die cytoplasmatischen Kinasedomänen. Die Kinasen wurden als rekombinante Fusionsproteine mit GST-(Glutathion-S-Transferase) oder HIS-Tag in Sf9-Zellkultur hergestellt. Je nach Substrattyp wurden die verschiedenen Kinasereaktionen in Sandwich-ELISA-Formaten oder mittels einfacher Substratadsorptionstest auf 96-Well Flashplates (Perkin Elmer) durchgeführt.

Nachfolgend wird die Substanztestung an der c-Raf-Mek-Erk-Kaskade genauer beschrieben. Ausgewählte Testergebnisse des c-Raf-Mek-Erk-Assays sind anschließend aufgeführt.

### Prozedere: c-Raf-Mek-Erk-ELISA

Potentielle Inhibitoren wurden zunächst bei einer Konzentration von 20µg/ml in initialen Single-Dose-Bestimmungen auf 96er Mikrotiterplatten (MTPs) untersucht. Substanzen >70% Inhibition wurden für Dosis-Wirkungsstudien eingesetzt.
Die Rekonstitution der c-Raf-Mek-Erk-Kaskade wurde mithilfe eines zellfreien ELI-SAs quantifiziert. Verwendet wurden folgende rekombinant hergestellte Kinaseproteine: 1.) konstitutiv aktive GST-c-Raf-DD aus Sf9-Zellen 2.) nicht aktive GST-Mek1 aus E. coli und 3.) nicht aktive His-Erk2 aus E. coli.
Ein typischer Kinaseansatz wurde in einem finalen Volumen von 50,u1 mit je 20-150ng Raf-, Mek-, Erk-Protein, 1 mM ATP, 10mM MgCl₂, 150mM NaCl, 25mM beta-Glycerophosphat, 25mM Hepes pH 7.5 durchgeführt. Vor der Kinasereaktion wurden die Testsubstanzen jeweils für 30 Minuten bei Raumtemperatur mit jedem der drei Kinaseproteine einzeln vorinkubiert. Für die Kinasereaktion wurden die mit Testsubstanz vorinkubierten Kinasen zusammengeführt und für 30 Minuten bei 26°C inkubiert. Durch eine finale Konzentration von 2% SDS und 10 Minuten bei 50°C im Heizblock wurde die Reaktion gestoppt.

Zur Immundetektion wurden die Reaktionsansätze auf anti-Erk-Ak(K-23, Santa Cruz Biotechnology)-beschichtete 96er MTPs übertragen, 60 Minuten bei Raumtemperatur inkubiert und 3x mit TBST gewaschen. Anti-phospho-Erk-Ak (#9106, New England Biolabs) 1:500 in 50µl TBST/1% BSA wurde zugegeben und über Nacht bei 4°C inkubiert. Nach 3x Wasch der MTPs mit TBST wurde mit sekundärem anti-Maus-IgG^{POD}-Konjugat (#NA931, Pharmacia) 1:2500 versetzt, 1h bei Raumtemperatur inkubiert und wiederum 3x mit TBST gewaschen. Zur kolorimetrischen Detektion der Kinasereaktion wurden je 50µl OPD (o-Phenyldiamin-dihydrochlorid)-Färbepuffer auf die Kavitäten pipettiert und 30 Minuten bei 37°C inkubiert. Die Farbreaktion wurde anschließend im ELISA-Reader bei 492nm bestimmt.
Die experimentelle Bestimmung von Dosis-Wirkungskurven erfolgte mittels des selben Versuchsaufbaus bei 10 halblogarithmisch abgestuften Konzentrationen von 31.6pM-100µM. Die IC₅₀-Werte wurden in GraphPadPrism kalkuliert.

Die erfindungsgemäßen Verbindungen zeigen eine effektive Inhibition der Erk-Phosphorylierung mit IC₅₀-Werten bis zu 400nM (siehe Ausführungsbeispiele 4 und 12).

| Ausführungsbeispiel | IC₅₀ (µM) |
|---|---|
| 1 | ca. 1.0 |
| 2 | 16 |
| 3 | ca. 1.0 |
| 4 | 0.4 |
| 5 | ca. 1.0 |
| 6 | ca. 100 |
| 7 | 43 |
| 8 | > 100 |
| 9 | > 100 |
| 10 | > 100 |
| 11 | 0.9 |
| 12 | 0.4 |
| 13 | > 100 |
| 14 | ca. 50 |
| 33 | > 100 |
| 34 | > 100 |
| 35 | 15 |

## Patentansprüche

1. Neue Pyrido[2,3-b]pyrazin-Derivate gemäß der allgemeinen Formel 1 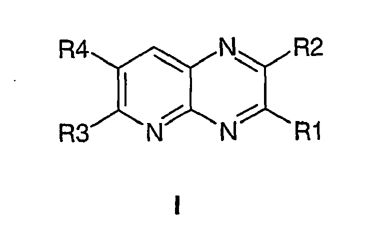 worin die Substituenten R1-R4 folgende Bedeutung haben :
R1 und R2 können unabhängig voneinander:
(i) Wasserstoff
(ii) Hydroxyl
(iii) Halogen
(iv) Alkyl, wobei der Alkylrest gesättigt ist und aus 1 bis 8 C-Atomen bestehen kann,
(v) unsubstituiertes oder substituiertes Aryl, wobei der Arylrest mit F, Cl, Br, I, CF₃, CN, NH₂, NH-Alkyl, NH-Cycloalkyl, NH-Heterocyclyl, NH-Aryl, NH-Heteroaryl, NH-Alkyl-Cycloalkyl, NH-Alkyl-Heterocyclyl, NH-Alkyl-Aryl, NH-Alkyl-Heteroaryl, NH-Alkyl-NH₂, NH-Alkyl-OH, N(Alkyl)₂, NHC(O)-Alkyl, NHC(O)-Cycloalkyl, NHC(O)-Heterocyclyl, NHC(O)-Aryl, NHC(O)-Heteroaryl, NHC(O)-Alkyl-Aryl, NHC(O)-Alkyl-Heteroaryl, NHSO₂-Alkyl, NHSO₂-Cycloalkyl, NHSO₂-Heterocyclyl, NHSO₂-Aryl, NHSO₂-Heteroaryl, NHSO₂-Alkyl-Aryl, NHSO₂-Alkyl-Heteroaryl, NO₂, SH, S-Alkyl, S-Aryl, S-Heteroaryl, OH, OCF₃, O-Alkyl, O-Cycloalkyl, O-Heterocyclyl, O-Aryl, O-Heteroaryl, 0-Alkyl-Cycloalkyl, O-Alkyl-Heterocyclyl, O-Alkyl-Aryl, O-Alkyl-Heteroaryl, 0-Alkyl-OH, O-(CH2)ₙ-O, OC(O)-Alkyl, OC(O)-Cycloalkyl, OC(O)-Heterocyclyl, OC(O)-Aryl, OC(O)-Heteroaryl, OC(O)-Alkyl-Aryl, OC(O)-Alkyl-Heteroaryl, OSO₃H, OSO₂-Alkyl, OSO₂-Cycloalkyl, OSO₂-Heterocyclyl, OSO₂-Aryl, OSO₂-Heteroaryl, OSO₂-Alkyl-Aryl, OSO₂-Alkyl-Heteroaryl, OP(O)(OH)₂, C(O)-Alkyl, C(O)-Aryl, C(O)-Heteroaryl, CO₂H, CO₂-Alkyl, CO₂-Cycloalkyl, CO₂-Heterocyclyl, CO₂-Aryl, CO₂-Heteroaryl, CO₂-Alkyl-Cycloalkyl, CO₂-Alkyl-Heterocyclyl, CO₂-Alkyl-Aryl, CO₂-Alkyl-Heteroaryl, C(O)-NH₂, C(O)NH-Alkyl, C(O)NH-Cycloalkyl, C(O)NH-Heterocyclyl, C(O)NH-Aryl, C(O)NH-Heteroaryl, C(O)NH-Alkyl-Cycloalkyl, C(O)NH-Alkyl-Heterocyclyl, C(O)NH-Alkyl-Aryl, C(O)NH-Alkyl-Heteroaryl, C(O)N(Alkyl)₂, C(O)N(Cycloalkyl)₂, C(O)N(Aryl)₂, C(O)N(Heteroaryl)₂, SO-Alkyl, SO-Aryl, SO₂-Alkyl, SO₂-Aryl, SO₂NH₂, SO₂NH-Alkyl, SO₂NH-Aryl, SO₂NH-Heteroaryl, SO₂NH-Alkyl-Aryl, SO3H, SO₂O-Alkyl, SO₂O-Aryl, SO₂O-Alkyl-Aryl, Alkyl, Cycloalkyl, Heterocyclyl, Aryl oder Heteroaryl, ein- oder mehrfach, gleich oder verschieden substituiert sein kann, n den Wert 1, 2 oder 3 annehmen kann, und die Alkyl-, Cycloalkyl-, Heterocyclyl-, Aryl-, Heteroaryl-, Alkyl-Cycloalkyl-, Alkyl-Heterocyclyl-, Alkyl-Aryl- und Alkyl-Heteroarylsubstituenten ihrerseits wiederum substituiert sein können,
(vi) unsubstituiertes oder substituiertes Heteroaryl, wobei der Heteroarylrest mit F, Cl, Br, I, CF₃, CN, NH₂, NH-Alkyl, NH-Cycloalkyl, NH-Heterocyclyl, NH-Aryl, NH-Heteroaryl, NH-Alkyl-Cycloalkyl, NH-Alkyl-Heterocyclyl, NH-Alkyl-Aryl, NH-Alkyl-Heteroaryl, NH-Alkyl-NH₂, NH-Alkyl-OH, N(Alkyl)₂, NHC(O)-Alkyl, NHC(O)-Cycloalkyl, NHC(O)-Heterocyclyl, NHC(O)-Aryl, NHC(O)-Heteroaryl, NHC(O)-Alkyl-Aryl, NHC(O)-Alkyl-Heteroaryl, NHSO₂-Alkyl, NHSO₂-Cycloalkyl, NHSO₂-Heterocyclyl, NHSO₂-Aryl, NHSO₂-Heteroaryl, NHSO₂-Alkyl-Aryl, NHSO₂-Alkyl-Heteroaryl, NO₂, SH, S-Alkyl, S-Aryl, S-Heteroaryl, OH, OCF₃, O-Alkyl, O-Cycloalkyl, O-Aryl, O-Heteroaryl, O-Alkyl-Cycloalkyl, 0-Alkyl-Heterocyclyl, O-Alkyl-Aryl, O-Alkyl-Heteroaryl, OC(O)-Alkyl, OC(O)-Cycloalkyl, OC(O)-Heterocyclyl, OC(O)-Aryl, OC(O)-Heteroaryl, OC(O)-Alkyl-Aryl, OC(O)-Alkyl-Heteroaryl, OSO₃H, OSO₂-Alkyl, OSO₂-Cycloalkyl, OSO₂-Heterocyclyl, OSO₂-Aryl, OSO₂-Heteroaryl, OSO₂-Alkyl-Aryl, OSO₂-Alkyl-Heteroaryl, OP(O)(OH)₂, C(O)-Alkyl, C(O)-Aryl, C(O)-Heteroaryl, CO₂H, CO₂-Alkyl, CO₂-Cycloalkyl, CO₂-Heterocyclyl, CO₂-Aryl, CO₂-Heteroaryl, CO₂-Alkyl-Cycloalkyl, CO₂-Alkyl-Heterocyclyl, CO₂-Alkyl-Aryl, CO₂-Alkyl-Heteroaryl, C(O)-NH₂, C(O)NH-Alkyl, C(O)NH-Cycloalkyl, C(O)NH-Heterocyclyl, C(O)NH-Aryl, C(O)NH-Heteroaryl, C(O)NH-Alkyl-Cycloalkyl, C(O)NH-Alkyl-Heterocyclyl, C(O)NH-Alkyl-Aryl, C(O)NH-Alkyl-Heteroaryl, C(O)N(Alkyl)₂, C(O)N(Cycloalkyl)₂, C(O)N(Aryl)₂, C(O)N(Heteroaryl)₂, SO₂NH₂, SO₂NH-Alkyl, SO₂NH-Aryl, SO₂NH-Heteroaryl, SO₂NH-Alkyl-Aryl, S03H, SO₂O-Alkyl, SO₂O-Aryl, SO₂O-Alkyl-Aryl, Alkyl, Cycloalkyl, Heterocyclyl, Aryl oder Heteroaryl, ein- oder mehrfach, gleich oder verschieden substituiert sein kann, und die Alkyl-, Cycloalkyl-, Heterocyclyl-, Aryl- und Heteroarylsubstituenten ihrerseits wiederum substituiert sein können,
(vii) OR5, wobei R5 Alkyl, Cycloalkyl, Heterocyclyl, Aryl, Heteroaryl, Alkyl-Cycloalkyl, Alkyl-Heterocyclyl, Alkyl-Aryl oder Alkyl-Heteroaryl sein kann, und die Alkyl-, Cycloalkyl-, Heterocyclyl-, Aryl-, Heteroaryl-, Alkyl-Cycloalkyl, Alkyl-Heterocyclyl, Alkyl-Aryl oder Alkyl-Heteroarylsubstituenten ihrerseits wiederum substituiert sein können,
(viii) SR6, wobei R6 Alkyl, Cycloalkyl, Heterocyclyl, Aryl, Heteroaryl, Alkyl-Cycloalkyl, Alkyl-Heterocyclyl, Alkyl-Aryl oder Alkyl-Heteroaryl sein kann, und die Alkyl-, Cycloalkyl-, Heterocyclyl-, Aryl- und Heteroaryl-, Alkyl-Cycloalkyl-, Alkyl-Heterocyclyl-, Alkyl-Aryl- oder Alkyl-Heteroarylsubstituenten ihrerseits wiederum substituiert sein können,
(ix) NR7R8, wobei R7 und R8 unabhängig voneinander Wasserstoff, Alkyl, Cycloalkyl, Heterocyclyl, Aryl, Heteroaryl, Alkyl-Cycloalkyl, Alkyl-Heterocyclyl, Alkyl-Aryl oder Alkyl-Heteroaryl sein können, und die Alkyl-, Cycloalkyl-, Heterocyclyl-, Aryl- und Heteroaryl-, Alkyl-Cycloalkyl, Alkyl-Heterocyclyl, Alkyl-Aryl oder Alkyl-Heteroarylsubstituenten ihrerseits wiederum substituiert sein können, oder R7 und R8 zusammen Cycloalkyl oder Heterocyclyl bedeuten, wobei Cycloalkyl und Heterocyclyl ihrerseits wiederum substituiert sein können,
bedeuten.
R3 und R4 können unabhängig voneinander Wasserstoff oder NR9R10 bedeuten, unter der Voraussetzung, dass, wenn R3 = NR9R10 ist, R4 = H ist, und wenn R4 = NR9R10 ist, R3 = H ist,
wobei R9 Wasserstoff, Alkyl, Cycloalkyl, Heterocyclyl, Aryl, Heteroaryl, Alkyl-Cycloalkyl, Alkyl-Heterocyclyl, Alkyl-Aryl oder Alkyl-Heteroaryl sein kann, und die Alkyl-, Cycloalkyl-, Heterocyclyl-, Aryl- und Heteroaryl-, Alkyl-Cycloalkyl, Alkyl-Heterocyclyl, Alkyl-Aryl oder Alkyl-Heteroarylsubstituenten ihrerseits wiederum substituiert sein können,
und R10:
-C(Y)NR11R12 bedeuten kann, wobei Y = O, S und R11 und R12 unabhängig voneinander
(i) Wasserstoff,
(ii) unsubstituiertes oder substituiertes Alkyl, wobei der Alkylrest mit F, Cl, Br, I, CF₃, CN, NH₂, NH-Alkyl, NH-Cycloalkyl, NH-Heterocyclyl, NH-Aryl, NH-Heteroaryl, NH-Alkyl-Cycloalkyl, NH-Alkyl-Heterocyclyl, NH-Alkyl-Aryl, NH-Alkyl-Heteroaryl, N(Alkyl)₂, NHC(O)-Alkyl, NHC(O)-Cycloalkyl, NHC(O)-Heterocyclyl, NHC(O)-Aryl, NHC(O)-Heteroaryl, NHC(O)-Alkyl-Aryl, NHC(O)-Alkyl-Heteroaryl, NHSO₂-Alkyl, NHSO₂-Cycloalkyl, NHSO₂-Heterocyclyl, NHSO₂-Aryl, NHSO₂-Heteroaryl, NHSO₂-Alkyl-Aryl, NHSO₂-Alkyl-Heteroaryl, NO₂, SH, S-Alkyl, S-Cycloalkyl, S-Heterocyclyl, S-Aryl, S-Heteroaryl, OH, OCF₃, O-Alkyl, O-Cycloalkyl, O-Heterocyclyl, O-Aryl, O-Heteroaryl, O-Alkyl-Cycloalkyl, O-Alkyl-Heterocyclyl, O-Alkyl-Aryl, O-Alkyl-Heteroaryl, OC(O)-Alkyl, OC(O)-Cycloalkyl, OC(O)-Heterocyclyl, OC(O)-Aryl, OC(O)-Heteroaryl, OC(O)-Alkyl-Aryl, OC(O)-Alkyl-Heteroaryl, OSO₃H, OSO₂-Alkyl, OSO₂-Cycloalkyl, OSO₂-Heterocyclyl, OSO₂-Aryl, OSO₂-Heteroaryl, OSO₂-Alkyl-Aryl, OSO₂-Alkyl-Heteroaryl, OP(O)(OH)₂, C(O)-Alkyl, C(O)-Aryl, C(O)-Heteroaryl, CO₂H, CO₂-Alkyl, CO₂-Cycloalkyl, CO₂-Heterocyclyl, CO₂-Aryl, CO₂-Heteroaryl, CO₂-Alkyl-Cycloalkyl, CO₂-Alkyl-Heterocyclyl, CO₂-Alkyl-Aryl, CO₂-Alkyl-Heteroaryl, C(O)-NH₂, C(O)NH-Alkyl, C(O)NH-Cycloalkyl, C(O)NH-Heterocyclyl, C(O)NH-Aryl, C(O)NH-Heteroaryl, C(O)NH-Alkyl-Cycloalkyl, C(O)NH-Alkyl-Heterocyclyl, C(O)NH-Alkyl-Aryl, C(O)NH-Alkyl-Heteroaryl, C(O)N(Alkyl)₂, C(O)N(Cycloalkyl)₂, C(O)N(Aryl)₂, C(O)N(Heteroaryl)₂, SO-Alkyl, SO-Aryl, SO₂-Alkyl, SO₂-Aryl, SO₂NH₂, SO₂NH-Alkyl, SO₂NH-Aryl, SO₂NH-Heteroaryl, SO₂NH-Alkyl-Aryl, SO₃H, SO₂O-Alkyl, SO₂O-Aryl, SO₂O-Alkyl-Aryl, Cycloalkyl, Heterocyclyl, Aryl oder Heteroaryl, ein- oder mehrfach, gleich oder verschieden substituiert sein kann,
(iii) unsubstituiertes oder substituiertes Cycloalkyl, wobei der Cycloalkylrest mit F, Cl, Br, I, NH₂, NH-Alkyl, NH-Cycloalkyl, NH-Heterocyclyl, NH-Aryl, NH-Heteroaryl, NH-Alkyl-Aryl, NH-Alkyl-Heteroaryl, N(Alkyl)₂, NHC(O)-Alkyl, NHC(O)-Cycloalkyl, NHC(O)-Heterocyclyl, NHC(O)-Aryl, NHC(O)-Heteroaryl, NHC(O)-Alkyl-Aryl, NHC(O)-Alkyl-Heteroaryl, NHSO₂-Alkyl, NHSO₂-Cycloalkyl, NHSO₂-Heterocyclyl, NHSO₂-Aryl, NHSO₂-Heteroaryl, NHSO₂-Alkyl-Aryl, NHSO₂-Alkyl-Heteroaryl, OH, O-Alkyl, O-Cycloalkyl, O-Heterocyclyl, O-Aryl, O-Heteroaryl, O-Alkyl-Aryl, O-Alkyl-Heteroaryl, OC(O)-Alkyl, OC(O)-Cycloalkyl, OC(O)-Heterocyclyl, OC(O)-Aryl, OC(O)-Heteroaryl, OC(O)-Alkyl-Aryl, OC(O)-Alkyl-Heteroaryl, OSO₃H, OSO₂-Alkyl, OSO₂-Cycloalkyl, OSO₂-Heterocyclyl, OSO₂-Aryl, OSO₂-Heteroaryl, OSO₂-Alkyl-Aryl, OSO₂-Alkyl-Heteroaryl, OP(O)(OH)₂, CO₂H, CO₂-Alkyl, CO₂-Cycloalkyl, CO₂-Heterocyclyl, CO₂-Aryl, CO₂-Heteroaryl, CO₂-Alkyl-Cycloalkyl, CO₂-Alkyl-Heterocyclyl, CO₂-Alkyl-Aryl, CO₂-Alkyl-Heteroaryl, C(O)-NH₂, C(O)NH-Alkyl, C(O)NH-Cycloalkyl, C(O)NH-Heterocyclyl, C(O)NH-Aryl, C(O)NH-Heteroaryl, C(O)NH-Alkyl-Cycloalkyl, C(O)NH-Alkyl-Heterocyclyl, C(O)NH-Alkyl-Aryl, C(O)NH-Alkyl-Heteroaryl, C(O)N(Alkyl)₂, C(O)N(Cycloalkyl)₂, C(O)N(Aryl)₂, C(O)N(Heteroaryl)₂, Alkyl, oder Aryl ein- oder mehrfach, gleich oder verschieden substituiert sein kann,
(iv) unsubstituiertes oder substituiertes Heterocyclyl, wobei der Heterocyclylrest mit OH, O-Alkyl, O-Aryl, NH₂, NH-Alkyl, NH-Aryl, Alkyl, Alkyl-Aryl oder Aryl ein- oder mehrfach, gleich oder verschieden substituiert sein kann,
(v) unsubstituiertes oder substituiertes Aryl, wobei der Arylrest mit F, Cl, Br, I, CF₃, CN, NH₂, NH-Alkyl, NH-Cycloalkyl, NH-Heterocyclyl, NH-Aryl, NH-Heteroaryl, NH-Alkyl-Cycloalkyl, NH-Alkyl-Heterocyclyl, NH-Alkyl-Aryl, NH-Alkyl-Heteroaryl, NH-Alkyl-NH₂, NH-Alkyl-OH, N(Alkyl)₂, NHC(O)-Alkyl, NHC(O)-Cycloalkyl, NHC(O)-Heterocyclyl, NHC(O)-Aryl, NHC(O)-Heteroaryl, NHC(O)-Alkyl-Aryl, NHC(O)-Alkyl-Heteroaryl, NHSO₂-Alkyl, NHSO₂-Cycloalkyl, NHSO₂-Heterocyclyl, NHSO₂-Aryl, NHSO₂-Heteroaryl, NHSO₂-Alkyl-Aryl, NHSO₂-Alkyl-Heteroaryl, NO₂, SH, S-Alkyl, S-Cycloalkyl, S-Heterocyclyl, S-Aryl, S-Heteroaryl, OH, OCF₃, O-Alkyl, O-Cycloalkyl, 0-Heterocyclyl, O-Aryl, O-Heteroaryl, O-Alkyl-Cycloalkyl, O-Alkyl-Heterocyclyl, O-Alkyl-Aryl, O-Alkyl-Heteroaryl, O-Alkyl-OH, O-(CH₂)ₙ-O, OC(O)-Alkyl, OC(O)-Cycloalkyl, OC(O)-Heterocyclyl, OC(O)-Aryl, OC(O)-Heteroaryl, OC(O)-Alkyl-Aryl, OC(O)-Alkyl-Heteroaryl, OSO₃H, OSO₂-Alkyl, OSO₂-Cycloalkyl, OSO₂-Heterocyclyl, OSO₂-Aryl, OSO₂-Heteroaryl, OSO₂-Alkyl-Aryl, OSO₂-Alkyl-Heteroaryl, OP(O)(OH)₂, C(O)-Alkyl, C(O)-Aryl, C(O)-Heteroaryl, CO₂H, CO₂-Alkyl, CO₂-Cycloalkyl, CO₂-Heterocyclyl, CO₂-Aryl, CO₂-Heteroaryl, CO₂-Alkyl-Cycloalkyl, CO₂-Alkyl-Heterocyclyl, CO₂-Alkyl-Aryl, CO₂-Alkyl-Heteroaryl, C(O)-NH₂, C(O)NH-Alkyl, C(O)NH-Cycloalkyl, C(O)NH-Heterocyclyl, C(O)NH-Aryl, C(O)NH-Heteroaryl, C(O)NH-Alkyl-Cycloalkyl, C(O)NH-Alkyl-Heterocyclyl, C(O)NH-Alkyl-Aryl, C(O)NH-Alkyl-Heteroaryl, C(O)N(Alkyl)₂, C(O)N(Cycloalkyl)₂, C(O)N(Aryl)₂, C(O)N(Heteroaryl)₂, SO-Alkyl, SO-Aryl, SO₂-Alkyl, SO₂-Aryl, SO₂NH₂, SO₂NH-Alkyl, SO₂NH-Aryl, SO₂NH-Heteroaryl, SO₂NH-Alkyl-Aryl, S03H, SO₂O-Alkyl, SO₂O-Aryl, SO₂O-Alkyl-Aryl, Alkyl, Cycloalkyl, Heterocyclyl, Aryl oder Heteroaryl, ein- oder mehrfach, gleich oder verschieden substituiert sein kann, und n den Wert 1, 2 oder 3 annehmen kann,
(vi) unsubstituiertes oder substituiertes Heteroaryl, wobei der Heteroarylrest F, Cl, Br, I, CF₃, CN, NH₂, NH-Alkyl, NH-Cycloalkyl, NH-Heterocyclyl, NH-Aryl, NH-Heteroaryl, NH-Alkyl-Cycloalkyl, NH-Alkyl-Heterocyclyl, NH-Alkyl-Aryl, NH-Alkyl-Heteroaryl, NH-Alkyl-NH₂, NH-Alkyl-OH, N(Alkyl)₂, NHC(O)-Alkyl, NHC(O)-Cycloalkyl, NHC(O)-Heterocyclyl, NHC(O)-Aryl, NHC(O)-Heteroaryl, NHC(O)-Alkyl-Aryl, NHC(O)-Alkyl-Heteroaryl, NHSO₂-Alkyl, NHSO₂-Cycloalkyl, NHSO₂-Heterocyclyl, NHSO₂-Aryl, NHSO₂-Heteroaryl, NHSO₂-Alkyl-Aryl, NHSO₂-Alkyl-Heteroaryl, NO₂, SH, S-Alkyl, S-Aryl, S-Heteroaryl, OH, OCF₃, O-Alkyl, O-Cycloalkyl, O-Aryl, O-Heteroaryl, O-Alkyl-Cycloalkyl, 0-Alkyl-Heterocyclyl, O-Alkyl-Aryl, O-Alkyl-Heteroaryl, OC(O)-Alkyl, OC(O)-Cycloalkyl, OC(O)-Heterocyclyl, OC(O)-Aryl, OC(O)-Heteroaryl, OC(O)-Alkyl-Aryl, OC(O)-Alkyl-Heteroaryl, OSO₃H, OSO₂-Alkyl, OSO₂-Cycloalkyl, OSO₂-Heterocyclyl, OSO₂-Aryl, OSO₂-Heteroaryl, OSO₂-Alkyl-Aryl, OSO₂-Alkyl-Heteroaryl, OP(O)(OH)₂, C(O)-Alkyl, C(O)-Aryl, C(O)-Heteroaryl, CO₂H, CO₂-Alkyl, CO₂-Cycloalkyl, CO₂-Heterocyclyl, CO₂-Aryl, CO₂-Heteroaryl, CO₂-Alkyl-Cycloalkyl, CO₂-Alkyl-Heterocyclyl, CO₂-Alkyl-Aryl, CO₂-Alkyl-Heteroaryl, C(O)-NH₂, C(O)NH-Alkyl, C(O)NH-Cycloalkyl, C(O)NH-Heterocyclyl, C(O)NH-Aryl, C(O)NH-Heteroaryl, C(O)NH-Alkyl-Cycloalkyl, C(O)NH-Alkyl-Heterocyclyl, C(O)NH-Alkyl-Aryl, C(O)NH-Alkyl-Heteroaryl, C(O)N(Alkyl)₂, C(O)N(Cycloalkyl)₂, C(O)N(Aryl)₂, C(O)N(Heteroaryl)₂, SO₂NH₂, SO₂NH-Alkyl, SO₂NH-Aryl, SO₂NH-Heteroaryl, SO₂NH-Alkyl-Aryl, SO₃H, SO₂O-Alkyl, SO₂O-Aryl, SO₂O-Alkyl-Aryl, Alkyl, Cycloalkyl, Heterocyclyl, Aryl oder Heteroaryl, ein- oder mehrfach, gleich oder verschieden substituiert sein kann,
(vii) -C(O)-R17, wobei R17 Alkyl, Aryl oder Heteroaryl sein kann, und die Alkyl und Arylsubstituenten ihrerseits wiederum substituiert sein können,
(viii) oder R11 und R12 zusammen Cycloalkyl oder Heterocyclyl bedeuten können,
-C(Y)NR13R14 bedeuten kann, wobei Y = NH und R13 und R14 unabhängig voneinander
(i) Wasserstoff,
(ii) unsubstituiertes oder substituiertes Alkyl, wobei der Alkylrest mit F, Cl, Br, I, CF₃, CN, NH₂, NH-Alkyl, NH-Cycloalkyl, NH-Heterocyclyl, NH-Aryl, NH-Heteroaryl, NH-Alkyl-Aryl, NH-Alkyl-Heteroaryl, N(Alkyl)₂, NHC(O)-Alkyl, NHC(O)-Cycloalkyl, NHC(O)-Heterocyclyl, NHC(O)-Aryl, NHC(O)-Heteroaryl, NHSO₂-Alkyl, NHSO₂-Cycloalkyl, NHSO₂-Aryl, NHSO₂-Heteroaryl, NO₂, SH, S-Alkyl, S-Cycloalkyl, S-Heterocyclyl, S-Aryl, S-Heteroaryl, OH, OCF₃, O-Alkyl, O-Cycloalkyl, O-Heterocyclyl, O-Aryl, O-Heteroaryl, O-Alkyl-Cycloalkyl, 0-Alkyl-Aryl, O-Alkyl-Heteroaryl, OC(O)-Alkyl, OC(O)-Cycloalkyl, OC(O)-Heterocyclyl, OC(O)-Aryl, OC(O)-Heteroaryl, OSO₂-Alkyl, OSO₂-Cycloalkyl, OSO₂-Aryl, OSO₂-Heteroaryl, C(O)-Alkyl, C(O)-Aryl, CO₂H, CO₂-Alkyl, CO₂-Cycloalkyl, CO₂-Heterocyclyl, CO₂-Aryl, CO₂-Heteroaryl, CO₂-Alkyl-Cycloalkyl, CO₂-Alkyl-Heterocyclyl, CO₂-Alkyl-Aryl, CO₂-Alkyl-Heteroaryl, C(O)-NH₂, C(O)NH-Alkyl, C(O)NH-Cycloalkyl, C(O)NH-Heterocyclyl, C(O)NH-Aryl, C(O)NH-Heteroaryl, C(O)NH-Alkyl-Cycloalkyl, C(O)NH-Alkyl-Heterocyclyl, C(O)NH-Alkyl-Aryl, C(O)NH-Alkyl-Heteroaryl, C(O)N(Alkyl)₂, C(O)N(Cycloalkyl)₂, C(O)N(Aryl)₂, C(O)N(Heteroaryl)₂, SO-Alkyl, SO-Aryl, SO₂-Alkyl, SO₂-Aryl, SO₂NH₂, SO₃H, Alkyl, Cycloalkyl, Heterocyclyl, Aryl oder Heteroaryl, ein- oder mehrfach, gleich oder verschieden substituiert sein kann,
(iii) unsubstituiertes oder substituiertes Cycloalkyl, wobei der Cycloalkylrest mit F, Cl, Br, I, NH₂, NH-Alkyl, NH-Cycloalkyl, NH-Heterocyclyl, NH-Aryl, NH-Heteroaryl, NH-Alkyl-Aryl, NH-Alkyl-Heteroaryl, N(Alkyl)₂, NHC(O)-Alkyl, NHC(O)-Cycloalkyl, NHC(O)-Heterocyclyl, NHC(O)-Aryl, NHC(O)-Heteroaryl, NHSO₂-Alkyl, NHSO₂-Cycloalkyl, NHSO₂-Aryl, NHSO₂-Heteroaryl, OH, O-Alkyl, O-Cycloalkyl, O-Heterocyclyl, O-Aryl, O-Heteroaryl, O-Alkyl-Aryl, O-Alkyl-Heteroaryl, OC(O)-Alkyl, OC(O)-Cycloalkyl, OC(O)-Heterocyclyl, OC(O)-Aryl, OC(O)-Heteroaryl, OSO₂-Alkyl, OSO₂-Cycloalkyl, OSO₂-Aryl, OSO₂-Heteroaryl, CO₂H, CO₂-Alkyl, CO₂-Cycloalkyl, CO₂-Heterocyclyl, CO₂-Aryl, CO₂-Heteroaryl, C(O)-NH₂, C(O)NH-Alkyl, C(O)NH-Cycloalkyl, C(O)NH-Heterocyclyl, C(O)NH-Aryl, C(O)NH-Heteroaryl, C(O)NH-Alkyl-Aryl, C(O)NH-Alkyl-Heteroaryl, C(O)N(Alkyl)₂, Alkyl, oder Aryl, ein- oder mehrfach, gleich oder verschieden substituiert sein kann,
(iv) unsubstituiertes oder substituiertes Heterocyclyl, wobei der Heterocyclylrest mit OH, O-Alkyl, O-Aryl, NH₂, NH-Alkyl, NH-Aryl, Alkyl, oder Aryl ein- oder mehrfach, gleich oder verschieden substituiert sein kann,
(v) unsubstituiertes oder substituiertes Aryl, wobei der Arylrest mit F, Cl, Br, I, CF₃, CN, NH₂, NH-Alkyl, NH-Cycloalkyl, NH-Heterocyclyl, NH-Aryl, NH-Heteroaryl, NH-Alkyl-Cycloalkyl, NH-Alkyl-Heterocyclyl, NH-Alkyl-Aryl, NH-Alkyl-Heteroaryl, NH-Alkyl-NH₂, NH-Alkyl-OH, N(Alkyl)₂, NHC(O)-Alkyl, NHC(O)-Cycloalkyl, NHC(O)-Heterocyclyl, NHC(O)-Aryl, NHC(O)-Heteroaryl, NHSO₂-Alkyl, NHSO₂-Aryl, NHSO₂-Heteroaryl, NO₂, SH, S-Alkyl, S-Cycloalkyl, S-Heterocyclyl, S-Aryl, S-Heteroaryl, OH, OCF₃, O-Alkyl, O-Cycloalkyl, 0-Heterocyclyl, O-Aryl, O-Heteroaryl, O-Alkyl-Cycloalkyl, O-Alkyl-Heterocyclyl, O-Alkyl-Aryl, O-Alkyl-Heteroaryl, O-Alkyl-OH, C)-(CH₂)ₙ-O, OC(O)-Alkyl, OC(O)-Cycloalkyl, OC(O)-Heterocyclyl, OC(O)-Aryl, OC(O)-Heteroaryl, OSO₂-Alkyl, OSO₂-Cycloalkyl, OSO₂-Aryl, OSO₂-Heteroaryl, C(O)-Alkyl, C(O)-Aryl, C(O)-Heteroaryl, CO₂H, CO₂-Alkyl, CO₂-Cycloalkyl, CO₂-Heterocyclyl, CO₂-Aryl, CO₂-Heteroaryl, CO₂-Alkyl-Cycloalkyl, CO₂-Alkyl-Heterocyclyl, CO₂-Alkyl-Aryl, CO₂-Alkyl-Heteroaryl, C(O)-NH₂, C(O)NH-Alkyl, C(O)NH-Cycloalkyl, C(O)NH-Heterocyclyl, C(O)NH-Aryl, C(O)NH-Heteroaryl, C(O)NH-Alkyl-Cycloalkyl, C(O)NH-Alkyl-Heterocyclyl, C(O)NH-Alkyl-Aryl, C(O)NH-Alkyl-Heteroaryl, C(O)N(Alkyl)₂, C(O)N(Cycloalkyl)₂, C(O)N(Aryl)₂, C(O)N(Heteroaryl)₂, SO-Alkyl, SO-Aryl, SO₂-Alkyl, SO₂-Aryl, SO₂NH₂, SO₂NH-Alkyl, SO₂NH-Aryl, SO₂NH-Heteroaryl, S03H, SO₂O-Alkyl, SO₂O-Aryl, SO₂O-Heteroaryl, Alkyl, Cycloalkyl, Heterocyclyl, Aryl oder Heteroaryl, ein- oder mehrfach, gleich oder verschieden substituiert sein kann, und n den Wert 1, 2 oder 3 annehmen kann,
(vi) unsubstituiertes oder substituiertes Heteroaryl, wobei der Heteroarylrest mit F, Cl, Br, I, CF₃, CN, NH₂, NH-Alkyl, NH-Cycloalkyl, NH-Heterocyclyl, NH-Aryl, NH-Heteroaryl, NH-Alkyl-Aryl, NH-Alkyl-Heteroaryl, N(Alkyl)₂, NHC(O)-Alkyl, NHC(O)-Cycloalkyl, NHC(O)-Heterocyclyl, NHC(O)-Aryl, NHC(O)-Heteroaryl, NHSO₂-Alkyl, NHSO₂-Aryl, NHSO₂-Heteroaryl, NO₂, SH, S-Alkyl, S-Aryl, OH, OCF₃, O-Alkyl, O-Cycloalkyl, O-Heterocyclyl, O-Aryl, O-Heteroaryl, OC(O)-Alkyl, OC(O)-Cycloalkyl, OC(O)-Heterocyclyl, OC(O)-Aryl, OC(O)-Heteroaryl, OSO₂-Alkyl, OSO₂-Cycloalkyl, OSO₂-Aryl, OSO₂-Heteroaryl, C(O)-Alkyl, C(O)-Aryl, C(O)-Heteroaryl, CO₂H, CO₂-Alkyl, CO₂-Cycloalkyl, CO₂-Heterocyclyl, CO₂-Aryl, CO₂-Heteroaryl, CO₂-Alkyl-Cycloalkyl, CO₂-Alkyl-Heterocyclyl, CO₂-Alkyl-aryl, CO₂-Alkyl-Heteroaryl, C(O)-NH₂, C(O)NH-Alkyl, C(O)NH-Cycloalkyl, C(O)NH-Heterocyclyl, C(O)NH-Aryl, C(O)NH-Heteroaryl, C(O)NH-Alkyl-Cycloalkyl, C(O)NH-Alkyl-Heterocyclyl, C(O)NH-Alkyl-Aryl, C(O)NH-Alkyl-Heteroaryl, C(O)N(Alkyl)₂, C(O)N(Cycloalkyl)₂, C(O)N(Aryl)₂, C(O)N(Heteroaryl)₂, SO₂-Alkyl, SO₂-Aryl, SO₂NH₂, SO₂NH-Alkyl, SO₂NH-Aryl, SO₂NH-Heteroaryl, S03H, SO₂O-Alkyl, SO₂O-Aryl, SO₂O-Heteroaryl, Alkyl, Cycloalkyl, Heterocyclyl, Aryl oder Heteroaryl, ein- oder mehrfach, gleich oder verschieden substituiert sein kann,
(vii) oder R13 und R14 zusammen Cycloalkyl oder Heterocyclyl bedeuten können,
-C(NR15)R16 bedeuten kann, wobei R15 = H und R16
(i) unsubstituiertes oder substituiertes Alkyl, wobei der Alkylrest mit F, Cl, Br, I, CF₃, NH₂, NH-Alkyl, NH-Cycloalkyl, NH-Heterocyclyl, NH-Aryl, NH-Heteroaryl, NH-Alkyl-Aryl, NH-Alkyl-Heteroaryl, N(Alkyl)₂, NHC(O)-Alkyl, NHC(O)-Cycloalkyl, NHC(O)-Heterocyclyl, NHC(O)-Aryl, NHC(O)-Heteroaryl, NHSO₂-Alkyl, NHSO₂-Cycloalkyl, NHSO₂-Aryl, NHSO₂-Heteroaryl, NO₂, SH, S-Alkyl, S-Cycloalkyl, S-Heterocyclyl, S-Aryl, S-Heteroaryl, OH, OCF₃, O-Alkyl, O-Cycloalkyl, O-Heterocyclyl, O-Aryl, O-Heteroaryl, O-Alkyl-Cycloalkyl, O-Alkyl-Aryl, O-Alkyl-Heteroaryl, OC(O)-Alkyl, OC(O)-Cycloalkyl, OC(O)-Heterocyclyl, OC(O)-Aryl, OC(O)-Heteroaryl, OSO₂-Alkyl, OSO₂-Cycloalkyl, OSO₂-Aryl, OSO₂-Heteroaryl, C(O)-Alkyl, C(O)-Aryl, CO₂H, CO₂-Alkyl, CO₂-Cycloalkyl, CO₂-Heterocyclyl, CO₂-Aryl, CO₂-Heteroaryl, CO₂-Alkyl-Cycloalkyl, CO₂-Alkyl-Heterocyclyl, CO₂-Alkyl-Aryl, CO₂-Alkyl-Heteroaryl, C(O)-NH₂, C(O)NH-Alkyl, C(O)NH-Cycloalkyl, C(O)NH-Heterocyclyl, C(O)NH-Aryl, C(O)NH-Heteroaryl, C(O)NH-Alkyl-Cycloalkyl, C(O)NH-Alkyl-Heterocyclyl, C(O)NH-Alkyl-Aryl, C(O)NH-Alkyl-Heteroaryl, C(O)N(Alkyl)₂, C(O)N(Cycloalkyl)₂, C(O)N(Aryl)₂, C(O)N(Heteroaryl)₂, SO-Alkyl, SO-Aryl, SO₂-Alkyl, SO₂-Aryl, SO₂NH₂, SO₃H, Alkyl, Cycloalkyl, Heterocyclyl, Aryl oder Heteroaryl, ein- oder mehrfach, gleich oder verschieden substituiert sein kann,
(ii) unsubstituiertes oder substituiertes Cycloalkyl, wobei der Cycloalkylrest mit F, Cl, Br, I, NH₂, NH-Alkyl, NH-Cycloalkyl, NH-Heterocyclyl, NH-Aryl, NH-Heteroaryl, NH-Alkyl-Aryl, NH-Alkyl-Heteroaryl, N(Alkyl)₂, NHC(O)-Alkyl, NHC(O)-Cycloalkyl, NHC(O)-Heterocyclyl, NHC(O)-Aryl, NHC(O)-Heteroaryl, NHSO₂-Alkyl, NHSO₂-Cycloalkyl, NHSO₂-Aryl, NHSO₂-Heteroaryl, OH, O-Alkyl, O-Cycloalkyl, O-Heterocyclyl, O-Aryl, O-Heteroaryl, O-Alkyl-Aryl, O-Alkyl-Heteroaryl, OC(O)-Alkyl, OC(O)-Cycloalkyl, OC(O)-Heterocyclyl, OC(O)-Aryl, OC(O)-Heteroaryl, OSO₂-Alkyl, OSO₂-Cycloalkyl, OSO₂-Aryl, OSO₂-Heteroaryl, CO₂H, CO₂-Alkyl, CO₂-Cycloalkyl, CO₂-Heterocyclyl, CO₂-Aryl, CO₂-Heteroaryl, C(O)-NH₂, C(O)NH-Alkyl, C(O)NH-Cycloalkyl, C(O)NH-Heterocyclyl, C(O)NH-Aryl, C(O)NH-Heteroaryl, C(O)NH-Alkyl-Aryl, C(O)NH-Alkyl-Heteroaryl, C(O)N(Alkyl)₂, Alkyl, oder Aryl, ein- oder mehrfach, gleich oder verschieden substituiert sein kann,
(iii) unsubstituiertes oder substituiertes Heterocyclyl, wobei der Heterocyclylrest mit OH, O-Alkyl, O-Aryl, NH₂, NH-Alkyl, NH-Aryl, Alkyl oder Aryl ein- oder mehrfach, gleich oder verschieden substituiert sein kann,
(iv) unsubstituiertes oder substituiertes Aryl, wobei der Arylrest mit F, Cl, Br, I, CF₃, NH₂, NH-Alkyl, NH-Cycloalkyl, NH-Heterocyclyl, NH-Aryl, NH-Heteroaryl, NH-Alkyl-Cycloalkyl, NH-Alkyl-Heterocyclyl, NH-Alkyl-Aryl, NH-Alkyl-Heteroaryl, NH-Alkyl-NH₂, NH-Alkyl-OH, N(Alkyl)₂, NHC(O)-Alkyl, NHC(O)-Cycloalkyl, NHC(O)-Heterocyclyl, NHC(O)-Aryl, NHC(O)-Heteroaryl, NHSO₂-Alkyl, NHSO₂-Aryl, NHSO₂-Heteroaryl, NO₂, SH, S-Alkyl, S-Cycloalkyl, S-Heterocyclyl, S-Aryl, S-Heteroaryl, OH, OCF₃, O-Alkyl, O-Cycloalkyl, O-Heterocyclyl, O-Aryl, O-Heteroaryl, O-Alkyl-Cycloalkyl, O-Alkyl-Heterocyclyl, O-Alkyl-Aryl, O-Alkyl-Heteroaryl, O-Alkyl-OH, O-(CH₂)ₙ-O, OC(O)-Alkyl, OC(O)-Cycloalkyl, OC(O)-Heterocyclyl, OC(O)-Aryl, OC(O)-Heteroaryl, OSO₂-Alkyl, OSO₂-Cycloalkyl, OSO₂-Aryl, OSO₂-Heteroaryl, C(O)-Alkyl, C(O)-Aryl, C(O)-Heteroaryl, CO₂H, CO₂-Alkyl, CO₂-Cycloalkyl, CO₂-Heterocyclyl, CO₂-Aryl, CO₂-Heteroaryl, CO₂-Alkyl-Cycloalkyl, CO₂-Alkyl-Heterocyclyl, CO₂-Alkyl-aryl, CO₂-Alkyl-Heteroaryl, C(O)-NH₂, C(O)NH-Alkyl, C(O)NH-Cycloalkyl, C(O)NH-Heterocyclyl, C(O)NH-Aryl, C(O)NH-Heteroaryl, C(O)NH-Alkyl-Cycloalkyl, C(O)NH-Alkyl-Heterocyclyl, C(O)NH-Alkyl-Aryl, C(O)NH-Alkyl-Heteroaryl, C(O)N(Alkyl)₂, C(O)N(Cycloalkyl)₂, C(O)N(Aryl)₂, C(O)N(Heteroaryl)₂, SO-Alkyl, SO-Aryl, SO₂-Alkyl, SO₂-Aryl, SO₂NH₂, SO₂NH-Alkyl, SO₂NH-Aryl, SO₂NH-Heteroaryl, S03H, SO₂O-Alkyl, SO₂O-Aryl, SO₂O-Heteroaryl, Alkyl, Cycloalkyl, Heterocyclyl, Aryl oder Heteroaryl, ein- oder mehrfach, gleich oder verschieden substituiert sein kann, und n den Wert 1, 2 oder 3 annehmen kann,
(v) unsubstituiertes oder substituiertes Heteroaryl, wobei der Heteroarylrest mit F, Cl, Br, I, CF₃, NH₂, NH-Alkyl, NH-Cycloalkyl, NH-Heterocyclyl, NH-Aryl, NH-Heteroaryl, NH-Alkyl-Aryl, NH-Alkyl-Heteroaryl, N(Alkyl)₂, NHC(O)-Alkyl, NHC(O)-Cycloalkyl, NHC(O)-Heterocyclyl, NHC(O)-Aryl, NHC(O)-Heteroaryl, NHSO₂-Alkyl, NHSO₂-Aryl, NHSO₂-Heteroaryl, NO₂, SH, S-Alkyl, S-Aryl, OH, OCF₃, O-Alkyl, O-Cycloalkyl, O-Heterocyclyl, O-Aryl, O-Heteroaryl, OC(O)-Alkyl, OC(O)-Cycloalkyl, OC(O)-Heterocyclyl, OC(O)-Aryl, OC(O)-Heteroaryl, OSO₂-Alkyl, OSO₂-Cycloalkyl, OSO₂-Aryl, OSO₂-Heteroaryl, C(O)-Alkyl, C(O)-Aryl, C(O)-Heteroaryl, CO₂H, CO₂-Alkyl, CO₂-Cycloalkyl, CO₂-Heterocyclyl, CO₂-Aryl, CO₂-Heteroaryl, CO₂-Alkyl-Cycloalkyl, CO₂-Alkyl-Heterocyclyl, CO₂-Alkyl-aryl, CO₂-Alkyl-Heteroaryl, C(O)-NH₂, C(O)NH-Alkyl, C(O)NH-Cycloalkyl, C(O)NH-Heterocyclyl, C(O)NH-Aryl, C(O)NH-Heteroaryl, C(O)NH-Alkyl-Cycloalkyl, C(O)NH-Alkyl-Heterocyclyl, C(O)NH-Alkyl-Aryl, C(O)NH-Alkyl-Heteroaryl, C(O)N(Alkyl)₂, C(O)N(Cycloalkyl)₂, C(O)N(Aryl)₂, C(O)N(Heteroaryl)₂, SO₂-Alkyl, SO₂-Aryl, SO₂NH₂, SO₂NH-Alkyl, SO₂NH-Aryl, SO₂NH-Heteroaryl, SO3H, SO₂O-Alkyl, SO₂O-Aryl, SO₂O-Heteroaryl, Alkyl, Cycloalkyl, Heterocyclyl, Aryl oder Heteroaryl, ein- oder mehrfach, gleich oder verschieden substituiert sein kann, bedeuten können;
sowie physiologisch verträgliche Salze, Derivate oder Analoga der Verbindungen nach Formel I, wobei die Salze erhältlich sind durch Neutralisation der basischen Verbindungen mit anorganischen und organischen Säuren bzw. Neutralisation der sauren Verbindungen mit anorganischen und organischen Basen, sowie deren Solvate, Hydrate und polymorphe Formen,
wobei die Verbindungen der allgemeinen Formel I sowie deren Salze, Derivate oder Analoga, deren Solvate, Hydrate und polymorphe Formen in Form ihrer Racemate, in Form der reinen Enantiomeren und/oder Diastereomeren oder in Form von Mischungen dieser Enantiomeren und/oder Diasteromeren oder in Form der Tautomeren vorliegen können.

2. Pyrido[2,3-b]pyrazin-Derivate der allgemeinen Formel I nach Anspruch 1, **dadurch gekennzeichnet, dass** der Alkylrest Methyl, Ethyl, *n*-Propyl, 2-Propyl, *n*-Butyl, sec.-Butyl, *tert*.*-*Butyl, *n*-Pentyl, *iso*-Pentyl, *neo*-Pentyl, *n*-Hexyl, 2-Hexyl, *n*-Octyl, Ethylenyl (Vinyl), Ethinyl, Propenyl (-CH₂CH=CH₂; -CH=CH-CH₃, -C(=CH₂)-CH₃), Propinyl (-CH₂-C≡CH, -C≡C-CH₃), Butenyl, Butinyl, Pentenyl, Pentinyl, Hexenyl, Hexinyl, Heptenyl, Heptinyl, Octenyl und Octinyl sein kann.

3. Pyrido[2,3-b]pyrazin-Derivate der allgemeinen Formel I nach Anspruch 1, **dadurch gekennzeichnet, dass** der Heterocyclyl-Rest Tetrahydrofuryl, Tetrahydropyranyl, Pyrrolidinyl, Piperidinyl, Piperazinyl und Morpholinyl sein kann.

4. Pyrido[2,3-b]pyrazin-Derivate der allgemeinen Formel I nach Anspruch 1, **dadurch gekennzeichnet, dass** der Heteroaryl-Rest Pyrrolyl, Furyl, Thienyl, Thiazolyl, Oxazolyl, Isoxazolyl, Pyrazolyl, Imidazolyl, Pyridinyl, Pyrimidinyl, Pyridazinyl, Pyrazinyl, Phthalazinyl, Indolyl, Indazolyl, Indolizinyl, Chinolinyl, Isochinolinyl, Chinoxalinyl, Chinazolinyl, Carbazolyl, Phenazinyl, Phenothiazinyl, Acridinyl sein kann.

5. Pyrido[2,3-b]pyrazin-Derivate der allgemeinen Formel I nach den Ansprüchen 1 bis 4, insbesondere eine der folgenden Verbindungen:
1-Allyl-3-(3-phenyl-pyrido[2,3-b]pyrazin-6-yl)-thioharnstoff
1-Allyl-3-(3-naphthalin-2-yl-pyrido[2,3-b]pyrazin-6-yl)-thioharnstoff
1-Allyl-3-[3-(4-methoxy-phenyl)-pyrido[2,3-b]pyrazin-6-yl]-thioharnstoff
1-Allyl-3-[3-(4-hydroxy-phenyl)-pyrido[2,3-b]pyrazin-6-yl]-thioharnstoff-Hydrochlorid
1-(2-Methyl-allyl)-3-(3-phenyl-pyrido[2,3-b]pyrazin-6-yl)-thioharnstoff
1-(2-Methyl-allyl)-3-(3-naphthalin-2-yl-pyrido[2,3-b]pyrazin-6-yl)-thioharnstoff
1-[3-(4-Methoxy-phenyl)-pyrido[2,3-b]pyrazin-6-yl]-3-(2-methyl-allyl)-thioharnstoff
1-(3-Naphthalin-2-yl-pyrido[2,3-b]pyrazin-6-yl)-3-(4-nitro-phenyl)-thioharnstoff
1-[3-(4-Methoxy-phenyl)-pyrido[2,3-b]pyrazin-6-yl]-3-(4-nitro-phenyl)-thioharnstoff
1-tert-Butyl-3-(3-phenyl-pyrido[2,3-b]pyrazin-6-yl)-thioharnstoff
1-Cyclopropyl-3-(3-phenyl-pyrido[2,3-b]pyrazin-6-yl)-thioharnstoff
1-Methyl-3-(3-phenyl-pyrido[2,3-b]pyrazin-6-yl)-thioharnstoff
1-Benzyl-3-(3-phenyl-pyrido[2,3-b]pyrazin-6-yl)-thioharnstoff
1-(4-Fluoro-phenyl)-3-(3-phenyl-pyrido[2,3-b]pyrazin-6-yl)-thioharnstoff
1-Cyclohexyl-3-(3-phenyl-pyrido[2,3-b]pyrazin-6-yl)-thioharnstoff
1-Isopropyl-3-(3-phenyl-pyrido[2,3-b]pyrazin-6-yl)-thioharnstoff
1-Furan-2-ylmethyl-3-(3-phenyl-pyrido[2,3-b]pyrazin-6-yl)-thioharnstoff
1-Methyl-3-[3-(4-nitro-phenyl)-pyrido[2,3-b]pyrazin-6-yl]-thioharnstoff
1-[3-(4-Hydroxy-phenyl)-pyrido[2,3-]pyrazin-6-yl]-3-methyl-thioharnstoff
1-Allyl-3-[3-(4-nitro-phenyl)-pyrido[2,3-b]pyrazin-6-yl]-thioharnstoff
4-[6-(3-Allyl-thioharnstoff)-pyrido[2,3-b]pyrazin-3-yl]-benzoesäureethyl-ester
1-Allyl-3-[3-(3-hydroxy-phenyl)-pyrido[2,3-b]pyrazin-6-yl]-thioharnstoff
1-Allyl-3-(3-benzo[1,3]dioxol-5-yl-pyrido[2,3-b]pyrazin-6-yl)-thioharnstoff
1-[3-(4-Hydroxy-phenyl)-pyrido[2,3-b]pyrazin-6-yl]-3-prop-2-ynyl-thioharnstoff
1-Allyl-3-[3-(4-hydroxy-phenyl)-pyrido[2,3-b]pyrazin-6-yl]-thioharnstoff
1-[3-(4-Hydroxy-phenyl)-pyrido[2,3-b]pyrazin-6-yl]-3-((propenyl)-thioharnstoff
1-Allyl-3-[2,3-bis-(4-hydroxy-phenyl)-pyrido[2,3-b]pyrazin-6-yl]-thioharnstoff
1-[2,3-Bis-(4-hydroxy-phenyl)-pyrido[2,3-b]pyrazin-6-yl]-3-((propenyl)-thioharnstoff
1-Allyl-3-[2-(4-hydroxy-phenyl)-pyrido[2,3-b]pyrazin-6-yl]-thioharnstoff
1-Allyl-3-[3-(4-nitro-phenyl)-pyrido[2,3-b]pyrazin-7-yl]-thioharnstoff
1-Cyclopropyl-3-(3-phenyl-pyrido[2,3-b]pyrazin-6-yl)-harnstoff
1-Allyl-3-[3-(4-hydroxy-phenyl)-pyrido[2,3-b]pyrazin-6-yl]-harnstoff
1-(3-Phenyl-pyrido[2,3-b]pyrazin-6-yl)-3-p-tolyl-harnstoff
1-(4-Chloro-3-trifluoromethyl-phenyl)-3-(3-phenyl-pyrido[2,3-b]pyrazin-6-yl)-harnstoff
1-(2-Morpholin-4-yl-ethyl)-3-(3-phenyl-pyrido[2,3-b]pyrazin-6-yl)-harnstoff
1-Phenethyl-3-(3-phenyl-pyrido[2,3-]pyrazin-6-yl)-harnstoff
1-(2,3-Di-pyridin-2-yl-pyrido[2,3-b]pyrazin-6-yl)-3-ethyl-harnstoff
1-(2,3-Dimethyl-pyrido[2,3-b]pyrazin-6-yl)-3-ethyl-harnstoff

6. Arzneimittel, enthaltend mindestens eine Verbindung der allgemeinen Formel I gemäß den Ansprüchen 1 bis 5.

7. Arzneimittel gemäß Anspruch 6, wobei die Verbindung in Kombination mit mindestens einem weiteren pharmazeutischen Wirkstoff und/oder pharmazeutischen Trägerstoffen und/oder Verdünnungsmitteln beziehungsweise sonstigen Hilfsmitteln in der Zusammensetzung vorliegt.

8. Verfahren zur Herstellung eines Arzneimittels nach Anspruch 6 und 7, **dadurch gekennzeichnet, daß** ein oder mehrere Pyrido[2,3-b]pyrazin-Derivate der allgemeinen Formel I nach einem der Ansprüche 1 bis 5 mit gebräuchlichen pharmazeutischen Trägerstoffen und/oder Verdünnungsmitteln beziehungsweise sonstigen Hilfsstoffen zu pharmazeutischen Zubereitungen verarbeitet, beziehungsweise in eine therapeutisch anwendbare Form gebracht werden.

9. Verbindung gemäß einem der Ansprüche 1 bis 5 zur Verwendung als pharmazeutisches Mittel.

10. Verwendung der Pyrido[2,3-b]pyrazin-Derivate der allgemeinen Formel I nach einem der Ansprüche 1 bis 5 als therapeutische Wirkstoffe zur Herstellung von Arzneimitteln zur Behandlung von Erkrankungen, die aus fehlgeleiteten zellulären Signaltransduktionsprozessen resultieren.

11. Verwendung gemäß Anspruch 10 zur Behandlung von auf pathologischen Zellproliferationen beruhenden Erkrankungen, wie Restenose, Psoriasis, Arteriosklerose und Leberzirrhose.

12. Verwendung gemäß Anspruch 10 zur Behandlung von malignen bzw. benignen Tumorerkrankungen, insbesondere der Brust, Prostata, Lunge, Haut und Eierstöcke.

13. Verwendung gemäß den Ansprüchen 10 bis 12 zur Behandlung von Erkrankungen, die aus fehlgeleiteten zellulären Signaltransduktionsprozessen resultieren, von auf pathologischen Zellproliferationen beruhenden Erkrankungen und malignen bzw. benignen Tumorerkrankungen im Menschen, in Säugetieren und in Geflügel.

14. Verwendung gemäß Anspruch 10 zur Modulation von fehlgeleiteten zellulären Signaltransduktionsprozessen, insbesondere zur Beeinflussung der Funktion von aktiven und inaktiven Tyrosin- und Serin/Threoninkinasen, wie c-Raf, B-Raf, Mek, MAPKs, PDGFRbeta, Flt-3, IGF1R, PKB/Akt1, c-Kit, c-Abl, FGFR1 und KDR.

## Claims

1. Novel pyrido[2,3-b]pyrazine derivatives of the general Formula I 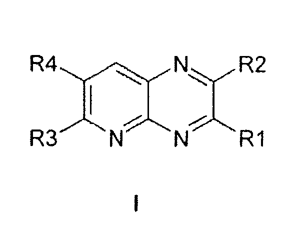 in which the substituents R1-R4 have the following meaning:
R1 and R2 may be independently of one another:
(i) hydrogen
(ii) hydroxyl
(iii) halogen
(iv) alkyl, where the alkyl radical is saturated and may consist of 1 to 8 C atoms,
(v) unsubstituted or substituted aryl, where the aryl radical may have one or more identical or different F, Cl, Br, I, CF₃, CN, NH₂, NH-alkyl, NH-cycloalkyl, NH-heterocyclyl, NH-aryl, NH-heteroaryl, NH-alkyl-cycloalkyl, NH-alkyl-heterocyclyl, NH-alkyl-aryl, NH-alkyl-heteroaryl, NH-alkyl-NH₂, NH-alkyl-OH, N(alkyl)₂, NHC(O)-alkyl, NHC(O)-cycloalkyl, NHC(O)-heterocyclyl, NHC(O)-aryl, NHC(O)-heteroaryl, NHC(O)-alkyl-aryl, NHC(O)-alkyl-heteroaryl, NHSO₂-alkyl, NHSO₂-cycloalkyl, NHSO₂-heterocyclyl, NHSO₂-aryl, NHSO₂-heteroaryl, NHSO₂-alkyl-aryl, NHSO₂-alkyl-heteroaryl, NO₂, SH, S-alkyl, S-aryl, S-heteroaryl, OH, OCF₃, O-alkyl, O-cycloalkyl, O-heterocyclyl, O-aryl, O-heteroaryl, O-alkyl-cycloalkyl, O-alkylheterocyclyl, O-alkyl-aryl, O-alkyl-heteroaryl, O-alkyl-OH, O-(CH₂)ₙ-O, OC(O)-alkyl, OC(O)-cycloalkyl, OC(O)-heterocyclyl, OC(O)-aryl, OC(O)-heteroaryl, OC(O)-alkyl-aryl, OC(O)-alkylheteroaryl, OSO₃H, OSO₂-alkyl, OSO₂-cycloalkyl, OSO₂-heterocyclyl, OSO₂-aryl, OSO₂-heteroaryl, OSO₂-alkyl-aryl, OSO₂-alkyl-heteroaryl, OP(O)(OH)₂, C(O)-alkyl, C(O)-aryl, C(O)-heteroaryl, CO₂H, CO₂-alkyl, CO₂-cycloalkyl, CO₂-heterocyclyl, CO₂-aryl, CO₂-heteroaryl, CO₂-alkyl-cycloalkyl, CO₂-alkylheterocyclyl, CO₂-alkyl-aryl, CO₂-alkyl-heteroaryl, C(O)-NH₂, C(O)NH-alkyl, C(O)NH-cycloalkyl, C(O)NH-heterocyclyl, C(O)NH-aryl, C(O)NH-heteroaryl, C(O)NH-alkyl-cycloalkyl, C(O)NH-alkyl-heterocyclyl, C(O)NH-alkyl-aryl, C(O)NH-alkylheteroaryl, C(O)N(alkyl)₂, C(O)N(cycloalkyl)₂, C(O)N(aryl)₂, C(O)N(heteroaryl)₂, SO-alkyl, SO-aryl, SO₂-alkyl, SO₂-aryl, SO₂NH₂, SO₂NH-alkyl, SO₂NH-aryl, SO₂NH-heteroaryl, SO₂NH-alkyl-aryl, SO₃H, SO₂O-alkyl, SO₂O-aryl, SO₂O-alkyl-aryl, alkyl, cycloalkyl, heterocyclyl, aryl or heteroaryl substituents, n can have the value 1, 2 or 3, and the alkyl, cycloalkyl, heterocyclyl, aryl, heteroaryl, alkyl-cycloalkyl, alkyl-heterocyclyl, alkyl-aryl and alkyl-heteroaryl substituents may in turn themselves be substituted,
(vi) unsubstituted or substituted heteroaryl, where the heteroaryl radical may have one or more identical or different F, Cl, Br, I, CF₃, CN, NH₂, NH-alkyl, NH-cycloalkyl, NH-heterocyclyl, NH-aryl, NH-heteroaryl, NH-alkyl-cycloalkyl, NH-alkylheterocyclyl, NH-alkyl-aryl, NH-alkyl-heteroaryl, NH-alkyl-NH₂, NH-alkyl-OH, N(alkyl)₂, NHC(O)-alkyl, NHC(O)-cycloalkyl, NHC(O)-heterocyclyl, NHC(O)-aryl, NHC(O)-heteroaryl, NHC(O)-alkyl-aryl, NHC(O)-alkyl-heteroaryl, NHSO₂-alkyl, NHSO₂-cycloalkyl, NHSO₂-heterocyclyl, NHSO₂-aryl, NHSO₂-heteroaryl, NHSO₂-alkyl-aryl, NHSO₂-alkylheteroaryl, NO₂, SH, S-alkyl, S-aryl, S-heteroaryl, OH, OCF₃, O-alkyl, O-cycloalkyl, O-aryl, O-heteroaryl, O-alkyl-cycloalkyl, O-alkylheterocyclyl, O-alkyl-aryl, O-alkyl-heteroaryl, OC(O)-alkyl, OC(O)-cycloalkyl, OC(O)-heterocyclyl, OC(O)-aryl, OC(O)-heteroaryl, OC(O)-alkyl-aryl, OC(O)-alkyl-heteroaryl, OSO₃H, OSO₂-alkyl, OSO₂-cycloalkyl, OSO₂-heterocyclyl, OSO₂-aryl, OSO₂-heteroaryl, OSO₂-alkyl-aryl, OSO₂-alkyl-heteroaryl, OP(O)(OH)₂, C(O)-alkyl, C(O)-aryl, C(O)-heteroaryl, CO₂H, CO₂-alkyl, CO₂-cycloalkyl, CO₂-heterocyclyl, CO₂-aryl, CO₂-heteroaryl, CO₂-alkyl-cycloalkyl, CO₂-alkyl-heterocyclyl, CO₂-alkyl-aryl, CO₂-alkylheteroaryl, C(O)-NH₂, C(O)NH-alkyl, C(O)NH-cycloalkyl, C(O)NH-heterocyclyl, C(O)NH-aryl, C(O)NH-heteroaryl, C(O)NH-alkyl-cycloalkyl, C(O)NH-alkyl-heterocyclyl, C(O)NH-alkyl-aryl, C(O)NH-alkyl-heteroaryl, C(O)N(alkyl)₂, C(O)N(cycloalkyl)₂, C(O)N(aryl)₂, C(O)N(heteroaryl)₂, SO₂NH₂, SO₂NH-alkyl, SO₂NH-aryl, SO₂NH-heteroaryl, SO₂NH-alkyl-aryl, SO₃H, SO₂O-alkyl, SO₂O-aryl, SO₂O-alkyl-aryl, alkyl, cycloalkyl, heterocyclyl, aryl or heteroaryl substituents, and the alkyl, cycloalkyl, heterocyclyl, aryl and heteroaryl substituents may in turn themselves be substituted,
(vii) OR5, where R5 can be alkyl, cycloalkyl, heterocyclyl, aryl, heteroaryl, alkylcycloalkyl, alkylheterocyclyl, alkylaryl or alkylheteroaryl, and the alkyl, cycloalkyl, heterocyclyl, aryl, heteroaryl, alkylcycloalkyl, alkylheterocyclyl, alkylaryl or alkylheteroaryl substituents can, for their part, in turn be substituted,
(viii) SR6, where R6 can be alkyl, cycloalkyl, heterocyclyl, aryl, heteroaryl, alkylcycloalkyl, alkylheterocyclyl, alkylaryl or alkylheteroaryl, and the alkyl, cycloalkyl, heterocyclyl, aryl and heteroaryl, alkylcycloalkyl, alkylheterocyclyl, alkylaryl or alkylheteroaryl substituents can, for their part, in turn be substituted,
(ix) NR7R8, where R7 and R8 can, independently of each other, be hydrogen, alkyl, cycloalkyl, heterocyclyl, aryl, heteroaryl, alkylcyclyl, alkylheterocyclyl, alkylaryl or alkylheteroaryl, and the alkyl, cycloalkyl, heterocyclyl, aryl and heteroaryl, alkylcycloalkyl, alkylheterocyclyl, alkylaryl or alkylheteroaryl substituents can, for their part, in turn be substituted, or R7 and R8 are together cycloalkyl or heterocyclyl, where cycloalkyl and heterocyclyl can, for their part, in turn be substituted.
R3 and R4 can, independently of each other, be hydrogen or NR9R10 with the proviso that, when R3 = NR9R10, R4 = H and when R4 = NR9R10, R3 = H,
where R9 can be hydrogen, alkyl, cycloalkyl, heterocyclyl, aryl, heteroaryl, alkylcycloalkyl, alkylheterocyclyl, alkylaryl or alkylheteroaryl, and the alkyl, cycloalkyl, heterocyclyl, aryl and heteroaryl, alkylcycloalkyl, alkylheterocyclyl, alkylaryl or alkylheteroaryl substituents can, for their part, in turn be substituted,
and R10 may be:
-C(Y)NR11R12, where Y is O, S and R11 and R12 may be independently of one another
(i) hydrogen,
(ii) unsubstituted or substituted alkyl, where the alkyl radical may have one or more identical or different F, Cl, Br, I, CF₃, CN, NH₂, NH-alkyl, NH-cycloalkyl, NH-heterocyclyl, NH-aryl, NH-heteroaryl, NH-alkyl-cycloalkyl, NH-alkylheterocyclyl, NH-alkyl-aryl, NH-alkyl-heteroaryl, N(alkyl)₂, NHC(O)-alkyl, NHC(O)-cycloalkyl, NHC(O)-heterocyclyl, NHC(O)-aryl, NHC(O)-heteroaryl, NHC(O)-alkyl-aryl, NHC(O)-alkyl-heteroaryl, NHSO₂-alkyl, NHSO₂-cycloalkyl, NHSO₂-heterocyclyl, NHSO₂-aryl, NHSO₂-heteroaryl, NHSO₂-alkyl-aryl, NHSO₂-alkyl-heteroaryl, NO₂, SH, S-alkyl, S-cycloalkyl, S-heterocyclyl, S-aryl, S-heteroaryl, OH, OCF₃, O-alkyl, O-cycloalkyl, O-heterocyclyl, O-aryl, O-heteroaryl, O-alkyl-cycloalkyl, O-alkylheterocyclyl, O-alkyl-aryl, O-alkyl-heteroaryl, OC(O)-alkyl, OC(O)-cycloalkyl, OC(O)-heterocyclyl, OC(O)-aryl, OC(O)-heteroaryl, OC(O)-alkyl-aryl, OC(O)-alkyl-heteroaryl, OSO₃H, OSO₂-alkyl, OSO₂-cycloalkyl, OSO₂-heterocyclyl, OSO₂-aryl, OSO₂-heteroaryl, OSO₂-alkyl-aryl, OSO₂-alkyl-heteroaryl, OP(O)(OH)₂, C(O)-alkyl, C(O)-aryl, C(O)-heteroaryl, CO₂H, CO₂-alkyl, CO₂-cycloalkyl, CO₂-heterocyclyl, CO₂-aryl, CO₂-heteroaryl, CO₂-alkyl-cycloalkyl, CO₂-alkyl-heterocyclyl, CO₂-alkyl-aryl, CO₂-alkylheteroaryl, C(O)-NH₂, C(O)NH-alkyl, C(O)NH-cycloalkyl, C(O)NH-heterocyclyl, C(O)NH-aryl, C(O)NH-heteroaryl, C(O)NH-alkyl-cycloalkyl, C(O)NH-alkyl-heterocyclyl, C(O)NH-alkyl-aryl, C(O)NH-alkyl-heteroaryl, C(O)N(alkyl)₂, C(O)N(cycloalkyl)₂, C(O)N(aryl)₂, C(O)N(heteroaryl)₂, SO-alkyl, SO-aryl, SO₂-alkyl, SO₂-aryl, SO₂NH₂, SO₂NH-alkyl, SO₂NH-aryl, SO₂NH-heteroaryl, SO₂NH-alkyl-aryl, SO₃H, SO₂O-alkyl, SO₂O-aryl, SO₂O-alkyl-aryl, cycloalkyl, heterocyclyl, aryl or heteroaryl substituents,
(iii) unsubstituted or substituted cycloalkyl, where the cycloalkyl radical may have one or more identical or different F, Cl, Br, I, NH₂, NH-alkyl, NH-cycloalkyl, NH-heterocyclyl, NH-aryl, NH-heteroaryl, NH-alkyl-aryl, NH-alkyl-heteroaryl, N(alkyl)₂, NHC(O)-alkyl, NHC(O)-cycloalkyl, NHC(O)-heterocyclyl, NHC(O)-aryl, NHC(O)-heteroaryl, NHC(O)-alkyl-aryl, NHC(O)-alkyl-heteroaryl, NHSO₂-alkyl, NHSO₂-cycloalkyl, NHSO₂-heterocyclyl, NHSO₂-aryl, NHSO₂-heteroaryl, NHSO₂-alkyl-aryl, NHSO₂-alkyl-heteroaryl, OH, O-alkyl, O-cycloalkyl, O-heterocyclyl, O-aryl, O-heteroaryl, O-alkylaryl, O-alkyl-heteroaryl, OC(O)-alkyl, OC(O)-cycloalkyl, OC(O)-heterocyclyl, OC(O)-aryl, OC(O)-heteroaryl, OC(O)-alkyl-aryl, OC(O)-alkylheteroaryl, OSO₃H, OSO₂-alkyl, OSO₂-cycloalkyl, OSO₂-heterocyclyl, OSO₂-aryl, OSO₂-heteroaryl, OSO₂-alkyl-aryl, OSO₂-alkyl-heteroaryl, OP(O)(OH)₂, CO₂H, CO₂-alkyl, CO₂-cycloalkyl, CO₂-heterocyclyl, CO₂-aryl, CO₂-heteroaryl, CO₂-alkylcycloalkyl, CO₂-alkyl-hetero-cyclyl, CO₂-alkyl-aryl, CO₂-alkylheteroaryl, C(O)-NH₂, C(O)NH-aryl, C(O)NH-cycloalkyl, C(O)NH-heterocyclyl, C(O)NH-aryl, C(O)NH-heteroaryl, C(O)NH-alkyl-cycloalkyl, C(O)NH-alkyl-heterocyclyl, C(O)NH-alkyl-aryl, C(O)NH-alkyl-heteroaryl, C(O)N(alkyl)₂, C(O)N(cycloalkyl)₂, C(O)N(aryl)₂, C(O)N(heteroaryl)₂, alkyl or aryl substituents,
(iv) unsubstituted or substituted heterocyclyl, where the heterocyclyl radical may have one or more identical or different OH, O-alkyl, O-aryl, NH₂, NH-alkyl, NH-aryl, alkyl, alkyl-aryl or aryl substituents,
(v) unsubstituted or substituted aryl, where the aryl radical may have one or more identical or different F, Cl, Br, I, CF₃, CN, NH₂, NH-alkyl, NH-cycloalkyl, NH-heterocyclyl, NH-aryl, NH-heteroaryl, NH-alkyl-cycloalkyl, NH-alkyl-heterocyclyl, NH-alkyl-aryl, NH-alkyl-heteroaryl, NH-alkyl-NH₂, NH-alkyl-OH, N(alkyl)₂, NHC(O)-alkyl, NHC(O)-cycloalkyl, NHC(O)-heterocyclyl, NHC(O)-aryl, NHC(O)-heteroaryl, NHC(O)-alkyl-aryl, NHC(O)-alkyl-heteroaryl, NHSO₂-alkyl, NHSO₂-cycloalkyl, NHSO₂-heterocyclyl, NHSO₂-aryl, NHSO₂-heteroaryl, NHSO₂-alkyl-aryl, NHSO₂-alkylheteroaryl, NO₂, SH, S-alkyl, S-cycloalkyl, S-heterocyclyl, S-aryl, S-heteroaryl, OH, OCF₃, O-alkyl, O-cycloalkyl, O-heterocyclyl, O-aryl, O-heteroaryl, O-alkyl-cycloalkyl, O-alkyl-heterocyclyl, O-alkyl-aryl, O-alkyl-heteroaryl, O-alkyl-OH, O-(CH₂)ₙ-O, OC(O)-alkyl, OC(O)-cycloalkyl, OC(O)-heterocyclyl, OC(O)-aryl, OC(O)-heteroaryl, OC(O)-alkyl-aryl, OC(O)-alkyl-heteroaryl, OSO₃H, OSO₂-alkyl, OSO₂-cycloalkyl, OSO₂-heterocyclyl, OSO₂-aryl, OSO₂-heteroaryl, OSO₂-alkyl-aryl, OSO₂-alkyl-heteroaryl, OP(O)(OH)₂, C(O)-alkyl, C(O)-aryl, C(O)-heteroaryl, CO₂H, CO₂-alkyl, CO₂-cycloalkyl, CO₂-heterocyclyl, CO₂-aryl, CO₂-heteroaryl, CO₂-alkyl-cycloalkyl, CO₂-alkyl-heterocyclyl, CO₂-alkyl-aryl, CO₂-alkyl-heteroaryl, C(O)-NH₂, C(O)NH-alkyl, C(O)NH-cycloalkyl, C(O)NH-heterocyclyl, C(O)NH-aryl, C(O)NH-heteroaryl, C(O)NH-alkyl-cycloalkyl, C(O)NH-alkyl-heterocyclyl, C(O)NH-alkyl-aryl, C(O)NH-alkyl-heteroaryl, C(O)N(alkyl)₂, C(O)N(cycloalkyl)₂, C(O)N(aryl)₂, C(O)N(heteroaryl)₂, SO-alkyl, SO-aryl, SO₂-alkyl, SO₂-aryl, SO₂NH₂, SO₂NH-alkyl, SO₂NH-aryl, SO₂NH-heteroaryl, SO₂NH-alkyl-aryl, SO₃H, SO₂O-alkyl, SO₂O-aryl, SO₂O-alkylaryl, alkyl, cycloalkyl, heterocyclyl, aryl or heteroaryl substituents, and n may have the value 1, 2 or 3,
(vi) unsubstituted or substituted heteroaryl, where the heteroaryl radical may have one or more identical or different F, Cl, Br, I, CF₃, CN, NH₂, NH-alkyl, NH-cycloalkyl, NH-heterocyclyl, NH-aryl, NH-heteroaryl, NH-alkyl-cycloalkyl, NH-alkylheterocyclyl, NH-alkyl-aryl, NH-alkyl-heteroaryl, NH-alkyl-NH₂, NH-alkyl-OH, N(alkyl)₂, NHC(O)-alkyl, NHC(O)-cycloalkyl, NHC(O)-heterocyclyl, NHC(O)-aryl, NHC(O)-heteroaryl, NHC(O)-alkyl-aryl, NHC(O)-alkyl-heteroaryl, NHSO₂-alkyl, NHSO₂-cycloalkyl, NHSO₂-heterocyclyl, NHSO₂-aryl, NHSO₂-heteroaryl, NHSO₂-alkyl-aryl, NHSO₂-alkylheteroaryl, NO₂, SH, S-alkyl, S-aryl, S-heteroaryl, OH, OCF₃, O-alkyl, O-cycloalkyl, O-aryl, O-heteroaryl, O-alkyl-cycloalkyl, O-alkyl-heterocyclyl, O-alkyl-aryl, O-alkyl-heteroaryl, OC(O)-alkyl, OC(O)-cycloalkyl, OC(O)-heterocyclyl, OC(O)-aryl, OC(O)-heteroaryl, OC(O)-alkyl-aryl, OC(O)-alkylheteroaryl, OSO₃H, OSO₂-alkyl, OSO₂-cycloalkyl, OSO₂-heterocyclyl, OSO₂-aryl, OSO₂-heteroaryl, OSO₂-alkyl-aryl, OSO₂-alkyl-heteroaryl, OP(O)(OH)₂, C(O)-alkyl, C(O)-aryl, C(O)-heteroaryl, CO₂H, CO₂-alkyl, CO₂-cycloalkyl, CO₂-heterocyclyl, CO₂-aryl, CO₂-heteroaryl, CO₂-alkyl-cycloalkyl, CO₂-alkylheterocyclyl, CO₂-alkyl-aryl, CO₂-alkyl-heteroaryl, C(O)-NH₂, C(O)NH-alkyl, C(O)NH-cycloalkyl, C(O)NH-heterocyclyl, C(O)NH-aryl, C(O)NH-heteroaryl, C(O)NH-alkyl-cycloalkyl, C(O)NH-alkyl-heterocyclyl, C(O)NH-alkyl-aryl, C(O)NH-alkylheteroaryl, C(O)N(alkyl)₂, C(O)N(cycloalkyl)₂, C(O)N(aryl)₂, C(O)N(heteroaryl)₂, SO₂NH₂, SO₂NH-alkyl, SO₂NH-aryl, SO₂NH-heteroaryl, SO₂NH-alkylaryl, SO₃H, SO₂O-alkyl, SO₂O-aryl, SO₂O-alkyl-aryl, alkyl, cycloalkyl, heterocyclyl, aryl or heteroaryl substituents,
(vii) -C(O)-R17, where R17 may be alkyl, aryl or heteroaryl, and the alkyl and aryl substituents may in turn themselves be substituted,
(viii) or R11 and R12 together may be cycloalkyl or heterocyclyl,
may be -C(Y)NR13R14, where Y is NH and R13 and R14 may be independently of one another
(i) hydrogen,
(ii) unsubstituted or substituted alkyl, where the alkyl radical may have one or more identical or different F, Cl, Br, I, CF₃, CN, NH₂, NH-alkyl, NH-cycloalkyl, NH-heterocyclyl, NH-aryl, NH-heteroaryl, NH-alkyl-aryl, NH-alkyl-heteroaryl, N(alkyl)₂, NHC(O)-alkyl, NHC(O)-cycloalkyl, NHC(O)-heterocyclyl, NHC(O)-aryl, NHC(O)-heteroaryl, NHSO₂-alkyl, NHSO₂-cycloalkyl, NHSO₂-aryl, NHSO₂-heteroaryl, NO₂, SH, S-alkyl, S-cycloalkyl, S-heterocyclyl, S-aryl, S-heteroaryl, OH, OCF₃, O-alkyl, O-cycloalkyl, O-heterocyclyl, O-aryl, O-heteroaryl, O-alkyl-cycloalkyl, O-alkyl-aryl, O-alkyl-heteroaryl, OC(O)-alkyl, OC(O)-cycloalkyl, OC(O)-heterocyclyl, OC(O)-aryl, OC(O)-heteroaryl, OSO₂-alkyl, OSO₂-cycloalkyl, OSO₂-aryl, OSO₂-heteroaryl, C(O)-alkyl, C(O)-aryl, CO₂H, CO₂-alkyl, CO₂-cycloalkyl, CO₂-heterocyclyl, CO₂-aryl, CO₂-heteroaryl, CO₂-alkyl-cycloalkyl, CO₂-alkylheterocyclyl, CO₂-alkyl-aryl, CO₂-alkyl-heteroaryl, C(O)-NH₂, C(O)NH-alkyl, C(O)NH-cycloalkyl, C(O)NH-heterocyclyl, C(O)NH-aryl, C(O)NH-heteroaryl, C(O)NH-alkyl-cycloalkyl, C(O)NH-alkyl-heterocyclyl, C(O)NH-alkyl-aryl, C(O)NH-alkyl-heteroaryl, C(O)N(alkyl)₂, C(O)N(cycloalkyl)₂, C(O)N(aryl)₂, C(O)N(heteroaryl)₂, SO-alkyl, SO-aryl, SO₂-alkyl, SO₂-aryl, SO₂NH₂, SO₃H, alkyl, cycloalkyl, heterocyclyl, aryl or heteroaryl substituents,
(iii) unsubstituted or substituted cycloalkyl, where the cycloalkyl radical may have one or more identical or different F, Cl, Br, I, NH₂, NH-alkyl, NH-cycloalkyl, NH-heterocyclyl, NH-aryl, NH-heteroaryl, NH-alkyl-aryl, NH-alkyl-heteroaryl, N(alkyl)₂, NHC(O)-alkyl, NHC(O)-cycloalkyl, NHC(O)-heterocyclyl, NHC(O)-aryl, NHC(O)-heteroaryl, NHSO₂-alkyl, NHSO₂-cycloalkyl, NHSO₂-aryl, NHSO₂-heteroaryl, OH, O-alkyl, O-cycloalkyl, O-heterocyclyl, O-aryl, O-heteroaryl, O-alkylaryl, O-alkyl-heteroaryl, OC(O)-alkyl, OC(O)-cycloalkyl, OC(O)-heterocyclyl, OC(O)-aryl, OC(O)-heteroaryl, OSO₂-alkyl, OSO₂-cycloalkyl, OSO₂-aryl, OSO₂-heteroaryl, CO₂H, CO₂-alkyl, CO₂-cycloalkyl, CO₂-heterocyclyl, CO₂-aryl, CO₂-heteroaryl, C(O)-NH₂, C(O)NH-alkyl, C(O)NH-cycloalkyl, C(O)NH-heterocyclyl, C(O)NH-aryl, C(O)NH-heteroaryl, C(O)NH-alkyl-aryl, C(O)NH-alkyl-heteroaryl, C(O)N(alkyl)₂, alkyl or aryl substituents,
(iv) unsubstituted or substituted heterocyclyl, where the heterocyclyl radical may have one or more identical or different OH, O-alkyl, O-aryl, NH₂, NH-alkyl, NH-aryl, alkyl or aryl substituents,
(v) unsubstituted or substituted aryl, where the aryl radical may have one or more identical or different F, Cl, Br, I, CF₃, CN, NH₂, NH-alkyl, NH-cycloalkyl, NH-heterocyclyl, NH-aryl, NH-heteroaryl, NH-alkyl-cycloalkyl, NH-alkylheterocyclyl, NH-alkyl-aryl, NH-alkyl-heteroaryl, NH-alkyl-NH₂, NH-alkyl-OH, N(alkyl)₂, NHC(O)-alkyl, NHC(O)-cycloalkyl, NHC(O)-heterocyclyl, NHC(O)-aryl, NHC(O)-heteroaryl, NHSO₂-alkyl, NHSO₂-aryl, NHSO₂-heteroaryl, NO₂, SH, S-alkyl, S-cycloalkyl, S-heterocyclyl, S-aryl, S-heteroaryl, OH, OCF₃, O-alkyl, O-cycloalkyl, O-heterocyclyl, O-aryl, O-heteroaryl, O-alkyl-cycloalkyl, O-alkylheterocyclyl, O-alkyl-aryl, O-alkyl-heteroaryl, O-alkyl-OH, O-(CH₂)ₙ-O, OC(O)-alkyl, OC(O)-cycloalkyl, OC(O)-heterocyclyl, OC(O)-aryl, OC(O)-heteroaryl, OSO₂-alkyl, OSO₂-cycloalkyl, OSO₂-aryl, OSO₂-heteroaryl, C(O)-alkyl, C(O)-aryl, C(O)-heteroaryl, CO₂H, CO₂-alkyl, CO₂-cycloalkyl, CO₂-heterocyclyl, CO₂-aryl, CO₂-heteroaryl, CO₂-alkylcycloalkyl, CO₂-alkyl-heterocyclyl, CO₂-alkyl-aryl, CO₂-alkyl-heteroaryl, C(O)-NH₂, C(O)NH-alkyl, C(O)NH-cycloalkyl, C(O)NH-heterocyclyl, C(O)NH-aryl, C(O)NH-heteroaryl, C(O)NH-alkyl-cycloalkyl, C(O)NH-alkyl-heterocyclyl, C(O)NH-alkyl-aryl, C(O)NH-alkyl-heteroaryl, C(O)N(alkyl)₂, C(O)N(cycloalkyl)₂, C(O)N(aryl)₂, C(O)N(heteroaryl)₂, SO-alkyl, SO-aryl, SO₂-alkyl, SO₂-aryl, SO₂NH₂, SO₂NH-alkyl, SO₂NH-aryl, SO₂NH-heteroaryl, SO₃H, SO₂O-alkyl, SO₂O-aryl, SO₂O-heteroaryl, alkyl, cycloalkyl, heterocyclyl, aryl or heteroaryl substituents, and n may have the value 1, 2 or 3,
(vi) unsubstituted or substituted heteroaryl, where the heteroaryl radical may have one or more identical or different F, Cl, Br, I, CF₃, CN, NH₂, NH-alkyl, NH-cycloalkyl, NH-heterocyclyl, NH-aryl, NH-heteroaryl, NH-alkyl-aryl, NH-alkyl-heteroaryl, N(alkyl)₂, NHC(O)-alkyl, NHC(O)-cycloalkyl, NHC(O)-heterocyclyl, NHC(O)-aryl, NHC(O)-heteroaryl, NHSO₂-alkyl, NHSO₂-aryl, NHSO₂-heteroaryl, NO₂, SH, S-alkyl, S-aryl, OH, OCF₃, O-alkyl, O-cycloalkyl, O-heterocyclyl, O-aryl, O-heteroaryl, OC(O)-alkyl, OC(O)-cycloalkyl, OC(O)-heterocyclyl, OC(O)-aryl, OC(O)-heteroaryl, OSO₂-alkyl, OSO₂-cycloalkyl, OSO₂-aryl, OSO₂-heteroaryl, C(O)-alkyl, C(O)-aryl, C(O)-heteroaryl, CO₂H, CO₂-alkyl, CO₂-cycloalkyl, CO₂-heterocyclyl, CO₂-aryl, CO₂-heteroaryl, CO₂-alkyl-cycloalkyl, CO₂-alkylheterocyclyl, CO₂-alkylaryl, CO₂-alkyl-heteroaryl, C(O)-NH₂, C(O)NH-alkyl, C(O)NH-cycloalkyl, C(O)NH-heterocyclyl, C(O)NH-aryl, C(O)NH-heteroaryl, C(O)NH-alkyl-cycloalkyl, C(O)NH-alkyl-heterocyclyl, C(O)NH-alkyl-aryl, C(O)NH-alkyl-heteroaryl, C(O)N(alkyl)₂, C(O)N(cycloalkyl)₂, C(O)N(aryl)₂, C(O)N(heteroaryl)₂, SO₂-alkyl, SO₂-aryl, SO₂NH₂, SO₂NH-alkyl, SO₂NH-aryl, SO₂NH-heteroaryl, SO₃H, SO₂O-alkyl, SO₂O-aryl, SO₂O-heteroaryl, alkyl, cycloalkyl, heterocyclyl, aryl or heteroaryl substituents,
(vii) or R13 and R14 together may be cycloalkyl or heterocyclyl,
may be -C(NR15)R16 where R15 is H and R16 may be
(i) unsubstituted or substituted alkyl, where the alkyl radical may have one or more identical or different F, Cl, Br, I, CF₃, NH₂, NH-alkyl, NH-cycloalkyl, NH-heterocyclyl, NH-aryl, NH-heteroaryl, NH-alkyl-aryl, NH-alkyl-heteroaryl, N(alkyl)₂, NHC(O)-alkyl, NHC(O)-cycloalkyl, NHC(O)-heterocyclyl, NHC(O)-aryl, NHC(O)-heteroaryl, NHSO₂-alkyl, NHSO₂-cycloalkyl, NHSO₂-aryl, NHSO₂-heteroaryl, NO₂, SH, S-alkyl, S-cycloalkyl, S-heterocyclyl, S-aryl, S-heteroaryl, OH, OCF₃, O-alkyl, O-cycloalkyl, O-heterocyclyl, O-aryl, O-heteroaryl, O-alkyl-cycloalkyl, O-alkyl-aryl, O-alkyl-heteroaryl, OC(O)-alkyl, OC(O)-cycloalkyl, OC(O)-heterocyclyl, OC(O)-aryl, OC(O)-heteroaryl, OSO₂-alkyl, OSO₂-cycloalkyl, OSO₂-aryl, OSO₂-heteroaryl, C(O)-alkyl, C(O)-aryl, CO₂H, CO₂-alkyl, CO₂-cycloalkyl, CO₂-heterocyclyl, CO₂-aryl, CO₂-heteroaryl, CO₂-alkyl-cycloalkyl, CO₂-alkylheterocyclyl, CO₂-alkyl-aryl, CO₂-alkyl-heteroaryl, C(O)-NH₂, C(O)NH-alkyl, C(O)NH-cycloalkyl, C(O)NH-heterocyclyl, C(O)NH-aryl, C(O)NH-heteroaryl, C(O)NH-alkyl-cycloalkyl, C(O)NH-alkyl-heterocyclyl, C(O)NH-alkyl-aryl, C(O)NH-alkyl-heteroaryl, C(O)N(alkyl)₂, C(O)N(cycloalkyl)₂, C(O)N(aryl)₂, C(O)N(heteroaryl)₂, SO-alkyl, SO-aryl, SO₂-alkyl, SO₂-aryl, SO₂NH₂, SO₃H, alkyl, cycloalkyl, heterocyclyl, aryl or heteroaryl substituents,
(ii) unsubstituted or substituted cycloalkyl, where the cycloalkyl radical may have one or more identical or different F, Cl, Br, I, NH₂, NH-alkyl, NH-cycloalkyl, NH-heterocyclyl, NH-aryl, NH-heteroaryl, NH-alkyl-aryl, NH-alkyl-heteroaryl, N(alkyl)₂, NHC(O)-alkyl, NHC(O)-cycloalkyl, NHC(O)-heterocyclyl, NHC(O)-aryl, NHC(O)-heteroaryl, NHSO₂-alkyl, NHSO₂-cycloalkyl, NHSO₂-aryl, NHSO₂-heteroaryl, OH, O-alkyl, O-cycloalkyl, O-heterocyclyl, O-aryl, O-heteroaryl, O-alkylaryl, O-alkyl-heteroaryl, OC(O)-alkyl, OC(O)-cycloalkyl, OC(O)-heterocyclyl, OC(O)-aryl, OC(O)-heteroaryl, OSO₂-alkyl, OSO₂-cycloalkyl, OSO₂-aryl, OSO₂-heteroaryl, CO₂H, CO₂-alkyl, CO₂-cycloalkyl, CO₂-heterocyclyl, CO₂-aryl, CO₂-heteroaryl, C(O)-NH₂, C(O)NH-alkyl, C(O)NH-cycloalkyl, C(O)NH-heterocyclyl, C(O)NH-aryl, C(O)NH-heteroaryl, C(O)NH-alkyl-aryl, C(O)NH-alkyl-heteroaryl, C(O)N(alkyl)₂, alkyl or aryl substituents,
(iii) unsubstituted or substituted heterocyclyl, where the heterocyclyl radical may have one or more identical or different OH, O-alkyl, O-aryl, NH₂, NH-alkyl, NH-aryl, alkyl or aryl substituents,
(iv) unsubstituted or substituted aryl, where the aryl radical may have one or more identical or different F, Cl, Br, I, CF₃, NH₂, NH-alkyl, NH-cycloalkyl, NH-heterocyclyl, NH-aryl, NH-heteroaryl, NH-alkyl-cycloalkyl, NH-alkyl-heterocyclyl, NH-alkyl-aryl, NH-alkyl-heteroaryl, NH-alkyl-NH₂, NH-alkyl-OH, N(alkyl)₂, NHC(O)-alkyl, NHC(O)-cycloalkyl, NHC(O)-heterocyclyl, NHC(O)-aryl, NHC(O)-heteroaryl, NHSO₂-alkyl, NHSO₂-aryl, NHSO₂-heteroaryl, NO₂, SH, S-alkyl, S-cycloalkyl, S-heterocyclyl, S-aryl, S-heteroaryl, OH, OCF₃, O-alkyl, O-cycloalkyl, O-heterocyclyl, O-aryl, O-heteroaryl, O-alkyl-cycloalkyl, O-alkylheterocyclyl, O-alkyl-aryl, O-alkyl-heteroaryl, O-alkyl-OH, O-(CH₂)ₙ-O, OC(O)-alkyl, OC(O)-cycloalkyl, OC(O)-heterocyclyl, OC(O)-aryl, OC(O)-heteroaryl, OSO₂-alkyl OSO₂-cycloalkyl, OSO₂-aryl, OSO₂-heteroaryl, C(O)-alkyl, C(O)-aryl, C(O)-heteroaryl, CO₂H, CO₂-alkyl, CO₂-cycloalkyl, CO₂-heterocyclyl, CO₂-aryl, CO₂-heteroaryl, CO₂-alkylcycloalkyl, CO₂-alkyl-heterocyclyl, CO₂-alkyl-aryl, CO₂-alkyl-heteroaryl, C(O)-NH₂, C(O)NH-alkyl, C(O)NH-cycloalkyl, C(O)NH-heterocyclyl, C(O)NH-aryl, C(O)NH-heteroaryl, C(O)NH-alkyl-cycloalkyl, C(O)NH-alkyl-heterocyclyl, C(O)NH-alkyl-aryl, C(O)NH-alkyl-heteroaryl, C(O)N(alkyl)₂, C(O)N(cycloalkyl)₂, C(O)N(aryl)₂, C(O)N(heteroaryl)₂, SO-alkyl, SO-aryl, SO₂-alkyl, SO₂-aryl, SO₂NH₂, SO₂NH-alkyl, SO₂NH-aryl, SO₂NH-heteroaryl, SO₃H, SO₂O-alkyl, SO₂O-aryl, SO₂O-heteroaryl, alkyl, cycloalkyl, heterocyclyl, aryl or heteroaryl substituents, and n may have the value 1, 2 or 3,
(v) unsubstituted or substituted heteroaryl, where the heteroaryl radical may have one or more identical or different F, Cl, Br, I, CF₃, NH₂, NH-alkyl, NH-cycloalkyl, NH-heterocyclyl, NH-aryl, NH-heteroaryl, NH-alkyl-aryl, NH-alkyl-heteroaryl, N(alkyl)₂, NHC(O)-alkyl, NHC(O)-cycloalkyl, NHC(O)-heterocyclyl, NHC(O)-aryl, NHC(O)-heteroaryl, NHSO₂-alkyl, NHSO₂-aryl, NHSO₂-heteroaryl, NO₂, SH, S-alkyl, S-aryl, OH, OCF₃, O-alkyl, O-cycloalkyl, O-heterocyclyl, O-aryl, O-heteroaryl, OC(O)-alkyl, OC(O)-cycloalkyl, OC(O)-heterocyclyl, OC(O)-aryl, OC(O)-heteroaryl, OSO₂-alkyl, OSO₂-cycloalkyl, OSO₂-aryl, OSO₂-heteroaryl, C(O)-alkyl, C(O)-aryl, C(O)-heteroaryl, CO₂H, CO₂-alkyl, CO₂-cycloalkyl, CO₂-heterocyclyl, CO₂-aryl, CO₂-heteroaryl, CO₂-alkyl-cycloalkyl, CO₂-alkyl-heterocyclyl, CO₂-alkyl-aryl, CO₂-alkyl-heteroaryl, C(O)-NH₂, C(O)NH-alkyl, C(O)NH-cycloalkyl, C(O)NH-heterocyclyl, C(O)NH-aryl, C(O)NH-heteroaryl, C(O)NH-alkylcycloalkyl, C(O)NH-alkyl-heterocyclyl, C(O)NH-alkyl-aryl, C(O)NH-alkyl-heteroaryl, C(O)N(alkyl)₂, C(O)N(cycloalkyl)₂, C(O)N(aryl)₂, C (O) N (heteroaryl)₂, SO₂-alkyl, SO₂-aryl, SO₂NH₂, SO₂NH-alkyl, SO₂NH-aryl, SO₂NH-heteroaryl, SO₃H, SO₂O-alkyl, SO₂O-aryl, SO₂O-heteroaryl, alkyl, cycloalkyl, heterocyclyl, aryl or heteroaryl substituents;
and physiologically tolerable salts, derivatives or analogues of the compounds according to Formula I, where the salts are obtainable by neutralization of the basic compounds with inorganic and organic acids or neutralization of the acidic compounds with inorganic and organic bases, and the solvates, hydrates and polymorphous forms thereof,
where the compounds of the general Formula I and the salts, derivatives or analogues thereof, the solvates, hydrates and polymorphous forms thereof may be present in the form of their racemates, in the form of the pure enantiomers and/or diastereomers or in the form of mixtures of these enantiomers and/or diastereomers or in the form of the tautomers.

2. Pyrido[2,3-b]pyrazine derivatives of the general Formula I according to Claim 1, **characterized in that** the alkyl radical may be methyl, ethyl, *n*-propyl, 2-propyl, *n*-butyl, *sec*-butyl, *tert*-butyl, *n*-pentyl, *iso-*pentyl, *neo*-pentyl, *n*-hexyl, 2-hexyl, *n*-octyl, ethylenyl (vinyl), ethynyl, propenyl (-CH₂CH=CH₂; -CH=CH-CH₃, -C (=CH₂) -CH₃), propynyl (-CH₂-C≡CH, -C≡C-CH₃), butenyl, butynyl, pentenyl, pentynyl, hexenyl, hexynyl, heptenyl, heptynyl, octenyl and octynyl.

3. Pyrido[2,3-b]pyrazine derivatives of the general Formula I according to Claim 1, **characterized in that** the heterocyclyl radical may be tetrahydrofuryl, tetrahydropyranyl, pyrrolidinyl, piperidinyl, piperazinyl and morpholinyl.

4. Pyrido[2,3-b]pyrazine derivatives of the general Formula I according to Claim 1, **characterized in that** the heteroaryl radical may be pyrrolyl, furyl, thienyl, thiazolyl, oxazolyl, isoxazolyl, pyrazolyl, imidazolyl, pyridinyl, pyrimidinyl, pyridazinyl, pyrazinyl, phthalazinyl, indolyl, indazolyl, indolizinyl, quinolinyl, isoquinolinyl, quinoxalinyl, quinazolinyl, carbazolyl, phenazinyl, phenothiazinyl, acridinyl.

5. Pyrido[2,3-b]pyrazine derivatives of the general Formula I according to Claims 1 to 4, especially one of the following compounds:
1-allyl-3-(3-phenylpyrido[2,3-b]pyrazin-6-yl)thiourea
1-allyl-3-(3-naphthalen-2-ylpyrido[2,3-b]pyrazin-6-yl)thiourea
1-allyl-3-[3-(4-methoxyphenyl)pyrido[2,3-b]pyrazin-6-yl]thiourea
1-allyl-3-[3-(4-hydroxyphenyl)pyrido[2,3-b]pyrazin-6-yl]thiourea hydrochloride
1-(2-methylallyl)-3-(3-phenylpyrido[2,3-b]pyrazin-6-yl)thiourea
1-(2-methylallyl)-3-(3-naphthalen-2-ylpyrido[2,3-b]pyrazin-6-yl)thiourea
1-[3-(4-methoxyphenyl)pyrido[2,3-b]pyrazin-6-yl)-3-(2-methylallyl)thiourea
1-(3-naphthalen-2-ylpyrido[2,3-b]pyrazin-6-yl)-3-(4-nitrophenyl) thiourea
1-[3-(4-methoxyphenyl)pyrido[2,3-b]pyrazin-6-yl]-3-(4-nitrophenyl)thiourea
1-*tert*-butyl-3-(3-phenylpyrido[2,3-b]pyrazin-6-yl)thiourea
1-cyclopropyl-3-(3-phenylpyrido[2,3-b]pyrazin-6-yl)thiourea
1-methyl-3-(3-phenylpyrido[2,3-b]pyrazin-6-yl)thiourea
1-benzyl-3-(3-phenylpyrido[2,3-b]pyrazin-6-yl)thiourea
1-(4-fluorophenyl)-3-(3-phenylpyrido[2,3-b]pyrazin-6-yl)thiourea
1-cyclohexyl-3-(3-phenylpyrido[2,3-b]pyrazin-6-yl)thiourea
1-isopropyl-3-(3-phenylpyrido[2,3-b]pyrazin-6-yl)thiourea
1-furan-2-ylmethyl-3-(3-phenylpyrido[2,3-b]pyrazin-6-yl)thiourea
1-methyl-3-[3-(4-nitrophenyl)pyrido[2,3-b]pyrazin-6-yl]thiourea
1-[3-(4-hydroxyphenyl)pyrido[2,3-]pyrazin-6-yl]-3-methylthiourea
1-allyl-3-[3-(4-nitrophenyl)pyrido[2,3-b]pyrazin-6-yl]-thiourea
ethyl 4-[6-(3-allylthiourea)pyrido[2,3-b]pyrazin-3-yl]-benzoate
1-allyl-3-[3-(3-hydroxyphenyl)pyrido[2,3-b]pyrazin-6-yl]thiourea
1-allyl-3-(3-benzo[1,3]dioxol-5-ylpyrido[2,3-b]pyrazin-6-yl)thiourea
1-[3-(4-hydroxyphenyl)pyrido[2,3-b]pyrazin-6-yl]-3-prop-2-ynylthiourea
1-allyl-3-[3-(4-hydroxyphenyl)pyrido[2,3-b]pyrazin-6-yl]thiourea
1-[3-(4-hydroxyphenyl)pyrido[2,3-b]pyrazin-6-yl]-3-(propenyl)thiourea
1-allyl-3-[2,3-bis(4-hydroxyphenyl)pyrido[2,3-b]pyrazin-6-yl]thiourea
1-[2,3-bis(4-hydroxyphenyl)pyrido[2,3-b]pyrazin-6-yl]-3-(propenyl)thiourea
1-allyl-3-[2-(4-hydroxyphenyl)pyrido[2,3-b]pyrazin-6-yl]thiourea
1-allyl-3-[3-(4-nitrophenyl)pyrido[2,3-b]pyrazin-7-yl]thiourea
1-cyclopropyl-3-(3-phenylpyrido[2,3-b]pyrazin-6-yl)urea
1-allyl-3-[3-(4-hydroxyphenyl)pyrido[2,3-b]pyrazin-6-yl]urea
1-(3-phenylpyrido[2,3-b]pyrazin-6-yl)-3-p-tolylurea
1-(4-chloro-3-trifluoromethylphenyl)-3-(3-phenylpyrido-[2,3-b]pyrazin-6-yl)urea
1-(2-morpholin-4-ylethyl)-3-(3-phenylpyrido[2,3-b]pyrazin-6-yl)urea
1-phenethyl-3-(3-phenylpyrido[2,3-]pyrazin-6-yl)urea
1-(2,3-dipyridin-2-ylpyrido[2,3-b]pyrazin-6-yl)-3-ethylurea
1-(2,3-dimethylpyrido[2,3-b]pyrazin-6-yl)-3-ethylurea

6. Medicament, containing at least one compound of the general Formula I according to Claims 1 to 5.

7. Medicament according to Claim 6, where the compound is present in the composition in combination with at least one further pharmaceutical active ingredient and/or pharmaceutical carriers and/or diluents or other excipients.

8. Process for producing a medicament according to Claim 6 and 7, **characterized in that** one or more pyrido[2,3-b]pyrazine derivatives of the general Formula I according to any of Claims 1 to 5 are processed to pharmaceutical preparations or converted into a therapeutically usable form with conventional pharmaceutical carriers and/or diluents or other auxiliary substances.

9. Compound according to one of Claims 1 to 5 for use as a pharmaceutical agent.

10. Use of the pyrido[2,3-b]pyrazine derivatives of the general Formula I according to any of Claims 1 to 5 as therapeutic active ingredients for producing medicaments for the treatment of disorders which result from misdirected cellular signal transduction processes.

11. Use according to Claim 10 for the treatment of disorders based on pathological cell proliferations such as restenosis, psoriasis, arteriosclerosis and cirrhosis of the liver.

12. Use according to Claim 10 for the treatment of malignant or benign oncoses, in particular of the breast, prostate, lung, skin and ovaries.

13. Use according to Claims 10 to 12 for the treatment of disorders which result from misdirected cellular signal transduction processes, and of disorders based on pathological cell proliferations and malignant or benign oncoses in humans, in mammals and in poultry.

14. Use according to Claim 10 for the modulation of misdirected cellular signal transduction processes, in particular for influencing the function of active and inactive tyrosine and serine/threonine kinases, such as c-Raf, B-Raf, Mek, MAPKs, PDGFRbeta, Flt-3, IGF1R, PKB/Akt1, c-Kit, c-Abl, FGFR1 and KDR.

## Revendications

1. Nouveaux dérivés de pyrido[2,3-b]pyrazine répondant à la formule générale 1 : 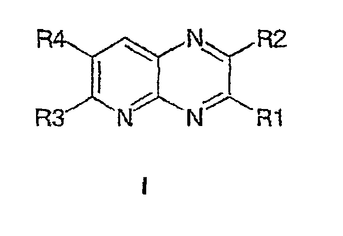 dans laquelle les substituants R1-R4 ont la signification suivante :
R1 et R2 peuvent représenter, indépendamment l'un de l'autre :
(i) hydrogène,
(ii) hydroxyle,
(iii) halogène,
(iv) alkyle, le radical alkyle étant saturé et pouvant être constitué de 1 à 8 atome(s) de carbone,
(v) aryle non substitué ou substitué, le radical aryle pouvant être substitué une ou plusieurs fois, de manière identique ou différente, par F, Cl, Br , I , CF₃, CN, NH₂, NH-alkyle, NH-cycloalkyle, NH-hétérocyclyle, NH-aryle, NH-hétéroaryle, NH-alkyl-cycloalkyle, NH-alkyl-hétérocyclyle, NH-alkyl-aryle, NH-alkyl-hétéroaryle, NH-Alkyl-NH₂, NH-alkyl-OH, N(alkyl)₂, NHC(O)-alkyle, NHC(O)-cycloalkyle, NHC(O)-hétérocyclyle, NHC(O)-aryle, NHC(O)-hétéroaryle, NHC(O)alkyl-aryle, NHC(O)-alkyl-hétéroaryle, NHSO₂-alkyle, NHSO₂-cycloalkyle, NHSO₂-hétérocyclyle, NHSO₂-aryle, NHSO₂-hétéroaryle, NHSO₂-alkyl-aryle, NHSO₂-alkyl-hétéroaryle, NO₂, SH, S-alkyle, S-aryle, S-hétéroaryle, OH, OCF₃, 0-alkyle, O-cycloalkyle, O-hétérocyclyle, O-aryle, O-hétéroaryle, O-alkyl-cycloalkyle, O-alkyl-hétérocyclyle, O-alkyl-aryle, O-alkyl-hétéroaryle, 0-alkyl-OH, O-(CH₂)ₙ-O, OC(O)-alkyle, OC(O)-cycloalkyle, OC(O)-hétérocyclyle, OC(O)-aryle, OC(O)-hétéroaryle, OC(O)-alkyl-aryle, OC(O)-alkyl-hétéroaryle, OSO₃H, OSO₂-alkyle, OSO₂-cycloalkyle, OSO₂-hétérocyclyle, OSO₂-aryle, OSO₂-hétéroaryle, OSO₂-alkyl-aryle, OSO₂-alkyl-hétéroaryle, OP(O)(OH)₂, C(O)-alkyle, C(O)-aryle, C(O)-hétéroaryle, CO₂H, CO₂-alkyle, CO₂-cycloalkyle, CO₂-hétérocyclyle, CO₂-aryle, CO₂-hétéroaryle, CO₂-alkyl-cycloalkyle, CO₂-alkyl-hétérocyclyle, CO₂-alkyl-aryle, CO₂-alkyl-hétéroalkyle, C(O)-NH₂, C(O)NH-alkyle, C(O)NH-cycloalkyle, C(O)NH-hétérocyclyle, C(O)NH-aryle, C(O)NH-hétéroaryle, C(O)NH-alkyl-cycloalkyle, C(O)NH-alkyl-hétérocyclyle, C(O)NH-alkyl-aryle, C(O)NH-alkyl-hétéroaryle, C(O)N(alkyl)₂, C(O)N(cycloalkyl)₂ , C(O)N(aryl)₂, C(O)N(hétéroaryl)₂, SO-alkyle, SO-aryle, SO₂-alkyle, SO₂-aryle, SO₂NH₂, SO₂NH-alkyle, SO₂NH-aryle, SO₂NH-hétéroaryle, SO₂NH-alkyl-aryle, S03H, SO₂O-alkyle, SO₂O-aryle, SO₂O-alkyl-aryle, alkyle, cycloalkyle, hétérocyclyle, aryle ou hétéroaryle, n pouvant avoir pour valeur 1, 2 ou 3 et les substituants alkyle, cycloalkyle, hétérocyclyle, aryle, hétéroaryle, alkyl-cycloalkyle, alkyl-hétérocyclyle, alkyle-aryle et alkyl-hétéroaryle pouvant eux-mêmes être à nouveau substitués,
(vi) hétéroaryle non substitué ou substitué, le radical hétéroaryle pouvant être substitué une ou plusieurs fois de manière identique ou différente par F, Cl, Br, I , CF₃, CN, NH₂, NH-alkyle, NH-cycloalkyle, NH-hétérocyclyle, NH-aryle, NH-hétéroaryle, NH-alkyl-cycloalkyle, NH-alkyl-hétérocyclyle, NH-alkyl-aryle, NH-alkyl-hétéroaryle, NH-Alkyl-NH₂, NH-alkyl-OH, N(alkyl)2 , NHC(O)-alkyle, NHC(O)-cycloalkyle, NHC(O)-hétérocyclyle, NHC(O)-aryle, NHC(O)-hétéroaryle, NHC(O)alkyl-aryle, NHC(O)-alkyl-hétéroaryle, NHSO₂-alkyle, NHSO₂-cycloalkyle, NHSO₂-hetérocyclyle, NHSO₂-aryle, NHSO₂-hétéroaryle, NHSO₂-alkyl-aryle, NHSO₂-alkyl-hétéroaryle, NO₂, SH, S-alkyle, S-aryle, S-hétéroaryle, OH, OCF₃, O-alkyle, O-cycloalkyle, O-aryle, O-hétéroaryle, O-alkyl-cycloalkyle, O-alkyl-hétérocyclyle, O-alkyl-aryle, O-alkyl-hétéroaryle, OC(O)-alkyle, OC(O)-cycloalkyle, OC(O)-hétérocyclyle, OC(O)-aryle, OC(O)-hétéroaryle, OC(O)-alkyl-aryle, OC(O)-alkyl-hétéroaryle, OSO₃H, OSO₂-alkyle, OSO₂-cycloalkyle, OSO₂-hétérocyclyle, OSO₂-aryle, OSO₂-hétéroaryle, OSO₂-alkyl-aryle, OSO₂-alkyl-hétéroaryle, OP(O)(OH)₂, CO(O)-alkyle, CO(O)-aryle, CO(O)-hétéroaryle, CO₂-H, CO₂-alkyle, CO₂-cycloalkyle, CO₂-hétérocyclyle, CO₂-aryle, CO₂-hétéroaryle, CO₂-alkyl-cycloalkyle, CO₂-alkyl-hétérocyclyle, CO₂-alkyl-aryle, CO₂-alkyl-hétéroalyle, C(O)-NH₂, C(O)NH-alkyle, C(O)NH-cycloalkyle, C(O)NH-hétérocyclyle, C(O)NH-aryle, C(O)NH-hétéroaryle, C(O)NH-alkyl-cycloalkyle, C(O)NH-alkyl-hétérocyclyle, C(O)NH-alkyl-aryle, C(O)NH-alkyl-hétéroaryle, C(O)N(alkyl)₂, C(O)N(cycloalkyl)₂, C(O)N(aryl)₂, C(O)N(hétéroaryl)₂, SO₂-NH₂, SO₂-NH-alkyle, SO₂-NH-aryle, SO₂-NH-hétéroaryle, SO₂-NH-alkyl-aryle, S03H, SO₂O-alkyle, SO₂O-aryle, SO₂O-alkyl-aryle, alkyle, cycloalkyle, hétérocyclyle, aryle ou hétéroaryle, et les substituants alkyle, cycloalkyle, hétérocyclyle, aryle et hétéroaryle, alkyl-cycloalkyle, alkyl-hétérocyclyle, alkyle-aryle et alkyl-hétéroaryle pouvant eux-mêmes être à nouveau substitués,
(vii) OR5, R5 pouvant être alkyle, cycloalkyle, hétérocyclyle, aryle, hétéroaryle, alkyl-cycloalkyle, alkyl-hétérocyclyle, alkyl-aryle ou alkyl-hétéroaryle et les substituants alkyle, cycloalkyle, hétérocyclyle, aryle, hétéroaryle, alkyl-cycloalkyle, alkyl-hétérocyclyle, alkyl-aryle ou alkyl-hétéroaryle pouvant être eux-mêmes à nouveau substitués,
(viii) SR6, R6 pouvant être alkyle, cycloalkyle, hétérocyclyle, aryle, hétéroaryle, alkyl-cycloalkyle, alkyl-hétérocyclyle, alkyl-aryle ou alkyl-hétéroaryle et les substituants alkyle, cycloalkyle, hétérocyclyle, aryle, hétéroaryle, alkyl-cycloalkyle, alkyl-hétérocyclyle, alkyl-aryle ou alkyl-hétéroaryle pouvant être eux-mêmes à nouveau substitués,
(ix) NR7R8, R7 et R8 pouvant être, indépendamment l'un de l'autre, hydrogène, alkyle, cycloalkyle, hétérocyclyle, aryle, hétéroaryle , alkyl-cycloalkyle, alkyl-hétérocyclyle, alkyl-aryle ou alkyl-hétéroaryle et les substituants alkyle, cycloalkyle, hétérocyclyle, aryle et hétéroaryle, alkyl-cycloalkyle, alkyl-hétérocyclyle, alkyl-aryle ou alkyl-hétéroaryle pouvant être eux-même à nouveau substitués ou R7 et R8 représentent ensemble cycloalkyle ou hétérocyclyle, cycloalkyle et hétérocyclyle pouvant être eux-mêmes à nouveau substitués,
R3 et R4 peuvent représenter, indépendamment l'un de l'autre, l'hydrogène ou le NR9R10, à la condition que si R3 = NR9R10, R4 = H et si R4 = NR9R10, R3 = H,
R9 pouvant être hydrogène, alkyle, cycloalkyle, hétérocyclyle, aryle, hétéroaryle, alkyl-cycloalkyle, alkyl-hétérocyclyle, alkyl-aryle, ou alkyl-hétéroaryle et les substituants alkyle, cycloalkyle, hétérocyclyle, aryle, et hétéroaryle, alkyl-cycloalkyle, alkyl-hétérocyclyle, alkyl-aryle ou alkyl-hétéroaryle pouvant être eux-mêmes à nouveau substitués,
et R10:
pouvant représenter C(Y) NR11R12 ou Y = O, S et R11 et R12 pouvant représenter, indépendamment l'un de l'autre,
(i) hydrogène,
(ii) alkyle non substitué ou substitué, le radical alkyle pouvant être substitué une ou plusieurs fois, de manière identique ou différente, par F, Cl, Br , I , CF₃, CN, NH₂, NH-alkyle, NH-cycloalkyle, NH-hétérocyclyle, NH-aryle, NH-hétéroaryle, NH-alkyl-cycloalkyle, NH-alkyl-hétérocyclyle, NH-alkyl-aryle, NH-alkyl-hétéroaryle, N(alkyl)₂, NHC(O)-alkyle, NHC(O)-cycloalkyle, NHC(O)-hétérocyclyle, NHC(O)-aryle, NHC(O)-hétéroaryle, NHC(O)alkyl-aryle, NHC(O)-alkyl-hétéroaryle, NHSO₂-alkyle, NHSO₂ - cycloalkyle, NHSO₂ -hetérocyclyle, NHSO₂ -aryle, NHSO₂-hétéroaryle, NHSO₂ -alkyl-aryle, NHSO₂-alkyl-hétéroaryle, NO₂, SH, S-alkyle, S-cycloalkyle, S-hétérocyclyle, S-aryle, S-hétéroaryle, OH, OCF₃, O-alkyle, O-cycloalkyle, O-hétérocyclyle, O-aryle, O-hétéroaryle, O-alkyl-cycloalkyle, O-alkyl-hétérocyclyle, O-alkyl-aryle, O-alkyl-hétéroaryle, OC(O)-alkyle, OC(O)-cycloalkyle, OC(O)-hétérocyclyle, OC(O)-aryle, OC(O)-hétéroaryle, OC(O)-alkyl-aryle, OC(O)-alkyl-hétéroaryle, OSO₃H, OSO₂-alkyle, OSO₂-cycloalkyle, OSO₂-hétérocyclyle, OSO₂-aryle, OSO₂-hétéroaryle, OSO₂-alkyl-aryle, OSO₂-alkyl-hétéroaryle, OP(O)(OH)₂, C(O)-alkyle, C(O)-aryle, C(O)-hétéroaryle, CO₂H, CO₂-alkyle, CO₂-cycloalkyle, CO₂-hétérocyclyle, CO₂- aryle, CO₂ - hétéroaryle, CO₂-alkyl-cycloalkyle, CO₂-alkyl-hétérocyclyle, CO₂-alkyl-aryle, CO₂-alkyl-hétéroaryle, C(O)-NH₂, C(O)NH-alkyle, C(O)NH-cycloalkyle, C(O)NH-hétérocyclyle, C(O)NH-aryle, C(O)NH-hétéroaryle, C(O)NH-alkyl-cycloalkyle, C(O)NH-alkyl-hétérocyclyle, C(O)NH-alkyl-aryle, C(O)NH-alkyl-hétéroaryle, C(O)N(alkyl)₂, C(O)N(cycloalkyl)₂ , C(O)N(aryl)₂, C(O)N(hétéroaryl)₂, SO-alkyle, SO-aryle, SO₂-alkyle, SO₂-aryle, SO₂-NH₂, SO₂-NH-alkyle, SO₂-NH-aryle, SO₂-NH-hétéroaryle, SO₂-NH-alkyl-aryle, S03H, SO₂O-alkyle, SO₂O-aryle, SO₂O-alkyl-aryle, cycloalkyle, hétérocyclyle, aryle ou hétéroaryle,
(iii) cycloalkyle non substitué ou substitué, le radical cycloalkyle pouvant être substitué une ou plusieurs fois, de manière identique ou différente, par F, Cl, Br , I , NH₂, NH-alkyle, NH-cycloalkyle, NH-hétérocyclyle, NH-aryle, NH-hétéroaryle, NH-alkyl-aryle, NH-alkyl-hétéroaryle, N(alkyl)₂, NHC(O)-alkyle, NHC(O)-cycloalkyle, NHC(O)-hétérocyclyle, NHC(O)-aryle, NHC(O)-hétéroaryle, NHC(O)alkyl-aryle, NHC(O)-alkyl-hétéroaryle, NHSO₂-alkyle, NHSO₂ -cycloalkyle, NHSO₂ -hétérocyclyle, NHSO₂ -aryle, NHSO₂-hétéroaryle, NHSO₂ -alkyl-aryle, NHSO₂-alkyl-hétéroaryle, OH, O-alkyle, O-cycloalkyle, O-hétérocyclyle, O-aryle, O-hétéroaryle, O-alkyl-aryle, 0-alkyl-hétéroaryle, OC(O)-alkyle, OC(O)-cycloalkyle, OC(O)-hétérocyclyle, OC(O)-aryle, OC(O)-hétéroaryle, OC(O)-alkyl-aryle, OC(O)-alkyl-hétéroaryle, OSO₃H, OSO₂-alkyle, OSO₂-cycloalkyle, OSO₂-hétérocyclyle, OSO₂-aryle, OSO₂-hétéroaryle, OSO₂-alkyl-aryle, OSO₂-alkyl-hétéroaryle, OP(O)(OH)₂, CO₂-H, CO₂-alkyle, CO₂-cycloalkyle, CO₂-hétérocyclyle, CO₂- aryle , CO₂-hétéroaryle, CO₂-alkyl-cycloalkyle, CO₂-alkyl-hétérocyclyle, CO₂-alkyl-aryle, CO₂-alkyl-hétéroalkyle, C(O)-NH₂, C(O)NH-alkyle, C(O)NH-cycloalkyle, C(O)NH-hétérocyclyle, C(O)NH-aryle, C(O)NH-hétéroaryle, C(O)NH-alkyl-cycloalkyle, C(O)NH-alkyl-hétérocyclyle, C(O)NH-alkyl-aryle, C(O)NH-alkyl-hétéroaryle, C(O)N(alkyl)₂, C(O)N(cycloalkyl)₂ , C(O)N(aryl)₂ , C(O)N(hétéroaryl)₂, alkyle ou aryle,
(iv) hétérocyclyle non substitué ou substitué, le radical hétérocyclyle pouvant être substitué une ou plusieurs fois, de manière identique ou différente, par OH, O-alkyle, O-aryle, NH₂, NH-alkyle, NH- aryle, alkyle, alkyl-aryle, ou aryle,
(v) aryle non substitué ou substitué, le radical aryle pouvant être substitué une ou plusieurs fois, de manière identique ou différente, par F, Cl, Br , I , CF₃, CN, NH₂, NH-alkyle, NH-cycloalkyle, NH-hétérocyclyle, NH-aryle, NH-hétéroaryle, NH-alkyl-cycloalkyle, NH-alkyl-hétérocyclyle, NH-alkyl-aryle, NH-alkyl-hétéroaryle, NH-Alkyl-NH₂, NH-alkyl-OH, N(alkyl)₂, NHC(O)-alkyle, NHC(O)-cycloalkyle, NHC(O)-hétérocyclyle, NHC(O)-aryle, NHC(O)-hétéroaryle, NHC(O)alkyl-aryle, NHC(O)-alkyl-hétéroaryle, NHSO₂-alkyle, NHSO₂-cycloalkyle, NHSO₂-hetérocyclyle, NHSO₂ -aryle, NHSO₂-hétéroaryle, NHSO₂ -alkyl-aryle, NHSO₂-alkyl-hétéroaryle, NO₂, SH, S-alkyle, S-cycloalkyle, S-hétérocyclyle, S-aryle, S-hétéroaryle, OH, OCF₃, O-alkyle, O-cycloalkyle, O-hétérocyclyle, O-aryle, O-hétéroaryle, O-alkyl-cycloalkyle, O-alkyl-hétérocyclyle, O-alkyl-aryle, O-alkyl-hétéroaryle, O-alkyl-OH, O-(CH₂)ₙ-O, OC(O)-alkyle, OC(O)-cycloalkyle, OC(O)-hétérocyclyle, OC(O)-aryle, OC(O)-hétéroaryle, OC(O)-alkyl-aryle, OC(O)-alkyl-hétéroaryle, OSO₃H, OSO₂-alkyle, OSO₂-cycloalkyle, OSO₂-hétérocyclyle, OSO₂-aryle, OSO₂-hétéroaryle, OSO₂-alkyl-aryle, OSO₂-alkyl-hétéroaryle, OP(O)(OH)₂, C(O)-alkyle, C(O)-aryle, C(O)-hétéroaryle, CO₂-H, CO₂-alkyle, CO₂-cycloalkyle, CO₂-hétérocyclyle, CO₂-aryle, CO₂-hétéroaryle, CO₂-alkyl-cycloalkyle, CO₂-alkyl-hétérocyclyle, CO₂-alkyl-aryle, CO₂-alkyl-hétéroalyle, C(O)-NH₂, C(O)NH-alkyle, C(O)NH-cycloalkyle, C(O)NH-hétérocyclyle, C(O)NH-aryle, C(O)NH-hétéroaryle, C(O)NH-alkyl-cycloalkyle, C(O)NH-alkyl-hétérocyclyle, C(O)NH-alkyl-aryle, C(O)NH-alkyl-hétéroaryle, C(O)N(alkyl)₂, C(O)N(cycloalkyl)₂, C(O)N(aryl)₂, C(O)N(hétéroaryl)₂, SO-alkyle, SO-aryle, SO₂-alkyle, SO₂-aryle, SO₂NH₂, SO₂-NH-alkyle, SO₂-NH-aryle, SO₂-NH-hétéroaryle, SO₂-NH-alkyl-aryle, S03H, SO₂O-alkyle, SO₂O-aryle, SO₂O-alkyl-aryle, alkyle, cycloalkyle, hétérocyclyle, aryle ou hétéroaryle , et n peut avoir pour valeur 1, 2 ou 3,
(vi) hétéroaryle non substitué ou substitué, le radical hétéroaryle pouvant être substitué une ou plusieurs fois, de manière identique ou différente, par F, Cl, Br, I , CF₃, CN, NH₂, NH-alkyle, NH-cycloalkyle, NH-hétérocyclyle, NH-aryle, NH-hétéroaryle, NH-alkyl-cycloalkyle, NH-alkyl-hétérocyclyle, NH-alkyl-aryle, NH-alkyl-hétéroaryle, NH-Alkyl-NH₂, NH-alkyl-OH, N(alkyl)₂, NHC(O)-alkyle, NHC(O)-cycloalkyle, NHC(O)-hétérocyclyle, NHC(O)-aryle, NHC(O)-hétéroaryle, NHC(O)alkyl-aryle, NHC(O)-alkyl-hétéroaryle, NHSO₂-alkyle, NHSO₂-cycloalkyle, NHSO₂-hetérocyclyle, NHSO₂-aryle, NHSO₂-hétéroaryle, NHSO₂ -alkyl-aryle, NHSO₂-alkyl-hétéroaryle, NHSO₂- alkyle, NHSO₂- cycloalkyle, NHSO₂- hétérocyclyle, NHSO₂- aryle, NHSO₂- hétéroaryle, NHSO₂- alkyl-aryle, NHSO₂- alkyl-hétéroaryle NO₂, SH, S-alkyle, S-aryle, S-hétéroaryle, OH, OCF₃, 0-alkyle, O-cycloalkyle, O-aryle, O-hétéroaryle, O-alkyl-cycloalkyle, O-alkyl-hétérocyclyle, O-alkyl-aryle, O-alkyl-hétéroaryle, OC(O)-alkyle, OC(O)-cycloalkyle, OC(O)-hétérocyclyle, OC(O)-aryle, OC(O)-hétéroaryle, OC(O)-alkyl-aryle, OC(O)-alkyl-hétéroaryle, OSO₃H, OSO₂-alkyle, OSO₂-cycloalkyle, OSO₂-hétérocyclyle, OSO₂-aryle, OSO₂-hétéroaryle, OSO₂-alkyl-aryle, OSO₂-alkyl-hétéroaryle, OP(O)(OH)₂, C(O)-alkyle, C(O)-aryle, CO(O)-hétéroaryle, CO₂H, CO₂-alkyle, CO₂-cycloalkyle, CO₂-hétérocyclyle, CO₂-aryle, CO₂-hétéroaryle, CO₂-alkyl-cycloalkyle, CO₂-alkyl-hétérocyclyle, CO₂-alkyl-aryle, CO₂-alkyl-hétéroalkyle, C(O)-NH₂, C(O)NH-alkyle, C(O)NH-cycloalkyle, C(O)NH-hétérocyclyle, C(O)NH-aryle, C(O)NH-hétéroaryle, C(O)NH-alkyl-cycloalkyle, C(O)NH-alkyl-hétérocyclyle, C(O)NH-alkyl-aryle, C(O)NH-alkyl-hétéroaryle, C(O)N(alkyl)₂, C(O)N(cycloalkyl)₂, C(O)N(aryl)₂, C(O)N(hétéroaryl)₂, SO₂NH₂, SO₂-NH-alkyle, SO₂-NH-aryle, SO₂-NH-hétéroaryle, SO₂-NH-alkyl-aryle, SO₃H, SO₂O-alkyle, SO₂O-aryle, SO₂O-alkyl-aryle, alkyle, cycloalkyle, hétérocyclyle, aryle ou hétéroaryle,
(vii) -C(O)-R17, R17 pouvant être alkyle, aryle ou hétéroaryle et les substituants alkyle et aryle pouvant eux-mêmes être à nouveau substitués,
(viii) ou R11 et R12 peuvent représenter , ensemble, cycloalkyle ou hétérocyclyle,
-C(Y)NR13R14, Y = NH et R13 et 14 représentant, indépendamment l'un de l'autre, :
(i) hydrogène,
(ii), alkyle non substitué ou substitué, le radical alkyle pouvant être substitué une ou plusieurs fois, de manière identique ou différente, par F, Cl, Br , I , CF₃, CN, NH₂, NH-alkyle, NH-cycloalkyle, NH-hétérocyclyle, NH-aryle, NH-hétéroaryle, NH-alkyl-aryle, NH-alkyl-hétéroaryle, N(alkyl)₂, NHC(O)-alkyle, NHC(O)-cycloalkyle, NHC(O)-hétérocyclyle, NHC(O)-aryle, NHC(O)-hétéroaryle, NHSO₂-alkyle, NHSO₂ -cycloalkyle, NHSO₂ -aryle, NHSO₂-hétéroaryle, NO₂, SH, S-alkyle, S-cycloalkyle, S-hétérocyclyle, S-aryle, S-hétéroaryle, OH, OCF₃, O-alkyle, O-cycloalkyle, O-hétérocyclyle, O-aryle, O-hétéroaryle, O-alkyl-cycloalkyle, O-alkyl-aryle, O-alkyl-hétéroaryle, OC(O)-alkyle, OC(O)-cycloalkyle, OC(O)-hétérocyclyle, OC(O)-aryle, OC(O)-hétéroaryle, OSO₂-alkyle, OSO₂-cycloalkyle, OSO₂-aryle, OSO₂-hétéroaryle, C(O)-alkyle, C(O)-aryle, CO₂H, CO₂-alkyle, CO₂-cycloalkyle, CO₂-hétérocyclyle, CO₂ -aryle, CO₂-hétéroaryle, CO₂-alkyl-cycloalkyle, CO₂-alkyl-hétérocyclyle, CO₂-alkyl-aryle, CO₂-alkylhétéroalkyle, C(O)-NH₂, C(O)NH-alkyle, C(O)NH-cycloalkyle, C(O)NH-hétérocyclyle, C(O)NH-aryle, C(O)NH-hétéroaryle, C(O)NH-alkyl-cycloalkyle, C(O)NH-alkyl-hétérocyclyle, C(O)NH-alkyl-aryle, C(O)NH-alkyl-hétéroaryle, C(O)N(alkyl)₂, C(O)N(cycloalkyl)₂, C(O)N(aryl)₂, C(O)N(hétéroaryl)₂, SO-alkyle, SO-aryle, SO₂-alkyle, SO₂-aryle, SO₂-NH₂, SO₃H, alkyle, cycloalkyle, hétérocyclyle, aryle ou hétéroaryle,
(iii) cycloalkyle non substitué ou substitué, le radical cycloalkyle pouvant être substitué une ou plusieurs fois, de manière identique ou différente, par F, Cl, Br , I , NH₂, NH-alkyle, NH-cycloalkyle, NH-hétérocyclyle, NH-aryle, NH-hétéroaryle, NH-alkyl-aryle, NH-alkyl-hétéroaryle, N(alkyl)₂, NHC(O)-alkyle, NHC(O)-cycloalkyle, NHC(O)-hétérocyclyle, NHC(O)-aryle, NHC(O)-hétéroaryle, NHSO₂-alkyle, NHSO₂ -cycloalkyle, NHSO₂ -aryle, NHSO₂-hétéroaryle, OH, , O-alkyle, O-cycloalkyle, O-hétérocyclyle, O-aryle, O-hétéroaryle, O-alkyl-aryle, O-alkyl-hétéroaryle, OC(O)-alkyle, OC(O)-cycloalkyle, OC(O)-hétérocyclyle, OC(O)-aryle, OC(O)-hétéroaryle, OSO₂-alkyle, OSO₂-cycloalkyle, OSO₂-aryle, OSO₂-hétéroaryle, CO₂H, CO₂-alkyle, CO₂-cycloalkyle, CO₂-hétérocyclyle, CO₂-aryle, CO₂-hétéroaryle, C(O)-NH₂, C(O)NH-alkyle, C(O)NH-cycloalkyle, C(O)NH-hétérocyclyle, C(O)NH-aryle, C(O)NH-hétéroaryle, C(O)NH-alkyl-aryle, C(O)NH-alkyl-hétéroaryle, C(O)N(alkyl)₂, alkyle ou aryle,
(iv) hétérocyclyle non substitué ou substitué, le radical hétérocyclyle pouvant être substitué une ou plusieurs fois, de manière identique ou différente, par OH , O-alkyle, O-aryle, NH₂, NH-alkyle, NH-aryle, Alkyle ou aryle ,
(v) aryle non substitué ou substitué, le radical aryle pouvant être substitué une ou plusieurs fois de manière identique ou différente par F, Cl, Br , I , CF₃, CN, NH₂, NH-alkyle, NH-cycloalkyle, NH-hétérocyclyle, NH-aryle, NH-hétéroaryle, NH-alkyl-cycloalkyle, NH-alkyl-hétérocyclyle, NH-alkyl-aryle, NH-alkyl-hétéroaryle, NH-Alkyl-NH₂, NH-alkyl-OH, N(alkyl)₂, NHC(O)-alkyle, NHC(O)-cycloalkyle, NHC(O)-hétérocyclyle, NHC(O)-aryle, NHC(O)-hétéroaryle, NHSO₂-alkyle, NHSO₂ -aryle, NHSO₂-hétéroaryle, NO₂, SH, S-alkyle, S-cycloalkyle, S-hétérocyclyle, S-aryle, S-hétéroaryle, OH, OCF₃ , O-alkyle, O-cycloalkyle, O-hétérocyclyle, O-aryle, O-hétéroaryle, O-alkyl-cycloalkyle, O-alkyl-hétérocyclyle, O-alkyl-aryle, O-alkyl-hétéroaryle, O-alkyl-OH, O-(CH ₂)ₙ-O, OC(O)-alkyle, OC(O)-cycloalkyle, OC(O)-hétérocyclyle, OC(O)-aryle, OC(O)-hétéroaryle, OSO₂-alkyle, OSO₂-cycloalyle, OSO₂-aryle, OSO₂-hétéroaryle, C(O)-alkyle, C(O)-aryle, C(O)-hétéroaryle, CO₂-H, CO₂-alkyle, CO₂-cycloalkyle, CO₂-hétérocyclyle, CO₂-aryle, CO₂-hétéroaryle CO₂-alkyl-cycloalkyle, CO₂-alkyl-hétérocyclyle, CO₂-alkyl-aryle, CO₂-alkyl-hétéroalkyle, C(O)-NH₂, C(O)NH-alkyle, C(O)NH-cycloalkyle, C(O)NH-hétérocyclyle, C(O)NH-aryle, C(O)NH-hétéroaryle, C(O)NH-alkyl-cycloalkyle, C(O)NH-alkyl-hétérocyclyle, C(O)NH-alkyl-aryle, C(O)NH-alkyl-hétéroaryle, C(O)N(alkyl)₂, C(O)N(cycloalkyl)₂ , C(O)N(aryl)₂ , C(O)N(hétéroaryl)₂, SO-alkyle, SO-aryle, SO₂-alkyle, SO₂-aryle, SO₂NH₂, SO₂NH-alkyle, SO₂-NH-aryle, SO₂-NH-hétéroaryle, SO3H, SO₂O-alkyle, SO₂O-aryle, SO₂O-hétéroaryle, alkyle, cycloalkyle, hétérocyclyle, aryle ou hétéroaryle, et n peut avoir pour valeur 1, 2 ou 3,
(vi) hétéroaryle non substitué ou substitué, le radical hétéroaryle pouvant être substitué une ou plusieurs fois, de manière identique ou différente, par F, Cl, Br , I , CF₃, CN, NH₂, NH-alkyle, NH-cycloalkyle, NH-hétérocyclyle, NH-aryle, NH-hétéroaryle, NH-alkyl-aryle, NH-alkyl-hétéroaryle, N(alkyl)₂, NHC(O)-alkyle, NHC(O)-cycloalkyle, NHC(O)-hétérocyclyle, NHC(O)-aryle, NHC(O)-hétéroaryle, NHSO₂-alkyle, NHSO₂ - aryle, NHSO₂-hétéroaryle, NO₂, SH, S-alkyle, S-aryle, OH, OCF₃, 0-alkyle, O-cycloalkyle, O-hétérocyclyle, O-aryle, O-hétéroaryle, OC(O)-alkyle, OC(O)-cycloalkyle, OC(O)-hétérocyclyle, OC(O)-aryle, OC(O)-hétéroaryle, OSO₂-alkyle, OSO₂-cycloalkyle, OSO₂-aryle, OSO₂-hétéroaryle, C(O)-alkyle, C(O)-aryle, C(O)-hétéroaryle, CO₂-H, CO₂-alkyle, CO₂-cycloalkyle, CO₂-hétérocyclyle, CO₂- aryle, CO₂-hétéroaryle, CO₂-alkyl-cycloalkyle, CO₂-alkyl-hétérocyclyle, CO₂-alkyl-aryle, CO₂-alkyl-hétéroalkyle, C(O)-NH₂, C(O)NH-alkyle, C(O)NH-cycloalkyle, C(O)NH-hétérocyclyle, C(O)NH-aryle, C(O)NH-hétéroaryle, C(O)NH-alkyl-cycloalkyle, C(O)NH-alkyl-hétérocyclyle, C(O)NH-alkyl-aryle, C(O)NH-alkyl-hétéroaryle, C(O)N(alkyl)₂, C(O)N(cycloalkyl)₂ , C(O)N(aryl)₂ , C(O)N(hétéroaryl)₂ , SO₂-alkyle, SO₂-aryle, SO₂-NH₂ , SO₂-NH-alkyle, SO₂-NH-aryle, SO₂-NH-hétéroaryle, S03H, SO₂O-alkyle, SO₂O-aryle, SO₂O-hétéroaryle, alkyle, cycloalkyle, hétérocyclyle, aryle ou hétéroaryle,
(vii) ou R13 et R14 peuvent représenter ensemble cycloalkyle ou hétérocyclyle,
-C(NR15) R16 avec R15 = H et R16
(i) alkyle non substitué ou substitué, le radical alkyle pouvant être substitué une ou plusieurs fois, de manière identique ou différente, par F, Cl, Br , I , CF₃, NH₂, NH-alkyle, NH-cycloalkyle, NH-hétérocyclyle, NH-aryle, NH-hétéroaryle, NH-alkyl-aryle, NH-alkyl-hétéroaryle, N(alkyl)₂, NHC(O)-alkyle, NHC(O)-cycloalkyle, NHC(O)-hétérocyclyle, NHC(O)-aryle, NHC(O)-hétéroaryle, NHSO₂-alkyle, NHSO₂ -cycloalkyle, NHSO₂ -aryle, NHSO₂-hétéroaryle, NO₂, SH, S-alkyle, S-cycloalkyle, S-hétérocyclyle, S-aryle, S-hétéroaryle, OH, OCF₃, O-alkyle, O-cycloalkyle, O-hétérocyclyle, O-aryle, O-hétéroaryle, O-alkyl-cycloalkyle, O-alkyl-aryle, O-alkyl-hétéroaryle, OC(O)-alkyle, OC(O)-cycloalkyle, OC(O)-hétérocyclyle, OC(O)-aryle, OC(O)-hétéroaryle, OSO₂-alkyle, OSO₂-cycloalkyle, OSO₂-aryle, OSO₂-hétéroaryle, CO(O)-alkyle, CO(O)-aryle, CO₂-H, CO₂-alkyle, CO₂-cycloalkyle, CO₂-hétérocyclyle, CO₂-aryle, CO₂-hétéroaryle, CO₂-alkyl-cycloalkyle, CO₂-alkyl-hétérocyclyle, CO₂-alkyl-aryle, CO₂-alkyl-hétéroalkyle, C(O)-NH₂, C(O)NH-alkyle, C(O)NH-cycloalkyle, C(O)NH-hétérocyclyle, C(O)NH-aryle, C(O)NH-hétéroaryle, C(O)NH-alkyl-cycloalkyle, C(O)NH-alkyl-hétérocyclyle, C(O)NH-alkyl-aryle, C(O)NH-alkyl-hétéroaryle, C(O)N(alkyl)₂, C(O)N(cycloalkyl)₂, C(O)N(aryl)₂ , C(O)N(hétéroaryl)₂ , SO-alkyle, SO-aryle, SO₂-alkyle, SO₂-aryle, SO₂-NH₂, S03H, alkyle, cycloalkyle, hétérocyclyle, aryle ou hétéroaryle,
(ii) cycloalkyle non substitué ou substitué, le radical cycloalkyle pouvant être substitué une ou plusieurs fois, de manière identique ou différente, par F, Cl, Br , I , NH₂ , NH-alkyle, NH-cycloalkyle, NH-hétérocyclyle, NH-aryle, NH-hétéroaryle, NH-alkyl-aryle, NH-alkyl-hétéroaryle, N(alkyl)₂ , NHC(O)-alkyle, NHC(O)-cycloalkyle, NHC(O)-hétérocyclyle, NHC(O)-aryle, NHC(O)-hétéroaryle, NHSO₂ - alkyle, NHSO₂ -cycloalkyle, NHSO₂ -aryle, NHSO₂-hétéroaryle, OH, O-alkyle, O-cycloalkyle, O-hétérocyclyle, O-aryle, O-hétéroaryle, O-alkyl-aryle, O-alkyl-hétéroaryle, OC(O)-alkyle, OC(O)-cycloalkyle, OC(O)-hétérocyclyle, OC(O)-aryle, OC(O)-hétéroaryle, OSO₂-alkyle, OSO₂-cycloalkyle, OSO₂-aryle, OSO₂-hétéroaryle, CO₂H, CO₂-alkyle, CO₂-cycloalkyle, CO₂-hétérocyclyle, CO₂-aryle, CO₂-hétéroaryle, C(O)-NH₂, C(O)NH-alkyle, C(O)NH-cycloalkyle, C(O)NH-hétérocyclyle, C(O)NH-aryle, C(O)NH-hétéroaryle, C(O)NH-alkyl-aryle, C(O)NH-alkyl-hétéroaryle, C(O)N(alkyl)₂, alkyle ou aryle,
(iii) hétérocyclyle non substitué ou substitué, le,radical hétérocyclyle pouvant être substitué une ou plusieurs fois, de manière identique ou différente, par OH, O-alkyle, O-aryle, NH₂, NH-alkyle, NH-aryle, alkyle ou aryle,
(iv) aryle non substitué ou substitué, le radical aryle pouvant être substitué une ou plusieurs fois, de manière identique ou différente, par F, Cl, Br , I, CF₃ , NH₂ , NH-alkyle, NH-cycloalkyle, NH-hétérocyclyle, NH-aryle, NH-hétéroaryle, NH-alkyl-cycloalkyle, NH-alkyl-hétérocyclyle, NH-alkyl-aryle, NH-alkyl-hétéroaryle, NH-Alkyl-NH₂ , NH-alkyl-OH, N(alkyl)₂, NHC(O)-alkyle, NHC(O)-cycloalkyle, NHC(O)-hétérocyclyle, NHC(O)-aryle, NHC(O)-hétéroaryle, NHSO₂-alkyle, NHSO₂-aryle, NHSO₂-hétéroaryle, NO₂, SH, S-alkyle, S-cycloalkyle, S-hétérocyclyle, S-aryle, S-hétéroaryle, OH, OCF₃, O-alkyle, O-cycloalkyle, O-hétérocyclyle, O-aryle, O-hétéroaryle, O-alkyl-cycloalkyle, O-alkyl-hétérocyclyle, O-alkyl-aryle, O-alkyl-hétéroaryle, 0-alkyl-OH, O-(CH₂)ₙ-O, OC(O)-alkyle, OC(O)-cycloalkyle, OC(O)-hétérocyclyle, OC(O)-aryle, OC(O)-hétéroaryle, OSO₂-alkyle, OSO₂-cycloalkyle, OSO₂-aryle, OSO₂-hétéroaryle, CO(O)-alkyle, CO(O)-aryle, CO(O)-hétéroaryle, CO₂H, CO₂-alkyle, CO₂-cycloalkyle, CO₂-hétérocyclyle, CO₂-aryle, CO₂-hétéroaryle, CO₂-alkyl-cycloalkyle, CO₂-alkyl-hétérocyclyle, CO₂-alkyl-aryle, CO₂-alkyl-hétéroalkyle, C(O)-NH₂, C(O)NH-alkyle, C(O)NH-cycloalkyle, C(O)NH-hétérocyclyle, C(O)NH-aryle, C(O)NH-hétéroaryle, C(O)NH-alkyl-cycloalkyle, C(O)NH-alkyl-hétérocyclyle, C(O)NH-alkyl-aryle, C(O)NH-alkyl-hétéroaryle, C(O)N(alkyl)₂, C(O)N(cycloalkyl)₂ , C(O)N(aryl)₂, C(O)N(hétéroaryl)₂, SO-alkyle, SO-aryle, SO₂-alkyle, SO₂-aryle, SO₂-NH₂, SO₂-NH-alkyle, SO₂-NH-aryle, SO₂-NH-hétéroaryle, S03H, SO₂O-alkyle, SO₂O-aryle, SO₂O-hétéroaryle, alkyle, cycloalkyle, hétérocyclyle, aryle ou hétéroaryle, et n peut avoir pour valeur 1, 2 ou 3,
(v) hétéroaryle non substitué ou substitué, le radical hétéroaryle pouvant être substitué une ou plusieurs fois, de manière identique ou différente, par F, Cl, Br , I, CF₃, NH₂, NH-alkyle, NH-cycloalkyle, NH-hétérocyclyle, NH-aryle, NH-hétéroaryle, NH-alkyl-aryle, NH-alkyl-hétéroaryle, N(alkyl)₂ , NHC(O)-alkyle, NHC(O)-cycloalkyle, NHC(O)-hétérocyclyle, NHC(O)-aryle, NHC(O)-hétéroaryle, NHSO₂-alkyle, NHSO₂-aryle, NHSO₂-hétéroaryle, NO₂, SH, S-alkyle, S-aryle, OH, OCF₃, O-alkyle, 0-cycloalkyle, O-hétérocyclyle, O-aryle, O-hétéroaryle, OC(O)-alkyle, OC(O)-cycloalkyle, OC(O)-hétérocyclyle, OC(O)-aryle, OC(O)-hétéroaryle, OSO₂-alkyle , OSO₂-cycloalkyle, OSO₂-aryle, OSO₂-hétéroaryle, C(O)-alkyle, C(O)-aryle, C(O)-hétéroaryle, CO₂H, CO₂-alkyle, CO₂-cycloalkyle, CO₂-hétérocyclyle, CO₂-aryle, CO₂-hétéroaryle, CO₂-alkyle-cycloalkyle, CO₂-alkyle-hétérocyclyle, CO₂-alkyl-aryle, CO₂-alkyl-hétéroalyle, C(O)-NH₂, C(O)NH-alkyle, C(O)NH-cycloalkyle, C(O)NH-hétérocyclyle, C(O)NH-aryle, C(O)NH-hétéroaryle, C(O)NH-alkyl-cycloalkyle, C(O)NH-alkyl-hétérocyclyle, C(O)NH-alkyl-aryle, C(O)NH-alkyl-hétéroaryle, C(O)N(alkyl)₂, C(O)N(cycloalkyl)₂ , C(O)N(aryl)₂ , C(O)N(hétéroaryl)₂ , SO₂-alkyle, SO₂-aryle, SO₂-NH₂ , SO₂-NH-alkyle, SO₂-NH-aryle, SO₂-NH-hétéroaryle, S03H, SO₂O-alkyle, SO₂O-aryle, SO₂O-hétéroaryle, alkyle, cycloalkyle, hétérocyclyle, aryle ou hétéroaryle,
ainsi que des sels, dérivés et analogues physiologiquement compatibles, des composés selon la formule I, les sels pouvant être obtenus par neutralisation des composés basiques avec des acides inorganiques et organiques ou neutralisation des composés acides avec des bases inorganiques et organiques, ainsi que leurs solvates, hydrates et formes polymorphes,
les composés répondant à la formule générale I ainsi que leurs sels dérivés ou analogues, leurs solvates , hydrates et formes polymorphes , peuvent exister sous forme de racémiques, sous forme des énantiomères et/ou diastéréomères purs ou sous forme de mélanges de ces énantiomères et/ou diatéréomères ou sous forme des tautomères.

2. Dérivés de pyrido[2,3-b]pyrazine répondant à la formule générale I, selon la revendication 1,
**caractérisés en ce que**
le radical alkyle peut être méthyle, éthyle, n-propyle, 2-propyle, n-butyle, sec.-butyle, tert.-butyle, n-pentyle, iso-pentyle, néo-pentyle, n-hexyle, 2-hexyle, n-octyle, éthylényle (vinyle), éthinyle, propényle, (-CH₂CH=CH₂ ;-CH=CH-CH₃, -C(=CH₂)-CH₃), propinyle (-CH₂-C=CH, -CH=C-CH₃), butényle, butinyle, pentényle, pentinyle, hexényle, hexinyle, heptényle, heptinyle, octényle et octinyle.

3. Dérivés de pyrido[2,3-b]pyrazine répondant à la formule générale I, selon la revendication 1,
**caractérisés en ce que**
le radical hétérocyclyle peut être tétrahydrofuryle, tétrahydropyranyle, pyrolidinyle, pipéridinyle, pipérazinyle et morpholinyle.

4. Dérivés de pyrido[2,3-b]pyrazine répondant à la formule générale I, selon la revendication 1,
**caractérisés en ce que**
le radical hétéroaryle peut être pyrrolyle, furyle, thienyle, thiazolyle, oxazolyle, isoxazolyle, pyrazolyle, imidazolyle, pyridinyle, pyrimidinyle, pyridazinyle, pyrazinyle, phthalazinyle, indolyle, indazolyle, indolizinyle, chinolinyle, isochinolinyle, chinoxalinyle, chinazolinyle, carbazolyle, phénazinyle, phénothiazinyle, acridinyle.

5. Dérivés de pyrido[2,3-b]pyrazine répondant à la formule générale I, selon les revendications 1 à 4 , en particulier un des composés suivants :
1-allyl-3-(3-phényl-pyrido[2, 3-b]pyrazin-6-yl)-thiourée
1-allyl-3-(3-naphtalène-2-yl-pyrido[2, 3-b]pyrazin-6-yl)-thiourée
1-allyl-3-[3-(4-méthoxy-phényl)-pyrido[2, 3-b]pyrazin-6-yl]-thiourée
Chlorhydrate de 1-allyl-3-[3-(4-hydroxy-phényl)-pyrido[2, 3-b]pyrazin-6-yl]-thiourée
1-(2-méthyl-allyl)-3-(3-phényl-pyrido[2,3-b]pyrazin-6-yl)-thiourée
1-(2-méthyl-allyl)-3-(3-naphtalène-2-yl-pyrido[2, 3-b]pyrazin-6-yl)-thiourée
1-[3-(4-méthoxy-phényl)-pyrido[2, 3-b]pyrazin-6-yl]-3-(2-méthyl-allyl)-thiourée
1-(3- naphtalène-2-yl-pyrido[2, 3-b]pyrazin-6-yl)-3-(4-nitro-phényl)-thiourée
1-[3-(4-méthoxy-phényl)-pyrido[2,3-b]pyrazin-6-yl]-3-(4-nitro-phényl)-thiourée
1-tert-butyl-3-(3-phényl-pyrido[2, 3-b]pyrazin-6-yl)-thiourée
1-cyclopropyl-3-(3-phényl-pyrido[2,3-b]pyrazin-6-yl)-thiourée
1-méthyl-3-(3-phényl-pyrido[2,3-b]pyrazin-6-yl)-thiourée
1-benzyl-3-(3-phényl-pyrido[2,3-b]pyrazin-6-yl)-thiourée
1-(4-fuoro-phényl)-3-(3-phényl-pyrido[2,3-b]pyrazin-6-yl)-thiourée
1-cyclohexyl-3-(3-phényl-pyrido[2,3-b]pyrazin-6-yl)-thiourée
1-isopropyl-3-(3-phényl-pyrido[2,3-b]pyrazin-6-yl)-thiourée
1-furan-2-ylméthyl-3-(3-phényl-pyrido[2,3-b]pyrazin-6-yl)-thiourée
1-méthyl-3-[3-(4-nitro-phényl)-pyrido[2,3-b]pyrazin-6-yl]-thiourée
1-[3-(4-hydroxy-phényl)-pyrido[2,3-b]pyrazin-6-yl]-3-méthyl-thiourée
1-allyl-3-[3-(4-nitro-phényl)-pyrido[2,3-b]pyrazin-6-yl]-thiourée
ester éthylique de l'acide 4-[6-(3-allyl-thiourée)-pyrido[2, 3-b]pyrazin-3-yl]benzoïque,
1-allyl-3-[3-(3-hydroxy-phényl)-pyrido[2, 3-b]pyrazin-6-yl]-thiourée
1-allyl-3-(3-benzo[1,3]dioxol-5-yl-pyrido[2,3-b]pyrazin-6-yl)-thiourée
1-[3-(4-hydroxy-phényl)-pyrido[2,3-b]pyrazin-6-yl]-3-prop-2-ynyl-thiourée
1-allyl-3-[3-(4-hydroxy-phényl)-pyrido[2,3-b]pyrazin-6-yl]-thiourée
1-[3-(4-hydroxy-phényl)-pyrido[2, 3-b]pyrazin-6-yl]-3-(propenyl)-thiourée
1-allyl-3-[2,3-bis-(4-hydroxy-phényl)-pyrido[2, 3-b]pyrazin-6-yl]-thiourée
1-[2,3-bis-(4-hydroxy-phényl)-pyrido[2, 3-b]pyrazin-6-yl]-3-(propènyl)-thiourée
1-allyl-3-[2-(4-hydroxy-phényl)-pyrido[2,3-b]pyrazin-6-yl]-thiourée
1-allyl-3-[3-(4-nitro-phényl)-pyrido[2,3-b]pyrazin-7-yl]-thiourée
1-cyclopropyl-3-(3-phényl-pyrido[2,3-b]pyrazin-6-yl)-urée
1-allyl-3-[3-(4-hydroxy-phényl)-pyrido[2,3-b]pyrazin-6-yl]-urée
1-3-(3-phényl-pyrido[2,3-b]pyrazin-6-yl)-3-p-tolyl-urée
1-(4-chloro-3-trifluorométhyl-phényl)-3-(3-phényl-pyrido[2,3-b]pyrazin-6-yl)-urée
1-(2-morpholin-4-yl-éthyl)-3-(3-phényl-pyrido[2,3-b]pyrazin-6-yl)-urée
1-phénéthyl-3-(3-phényl-pyrido[2,3-b]pyrazin-6-yl)-urée
1-(2,3-Di-pyridin-2-yl-pyrido[2, 3-b]pyrazin-6-yl)-3-éthyl-urée
1-(2,3- diméthyl-pyrido[2, 3-b]pyrazin-6-yl)-3-éthyl-urée.

6. Médicament , contenant au moins un composé répondant à la formule générale 1, selon les revendications 1 à 5.

7. Médicament selon la revendication 6, le composé existant en combinaison avec au moins une autre substance active pharmaceutique et/ou des excipients et/ou des diluants ou d'autres adjuvants dans la composition.

8. Procédé pour la production d'un médicament selon les revendications 6 et 7,
**caractérisé en ce que** :
un ou plusieurs dérivé(s) de pyrido-[2,3-b]pyrazine répondant à la formule générale 1 selon une des revendications 1 à 5 est (sont) transformé(s) par mélange avec des excipients et/ou des diluants pharmaceutiques courants ou d'autres adjuvants en préparations pharmaceutiques, ou mis sous une forme thérapeutiquement utilisable.

9. Composé selon une des revendications 1 à 5 pour utilisation comme produit pharmaceutique.

10. Utilisation des dérivés de pyrido[2,3-b]pyrazine répondant à la formule générale 1 selon une des revendications 1 à 5 comme substances actives thérapeutiques pour la production de médicaments destinés au traitement de maladies qui résultent de processus de transduction des signaux cellulaires mal dirigés.

11. Utilisation selon la revendication 10 pour le traitement de maladies reposant sur des proliférations pathologiques des cellules, comme la resténose, le psoriasis, l'artériosclérose et la cirrhose du foie.

12. Utilisation selon la revendication 10 pour le traitement de tumeurs malignes ou bénignes, en particulier du sein, de la prostate, du poumon, de la peau et des ovaires.

13. Utilisation selon les revendications 10 à 12 pour le traitement de maladies qui résultent de processus de transduction de signaux cellulaires mal dirigés, de maladies reposant sur des proliférations pathologiques des cellules et des tumeurs malignes ou bénignes chez l'homme, les mammifères et la volaille.

14. Utilisation selon la revendication 10 pour la modulation de processus de transduction des signaux cellulaires mal dirigés, en particulier pour influencer la fonction de kinases de tyrosine et de sérine/thréonine actives et inactives, comme c-Raf, B-Raf, Mek, MAPKs, PDGFRbeta, FIT-3, IGF1R, PKB / Akt1, c-Kit, c-Ab1, FGR1 et KDR.
